# EUROPÄISCHE PATENTSCHRIFT

(11) **EP 2 135 857 B1**
(45) Veröffentlichungstag und Bekanntmachung des Hinweises auf die Patenterteilung: **06.05.2015**
(21) Anmeldenummer: 09007511.0
(22) Anmeldetag: 06.06.2009
(51) Int. Cl.: C07C 68/06, C07C 69/96

(54) **Verfahren zur Herstellung von Diarylcarbonaten aus Dialkylcarbonaten**
Method for manufacturing diaryl carbonates from dialkyl carbonates
Procédé de fabrication de diarylcarbonates à partir de dialkylcarbonates

(30) Priorität: 21.06.2008 DE 102008029514
(43) Veröffentlichungstag der Anmeldung: 23.12.2009
(73) Patentinhaber: Bayer Intellectual Property GmbH, 40789 Monheim (DE)
(72) Erfinder: Düx, Andre, 50321 Brühl (DE); Olf, Günther, Dr., 51373 Leverkusen (DE); Hallenberger, Kaspar, 51375 Leverkusen (DE); Ronge, Georg, Dr., 40549 Düsseldorf (DE); Leiberich, Ricarda, Dr., 63263 Neu-Isenburg (DE); Eynde, Johan Vanden, 9052 Zwijnaarde (BE); Wuytack, Wim, 9240 Zele (BE); Ooms, Pieter, Dr., 47800 Krefeld (DE); Rechner, Johann, Dr., 47906 Kempen (DE)
(74) Vertreter: BIP Patents

(56) Entgegenhaltungen:
- EP-A1- 0 781 760
- EP-A1- 1 795 522
- AGRAWAL, R., FIDKOWSKI, Z. T.: "On the Use of Intermediate Reboilers in the Rectifying Section and Condensers in the Stripping Section of a Distillation Column" IND. ENG. CHEM. RES., Bd. 35, 1996, Seiten 2801-2807, XP002578007
- D. STEINMEYER: "Process Energy Conservation", KIRK-OTHMER ENCYCLOPEDIA OF CHEMICAL TECHNOLOGY, 4 December 2000 (2000-12-04), pages 1-27, DOI: 10.1002/0471238961.161815031920050

## Beschreibung

Gegenstand der Erfindung ist ein Verfahren zur Herstellung von Diarylcarbonaten aus Dialkylcarbonaten und aromatischen Hydroxyverbindungen unter Einsatz von wenigstens zwei Reaktionskolonnen, einem Verfahrensabschnitt zur Rückgewinnung des bei der Reaktion eingesetzten Dialkylcarbonats und zur Abtrennung des Reaktionsalkohols, einem oder mehren Verfahrensschritten zur Abtrennung der im Verfahren anfallenden Nebenprodukte mit einem Siedepunkt zwischen dem des Dialkylcarbonats und dem des während der Herstellung des Diarylcarbonats gebildeten Alkylarylcarbonats sowie einem Verfahrensschritt zur weiteren Aufreinigung des aus den Reaktionskolonnen erhaltenen Diarylcarbonats.

Die Herstellung von aromatischen und aliphatisch-aromatischen Kohlensäurestern (Carbonaten) durch Umesterung ausgehend von aliphatischen Kohlensäurestern und aromatischen Hydroxyverbindungen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv auf die Seite der aromatischen Carbonate zu verschieben, wobei nicht nur sehr aktive Katalysatoren, sondern auch geeignete Verfahrensführungen zur Anwendung gelangen müssen.

Es ist bekannt, derartige Gleichgewichtsreaktionen in Kolonnen durchzuführen und sie auf diese Weise vorteilhaft in Richtung der gewünschten Produktbildung zu verschieben (z.B. U. Block, Chem.-Ing. Techn. 49, 151 (1977); DE-OS 38 09 417; B. Schleper, B. Gutsche, J. Wnuck und L.Jeromin, Chem.-Ing.-Techn. 62, 226 (1990); Ullmans Encyclopädie der techn. Chemie, 4. Aufl., Bd. 3; S375 ff. 1973).

In den bekannten Verfahren erfolgt die Umesterung daher auch vorzugsweise kontinuierlich im Sinne einer Gegenstromumesterung in einer oder mehreren Reaktionskolonnen.

Die aus der Literatur bekannten Verfahren, wie z.B. EP-A 461 274, DE-A 42 26 755, DE-A 42 26 756, beschreiben jedoch in der Regel nur diejenigen Verfahrensschritte, bei denen die Reaktion zum Diarylcarbonat durch Umesterung und/oder Disproportionierung stattfmdet. In WO-A 2006/033291, EP-A 1 775 280, EP-A 1 767 516, EP-A 1 767 517, EP-A 1767 518, EP-A 1 762 559 und EP-A 1 762 560 werden zudem Hinweise bezüglich der apparativen Ausführungen von Reaktionskolonnen zur Herstellung von Diarylcarbonaten gegeben. Für die Wirtschaftlichkeit eines Verfahrens sind aber nicht nur die Verfahrensabschnitte im Bereich der Reaktion sondern zum Teil in weit größerem Maße die sich anschließenden Schritte für die Aufarbeitung von Relevanz. Diesbezüglich finden sich in der Literatur bisher nur wenige Informationen.

Da die Herstellung von Diarylcarbonaten durch Umsetzung einer aromatischen Hydroxyverbindung mit einem Dialkylcarbonat erfahrungsgemäß energetisch sehr aufwändig ist, spielen Maßnahmen zur Reduzierung des Energieverbrauchs ebenfalls eine bedeutende Rolle. Auch diesbezüglich gibt die derzeit vorliegende Literatur nur wenig Aufschluss.

Die EP-A 781 760 beschreibt ein kontinuierliches Verfahren zur Herstellung aromatischer Carbonate durch Reaktion eines Dialkylcarbonates mit einer aromatischen Hydroxyverbindung in Anwesenheit eines Katalysators, kontinuierlicher Entfernung des bei der Reaktion entstehenden aromatischen Carbonats, der alkoholischen Nebenprodukte, des Dialkylcarbonats und der aromatischen Hydroxyverbindung, wobei das Dialkylcarbonat und die aromatische Hydroxyverbindung wieder in die Reaktion zurückgeführt werden. Allerdings wird nicht beschrieben, wie die Trennung des in der Reaktion eingesetzten Dialkylcarbonats vom alkoholischen Nebenprodukt (Reaktionsalkohol) erfolgt. Dieser Schritt ist aber erfahrungsgemäß sehr energienintensiv und aufwändig, insbesondere dann, wenn Dialkylcarbonat und Reaktionsalkohol schlecht voneinander zu trennen sind. Des Weiteren wird auch die Isolierung des Diarylcarbonats nach der Reaktion nicht beschrieben, welche wegen der hohen Reinheitsanforderungen an dieses jedoch sehr aufwändig ist. Ferner werden keine Angaben bezüglich möglicher Energieeinsparungen gemacht.

EP-A 1 638 917 beschreibt ein Verfahren zur Rückgewinnung eines Produktes aus einem Abfallstrom durch Kontaktieren mit einem Alkylalkohol, wobei das rückgewonnene Produkt Diarylcarbonat, aromatischen Alkohol, Alkylsalicylat und Alkylalkohol enthält. Nachteilig am beschriebenen Verfahren ist zum einen, dass die Reaktion in drei Stufen erfolgt wodurch diese sehr aufwändig wird. Zum anderen fallen an zwei Stellen hochsiedende Abfallströme an. Durch Abtrennung des Katalysators vor der Isolierung des Diarylcarbonats fällt bereits der erste Abfallstrom, bei der sich anschließenden, aus zwei Destillationskolonnen bestehenden Aufarbeitung der zweite Abfallstrom an. Somit ist die Aufarbeitung zur Isolierung des Diarylcarbonats sowohl apparativ als auch energetisch sehr aufwändig. Zudem ist die Qualität des so hergestellten Diarylcarbonats mit 99,5 Gew.-% sehr schlecht und eine Eignung für die Herstellung von Polycarbonat fragwürdig. Auch die Trennung des bei der Reaktion anfallenden Gemisches aus Reaktionsalkohol und Dialkylcarbonat wird nicht beschrieben.

WO-A 2005/000776 beschreibt ein Verfahren zur Herstellung eines Alkylarylethers, welcher bei der Umsetzung eines Dialkylcarbonats mit einer aromatischen Hydroxyverbindung gebildet wird. Bei diesem Verfahren wird zudem auch Diarylcarbonat erhalten. Der Verfahrensaufbau umfasst drei Reaktionskolonnen und zwei weiteren Destillationskolonnen zwecks Isolierung des Alkylarylethers. Die Tatsache, dass beim hier beschriebenen Verfahren eine gezielte Aufreinigung des Alkylarylethers angestrebt wird lässt darauf schließen, dass die in der Reaktion gebildete Menge hoch ist. Bei der Herstellung von Diarylcarbonaten steht jedoch die Gewinnung eines hochreinen Alkylaralyethers nicht im Vordergrund, sonders es ist vielmehr eine möglichst geringe Bildung dieses- bei- der- Umesterung anfallenden Nebenproduktes anzustreben. Zudem ist die-Reaktionführung mit drei Reaktionsstufen sehr aufwändig und bezuglich der Aufarbeitung des Diarylcarbantats und der Trennung des bei- der Reaktion anfallenden Gemisches, enthaltend Dialkylcarbonat und Reaktionsalkohol, werden keine Angaben gemacht. Auch in EP-A 1 237 842 wird ein vergleichbares Verfahren beschrieben, weshalb diesbezüglich die bereits genannten Nachteile ebenfalls gelten.

Die WO-A 2004/016577 beschreibt ein Verfahren zur Herstellung von aromatischen Carbonaten aus Dialkylcarbonat und einer aromatischen Hydroxyverbindung in Gegenwart eines Katalysators in mehreren getrennten und in Serie geschalteten Reaktionszonen einer Reaktoranordnung, wobei die anfallende Kondensationswärme bei der Kondensation des Dampfstromes der letzten Reaktionszone zur Erwärmung des in die erste Reaktionszone eingeleiteten Flussigkeitsstromes verwendet wird. Nachteilig- an diesem Verfahren ist jedoch die aufwändige Reaktoranordnung. Zudem ist die energetische Integration dieses Verfahrens verbesserungswurdig und nur auf den Verfahrensabschnitt Reaktion begrenzt. Anschließende Schritte für die Aufarbeitung werden nicht beschrieben.

JP-A 2002-020351 beschreibt ein diskontinuierliches Verfahren zur- Herstellung von Diarylcarbonat, aus dem Wärme zur Dampferzeugung genutzt werden kann. Nachteiling an diesem Verfahren sind jedoch die diskontinuierliche Durchführung sowie die zur Umsetzung verwendete Reaktoranordnung mit aufgesetzter Destillationskolonne. Anschließend Schritte für die-Aufarbeitung werdennich beschrieben.

EP 1 795 522 A1 offenbart ein Verfahren zur Herstellung eines Diarylcarbonats aus einem Dialkylcarbonat und einer aromatischen Hydroxyverbindung, wobei der am Kopf einer ersten Reaktionskolonne (Kolonne A) anfallende Dampf einem Kondensator zugeführt und die dort gewonnene Wärme zur Aufheizung eines Eduktstroms verwendet wird. Die dort verfügbaren Energieniveaus sind jedoch nicht für eine Energieintegration gemäß vorliegendem Anspruch möglich.

AGRAWAL, R.; FIDKOWSKI, Z. T.: "On the Use of Intermediate Reboilers in the Rectifying Section and Condensers in the Stripping Section of a Distillation Column" IND. ENG. CHEM. RES., Bd. 35, 1996, Seiten 2801-2807 beschreibt eine Destillation mit der Verwendung eines Zwischenkondensators im Verstärkungsteil der Destillationskolonne.

Es bestand demnach weiterhin Bedarf, ein Verfahren zur Herstellung von aromatischen Carbonaten, vorzugweise Diarylcarbonaten, bereitzustellen, welches eine Aufarbeitung von Produkt und Abfallströmen umfasst, die-vorangehend- genannten- Nachteile nicht aufweist und in welchem gegenüber den vorangehend genannten bekannten Verfahren eine Energieintegration in effizienter Weise möglich ist bzw eine verbesserte Energieintegration erreicht werden kann.

Die Aufgabe, die der Erfindung zugrunde lag, bestand demnach darin, ein Verfahren zur Herstellung von aromatischen Carbonaten, vorzugsweise Diarylcarbonaten, bereitzustellen, welches eine Aufarbeitung von Produkt und Abfallströmen umfasst und in welchem gegenüber bekannten Verfahren eine Energieintegration in effrizienter Weise möglich ist bzw. eine verbesserte Energieintegration erreicht werden kann.

Überraschend wurde gefunden, dass ein Verfahren zur Herstellung von wenigstens einem Diarylcarbonat aus wenigstens einem Dialkylcarbonat und wenigstens einer aromatischen Hydroxyverbindung wie in Anspruch 1 definiert sowohl eine Aufarbeitung von Produkt- und Abfallströmen als auch eine effiziente Energieintegration ermöglicht.

Gegenstand der vorliegenden Erfindung ist daher ein Verfahren zur Herstellung von wenigstens einem Diarylcarbonat aus wenigstens einem Dialkylcarbonat und wenigstens einer aromatischen Hydroxyverbindung wie in Anspruch 1 definiert.

Im Rahmen der Erfindung hergestellte Diarylcarbonate sind bevorzugt solche der allgemeinen Formel (I) 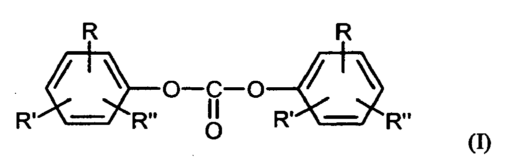 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (I) gleich oder verschieden sein können. R kann auch -COO-R''' bedeuten, wobei R''' H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenyl-carbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung bevorzugt eingesetzte Dialkylcarbonate sind solche der Formel (II) 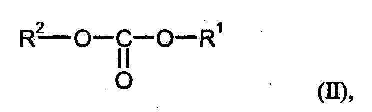 wobei R¹ und R² unabhängig voneinander für lineares oder verzweigtes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, **C₁-C₃₄-Alkyl** darüber hinaus beispielsweise für n-Heptyl und n-Octyl, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

**Aryl** steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, verzweigten oder unverzweigten Alkyl-Rest nach vorstehender Definition, der einfach, mehrfach oder vollständig durch Aryl-Reste gemäß vorstehender Definition substituiert sein kann.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Im Rahmen der Erfindung geeignete aromatische Hydroxyverbindungen sind bevorzugt solche der allgemeinen Formel (III) 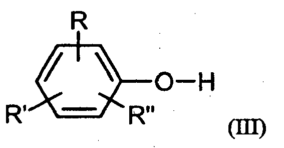 worin R, R' und R" unabhängig voneinander die für die allgemeine Formel (I) genannte Bedeutung haben können.

Solche aromatischen Hydroxyverbindungen sind beispielsweise: Phenol, O-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, O-, m-oder p-Chlorphenol, O-, m- oder p-Ethylphenol, O-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-isoOctylphenol, 4-n-Nonylphenol, O-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte Diarylverbindungen sind Phenol, 4-tert-Butylphenol, Biphenyl-4-ol und 4-(1-Methyl-1-phenylethyl)-phenol.

Besonders bevorzugt ist Phenol.

Im Rahmen der Erfindung als Zwischenprodukte erhaltene Alkylarylcarbonate sind bevorzugt solche der allgemeinen Formel (IV) 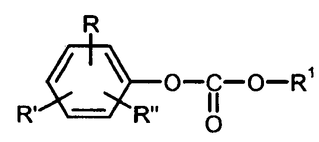 worin R, R' und R" die für die allgemeine Formel (I) und R¹ die für die allgemeine Formel (II) genannte Bedeutung haben können.

Bevorzugte Alkylarylcarbonate sind Methyl-phenyl-carbonat, Ethyl-phenyl-carbonat, Propyl-phenyl-carbonat, Butylphenyl-carbonat und Hexyl-phenyl-carbonat, Methyl-(o-kresyl)-carbonat, Methyl-(p-kresyl)-carbonat, Ethyl-(o-kresyl)-carbonat, Ethyl-(p-kresyl)-carbonat, Methyl- oder Ethyl-(p-chlorphenyl)-carbonat. Besonders bevorzugte Alkylarylcarbonate sind Methyl-phenyl-carbonat und Ethyl-phenyl-carbonat. Ganz besonders bevorzugt ist Methyl-phenyl-carbonat.

Sowohl die für das erfmdungsgemäße Verfahren geeigneten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

Im erfmdungsgemäßen Verfahren werden die aromatische(n) Hydroxyverbindung(en) und das oder die Dialkylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 in der ersten Reaktionskolonne eingesetzt. Dabei berücksichtigt das angegebene Molverhältnis nicht die Rückführung von aromatischer Hydroxyverbindung oder Dialkylcarbonat in die Reaktionskolonne über einen oder mehrere Kopffcondensator(en) oder einen oder mehrere etwaig vorhandene(n) Sumpfverdampfer.

Das erfindungsgemäße Verfahren wird in mindestens zwei Reaktionskolonnen durchgeführt.

Als erste und zweite Reaktionskolonne oder gegebenenfalls dritte bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die erste Reaktionskolonne enthält wenigstens einen Verstärkungsteil im oberen Teil der Kolonne und wenigstens eine Reaktionszone unterhalb des Verstärkungsteils, welcher mindestens zwei Sektionen aufweist. Jede der zwei Sektionen weist unabhängig voneinander bevorzugt jeweils 0 bis 20, bevorzugt 0,1 bis 20 theoretische Stufen auf. In bevorzugten Ausführungsformen ist wenigstens ein Verstärkungsteil der ersten Reaktionskolonne mit wenigstens einem Zwischenkondensator ausgestattet. Der Zwischenkondensator ist vorzugsweise zwischen den beiden Sektionen des Verstärkungsteils angebracht. In diesem Fall wird der Verstärkungsteil in einen oberen und einen unteren Verstärkungsteil geteilt. Eine Sektion im Rahmen der Erfindung zeichnet sich dadurch aus, dass sich unter- und/oder oberhalb dieser Sektion eine Zufuhr- und/oder Entnahmestelle befindet.

Die erste Reaktionskolonne wird bevorzugt im Gegenstrom betrieben, wobei vorzugsweise in wenigstens einer Reaktionszone dieser Kolonne die aromatische Hydroxyverbindung flüssig vom Kopf zum Sumpf geführt und das Dialkylcarbonat gasförmig diesem flüssigen Strom entgegengeführt wird. Dabei wird die erste Reaktionskolonne vorzugsweise so betrieben, dass man wenigstens einer Reaktionszone, bevorzugt in das obere Drittel der Reaktionszone, einen oder mehrere, die aromatische Hydroxyverbindung und gegebenenfalls gelösten Umesterungskatalysator enthaltende Ströme, bevorzugt mit der an dieser Stelle der Kolonne herrschenden Temperatur, flüssig oder mit nur geringem Gasanteil eindosiert, wobei der Gasanteil bevorzugt weniger als 20 Gew.-% beträgt. Zudem werden einer oder mehrere, das Dialkylcarbonat enthaltende Ströme in die Reaktionszone, bevorzugt im unteren Drittel dieser Reaktionszone, geleitet, wobei die Zudosierung vorzugsweise gasförmig oder überhitzt erfolgt. In bevorzugten Ausführungsformen kann die Überhitzung des Dampfstroms 0 bis 50 °C betragen. Des Weiteren richtet sich die Taupunktstemperatur bevorzugt nach dem in der Reaktionszone an der Zudosierstelle des jeweiligen Dialkylcarbonat enthaltenden Stromes vorliegenden Druck.

Nach Passieren der Reaktionszone(n) wird der während der Reaktion gebildete Alkylalkohol, nach Durchlaufen des oder der Verstärkungsteile, am Kopf der ersten Reaktionskolonne entnommen. Beim während der Reaktion gebildeten Alkylalkohol, auch Reaktionsalkohol genannt, handelt es sich im Rahmen der Erfindung um den bei der Umesterung frei werdenden Alkohol, bevorzugt R¹-OH bzw. R²-OH, wobei R¹ und R² die für die allgemeine Formel (II) genannte Bedeutung haben. Der am Kopf der ersten Reaktionskolonne entnommene Strom enthält im Allgemeinen zusätzlich zum während der Reaktion gebildeten Alkylalkohol noch überschüssiges oder nicht umgesetztes Dialkylcarbonat und leichtsiedende Nebenverbindungen, wie beispielsweise Kohlendioxid oder Dialkylether. Aufgrund des oder der vorhandenen Verstärkungsteil(e) enthält dieser Strom nur geringe Mengen an höher siedenden Komponenten, wie z.B. der aromatischen Hydroxyverbindung. Der Verstärkungsteil dient zur Abtrennung der in der Reaktionszone mitverdampften höher siedenden Komponenten, wie z.B. der aromatischen Hydroxyverbindung oder Alkylarylcarbonat von den leichtsiedenden Reaktionsalkoholen oder Dialkylcarbonaten. Dies hat den Vorteil, dass die Trennung der während der Reaktion gebildeten Alkylalkohole von den Dialkylcarbonaten bei einem niedrigen Temperaturniveau durchgeführt werden kann.

Die erste Reaktionskolonne wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgefiihrtes Kondensat entspricht.

In bevorzugten Ausführungsformen weist die erste Reaktionskolonne unterhalb einer Reaktionszone wenigstens einen Abtriebsteil auf.

Die erste Reaktionskolonne kann weiterhin bevorzugt mit einem oder mehreren Sumpfverdampfer(n) ausgestattet sein. Bei Ausführung der ersten Reaktionskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die erste Reaktionskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die erste Reaktionskolonne zurückgeführt.

Weiterhin bevorzugt kann die erste Reaktionskolonne im Bereich des Abtriebsteils und/oder der Reaktionszone einen oder mehrere Zwischenerhitzer oder Zwischenverdampfer aufweisen.

In den bevorzugten Ausführungsformen, in denen wenigstens ein Verstärkungsteil der ersten Reaktionskolonne mit wenigstens einem Zwischenkondensator ausgestattet ist, ist der Verstärkungsteil der ersten Reaktionskolonne, der mit wenigstens einem Zwischenkondensator ausgestattet ist, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden.

Der oder die Verstärkungsteil(e) mit wenigstens einem Zwischenkondensator können in bevorzugten Ausführungsformen zusammen mit dem oder den Reaktionsteil(en) und gegebenenfalls wenigstens einem Abtriebsteil in der Reaktionskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung der aromatischen Hydroxyverbindung stattfindet. Das aus dieser unteren Sektion bzw. gegebenenfalls dem unteren Verstärkungsteil kommende dampfförmige Gemisch wird in einen Zwischenkondensator geleitet, wo dieses teilweise auskondensiert und das anfallende Kondensat am oberen Ende der unteren Sektion des Verstärkungsteils bzw. gegebenenfalls dem unteren Verstärkungsteil zugeführt wird.

In einer weiteren bevorzugten Ausführungsform des erfindungsgemäßen Verfahrens wird der Zwischenkondensator nicht in die erste Reaktionskolonne integriert sondern als separater Zwischenkondensator außerhalb der ersten Reaktionskolonne ausgeführt.

In einer weiteren bevorzugten Ausführungsform des erfindungsgemäßen Verfahrens werden Zwischenkondensator und die obere Sektion des Verstärkungsteils nicht in die Reaktionskolonne integriert sondern separat außerhalb der ersten Reaktionskolonne untergebracht.

Unterhalb der Reaktionszone und eines gegebenenfalls vorhandenen Abtriebsteils wird ein Gemisch enthaltend Alkylarylcarbonat, überschüssigem oder nichtumgesetztem Phenol, Diarylcarbonat, Umesterungskatalysatoren, Dialkylcarbonat, Reaktionsalkohol und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Reaktionsalkohol reduziert, wobei in Anwesenheit des Umesterungskatalysators unter Umständen weiteres Alkylarylcarbonat und/oder Diarylcarbonat gebildet werden. Die hierzu erforderliche Energie, wird bevorzugt durch einen oder mehrere Verdampfer zugeführt.

In allen Abschnitten der ersten Reaktionskolonne sowie auch der im Folgenden beschriebenen Kolonnen, d.h. sowohl in Verstärkungs- und/oder gegebenenfalls Abtriebsteil und/oder in der Reaktionszone, können zur Erreichung der betreffenden Trennleistung Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Bei der Verwendung von Füllkörperschüttungen und/oder strukturierten Packungen kann eine Sektion in mehrere Abschnitte unterteilt sein, wenn die Sektion mehr als 4, bevorzugt mehr als 10 und besonders bevorzugt mehr als 15 theoretische Stufen aufweist.

Alternativ eignen sich auch Kolonnenböden, wie beispielesweise Sieb-, Glocken-, Ventil-, Tunnelböden. In der oder den Reaktionszone(n) der Reaktionskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt.

Die theoretische Bodenzahl der Reaktionszone der ersten Reaktionskolonne beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Bei der ersten Reaktionskolonne richtet sich der Kolonnendurchmesser im Bereich der Reaktionszone allerdings nur mit Einschränkungen nach dem Gasdurchsatz. Er wird ferner beeinflusst durch den zu realisierenden Hold-up.

Bei Verwendung von Verweilzeitböden sollte der Flüssigkeitsstand auf den Böden vorzugsweise 50 bis 1000, besonders bevorzugt 100 bis 500 und ganz besonders bevorzugt 100 bis 250 mm betragen um den Druckverlust der Kolonne auf ein sinnvolles Maß zu begrenzen. Der Druckverlust der Kolonne sollte vorzugsweise weniger als 50, besonders bevorzugt weniger als 30 und ganz besonders bevorzugt weniger als 25 % des Kopfdruckes betragen.

Unter diesen Randbedingungen liegt der F-Faktor in der Kolonne vorzugsweise zwischen 0,05 und 2,5, bevorzugt 0,05 bis 1,5 und besonders bevorzugt zwischen 0,08 und 1 Pa^{0.5}. Der Bodenabstand kann vorzugsweise 250 bis 1500 mm, besonders bevorzugt 300 bis 1000 und ganz besonders bevorzugt 500 bis 1000 mm betragen. Der F-Faktor ist ein Maß für die gasseitige hydrodynamische Belastung der Kolonne und berechnet sich wie folgt:

F-Faktor = Gasdichte^{1/2} • Gasgeschwindigkeit

Ein geeignetes Kolonnendesign der restlichen im Verfahren eingesetzten Destillations- und/oder Reaktionskolonnen, welches sowohl die Festlegung der Kolonnenhöhe, des Kolonnendurchmessers, die Auswahl der Kolonneneinbauten als auch die Dimensionierung der Zulauf- und Entnahmeleitungen umfasst, ist dem Fachmann bekannt und kann der einschlägigen Literatur (z.B. Distillation Design, Henry Z. Kister, Mc Gra Hill; Distillation Operation, Henry Z. Kister, Mc Gra Hill; Perry's Chemical Engineering Handbook; Perry & Green) entnommen werden.

Die Temperatur der Reaktionszone(n) liegt bevorzugt im Bereich von 100 bis 300°C, besonders bevorzugt von 120 bis 250°C, ganz besonders bevorzugt von 150 bis 240°C. In bevorzugten Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0.5 bis 20 bar (absolut), besonders bevorzugt von 0.8 bis 15 bar (absolut), ganz besonders bevorzugt von 0.9 bis 10 bar (absolut).

Für die in der ersten Reaktionskolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie z. B. Metallverbindungen wie AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfindung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)_{4-X}-Sn(Y)_{X}, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenylstannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen in Betracht, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiCl, Pb(OCO-CH₃)₂ • 2PPh3 in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den erfindungsgemäßen Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die erste Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der ersten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) als auch mit dem gasförmig eindosierten, Dialkylcarbonat enthaltenden Strom eingebracht werden. Insbesondere im Bereich der Reaktionszone(n) kann eine Zufuhr von Wärme auf diese Weise erfolgen. Vorzugsweise wird diese Wärme im Bereich der Reaktionszone(n) ganz oder teilweise mittels Verdampfer oder beheizbarer Kolonnenböden zugeführt. Besonders vorteilhaft ist es, die für die Reaktion in der ersten Reaktionskolonne erforderliche Energie wenigstens teilweise sowohl mit dem flüssigen Strom enthaltend die aromatische(n) Hydroxyverbindung(en) als auch mit dem gasförmig eindosierten, Dialkylcarbonat enthaltenden Strom in die ersten Reaktionskolonne und zusätzlich durch innen- und/oder außen liegende Wärmetauscher einzubringen.

Im erfindungsgemäßen Verfahren wird das Sumpfprodukt der ersten Reaktionskolonne einer zweiten Reaktionskolonne zugeführt.

Die zweite Reaktionskolonne enthält wenigstens einen Verstärkungsteil im oberen Teil der Kolonne und wenigstens eine Reaktionszone unterhalb des Verstärkungsteils. Der Verstärkungsteil weist bevorzugt 1 bis 50, besonders bevorzugt 1 bis 25 theoretische Stufen auf.

In der zweiten Reaktionskolonne wird das Sumpfprodukt der ersten Reaktionskolonne, welches bereits gebildetes Alkylarylcarbonat und Diarylcarbonat enthält, flüssig oder als Dampf-Flüssigkeits-Gemisch bevorzugt der Reaktionszone, besonders bevorzugt dem oberen Teil der Reaktionszone, ganz besonders bevorzugt im oberen Drittel der Reaktionszone zugeführt. Dabei wird die zweite Reaktionskolonne vorzugsweise so betrieben, dass man das Alkylarylcarbonat teilweise oder vollständig, beispielsweise durch weitere Umesterung oder Disproportionierung, bevorzugt durch Disproportionierung, zum Diarylcarbonat umsetzt. Zusätzlich zum Sumpfpdrodukt der ersten Reaktionskolonne können einer oder mehrere, Alkylarylcarbonat enthaltende Ströme flüssig oder als Dampf-Flüssigkeitsgemisch im Bereich der Reaktionszone eindosiert werden. Solche zusätzlichen Alkylarylcarbonat enthaltenden Ströme können beispielsweise aus der weiteren Aufarbeitung stammen und so in das Verfahren zurückgeführt werden.

Am Kopf der zweiten Reaktionskolonne werden nicht umgesetzte aromatische Hydroxyverbindung, Dialkylcarbonat, Reaktionsalkohol, mittelsiedende Nebenverbindungen - wie beispielsweise Alkylarylether - und in geringem Maße leichtsiedende Nebenverbindungen abgetrennt. Im Rahmen der Erfindung sind unter mittelsiedenden Nebenverbindungen solche mit einem Siedepunkt unterhalb dem des Alkylarylcarbonats und oberhalb dem des Dialkylcarbonats zu verstehen. Solche mittelsiedenden Nebenverbindungen sind z.B. Alkylarylether, wie beispielsweise Anisol oder Phenetol. Die in der zweiten Reaktionskolonne abgetrennten mittelsiedenden Nebenverbindungen können in der ersten und/oder zweiten Reaktionskolonne bei der Reaktion entstehen oder bereits durch die Edukte in das Verfahren eingeschleust worden sein.

Der Verstärkungsteil der zweiten Reaktionskolonne dient zur Abtrennung der in der Reaktionszone mitverdampften höher siedenden Komponenten, wie z.B. Alkylarylcarbonat.

Die zweite Reaktionskolonne wird in bevorzugten Ausführungsformen ebenfalls unter solchen für die erste Reaktionskolonne beschriebenen Rückflussbedingungen betrieben.

Die zweite Reaktionskolonne kann unterhalb einer Reaktionszone wenigstens einen Abtriebsteil aufweisen. In bevorzugten Ausführungsformen kann die Reaktionszone der zweiten Reaktionskolonne jedoch gleichzeitig als Abtriebsteil fungieren. Dabei wird das bei der Disproportionierung freigesetzte Dialkylcarbonat, durch Umesterung freigesetzter Reaktionsalkohol und nicht umgesetzte aromatische Hydroxyverbindung abgetrennt und gleichzeitig Diarylcarbonat und das im Wesentlichen durch Disproportionierung abreagierende Alkylarylcarbonat aufkonzentriert.

Die zweite Reaktionskolonne kann weiterhin bevorzugt mit einem oder mehreren Sumpfverdampfer(n) ausgestattet sein.

Weiterhin bevorzugt kann die zweite Reaktionskolonne im Bereich des Abtriebsteil und/oder der Reaktionszone einen oder mehrere Zwischenerhitzer oder Zwischenverdampfer aufweisen.

Prinzipiell kann der Verstärkungsteil der zweiten Reaktionskolonne ebenfalls mit einem oder mehreren Zwischenkondensatoren ausgestattet werden. Hierdurch wird der Verstärkungsteil, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden. In einer bevorzugten Ausführungsform weist wird die zweite Reaktionskolonne keinen Zwischenkondensator auf.

Die zweite Reaktionskolonne ist mit einem oder mehreren Kondensatoren ausgestattet. Bevorzugt handelt es sich dabei um einen oder mehrere Kondensatoren am Kopf der zweiten Reaktionskolonne (Kopfkondensator(en)). Besonders bevorzugt wird eine Kaskade von Kopfkondensatoren verwendet.

Im Laufe der Kondensation in dem oder den Kondensator(en) am Kopf der zweiten Reaktionskolonne verarmen die Dämpfe an höher siedenden Komponenten, wie z.B. aromatischer Hydroxyverbindung. Um die anfallende Kondensationswärme im Sinne einer Wärmeintegration besonders effizient nutzen zu können, erfolgt die Kondensation daher bevorzugt mehrstufig, besonders bevorzugt mindestens zweistufig, in bevorzugten Ausführungsformen zwei- oder dreistufig.

Bei der besonders bevorzugten Ausführungsform der zwei- oder dreistufigen Kondensation wird die Kondensationswärme der ersten oder der ersten und zweiten Kondensationsstufe direkt oder indirekt zum Erwärmen eines Stoffstromes oder einer Kolonne innerhalb des Verfahrens verwendet, während die anfallende Kondensationswärme der zweiten oder dritten Kondensationsstufe durch Kühlwasser oder Luftkühlung abgeführt wird.

Die Kondensation am Kopf der zweiten Reaktionskolonne kann in weiteren bevorzugten Ausführungsformen zudem so durchgeführt werden, dass ein Teil der am Kopf der zweiten Reaktionskolonne entnommenen Dämpfe nicht kondensiert wird, um mittelsiedende Nebenverbindungen selektiv ausschleusen zu können.

Unterhalb der Reaktionszone und eines gegebenenfalls vorhandenen Abtriebsteils wird ein Gemisch enthaltend Alkylarylcarbonat, überschüssiger oder nichtumgesetzter aromatischer Hydroxyverbindung, Diarylcarbonat, Umesterungskatalysator(en), Dialkylcarbonat, Reaktionsalkohol und bei der Reaktion entstehende oder bereits in den Edukten enthaltene mittel- oder schwersiedende Nebenverbindungen erhalten. Im Rahmen der Erfindung sind unter schwersiedenden Nebenverbindungen solche mit einem Siedepunkt oberhalb dem des Alkylarylcarbonats zu verstehen. Solche schwersiedenden Nebenverbindungen lassen sich noch in solche, deren Siedepunkt zwischen dem des Alkylarylcarbonats und dem des Diarylcarbonats liegt (Schwersieder), und solche, deren Siedepunkt oberhalb des Siedepunktes des Diarylcarbonats liegt (Hochsieder), unterteilen.

In allen Abschnitten der zweiten Reaktionskolonne, d.h. sowohl in Verstärkungs- und gegebenenfalls Abtriebsteil als auch in der Reaktionszone können die bereits vorangehend für die erste Reaktionskolonne genannten Füllkörper oder geordnete Packungen zum Einsatz kommen.

Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 180 bis 250°C.

In besonderen Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck, bevorzugt bei erniedrigtem Druck, durchzuführen. Der Druck der zweiten Reaktionskolonne liegt daher bevorzugt im Bereich von 0.05 bis 20 bar (absolut), besonders bevorzugt von 0.1 bis 10 bar (absolut), ganz besonders bevorzugt von 0.1 bis 2 bar (absolut).

Für die in der zweiten Reaktionskolonne auftretenden Reaktionsschritte können die vorangehend bereits für die Umesterung in der ersten Reaktionskolonne genannten Umesterungskatalysatoren eingesetzt werden. In einer bevorzugten Ausführungsform werden in der ersten und zweiten Reaktionskolonne identische Katalysatoren verwendet.

Der Katalysator wird bevorzugt zusammen mit dem Sumpfprodukt der ersten Reaktionskolonne in gelöster oder suspendierter Form in die zweite Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der zweiten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits mit dem flüssigen Strom enthaltend die aromatische(n) Hydroxyverbindung(en) eingebracht werden. Vorzugsweise wird diese Wärme im Bereich der Reaktionszone(n) ganz oder teilweise mittels Verdampfer zugeführt.

An die zweite Reaktionskolonne können sich eine oder mehrere weitere Reaktionskolonnen anschließen. Für solche weiteren Reaktionskolonnen gelten die vorangehend für die zweite Reaktionskolonne genannten Bedingungen und Parameterbereiche, wobei die Bedingungen und Parameter weiterer Reaktionskolonnen jedoch nicht identisch zu denen in der zweiten Reaktionskolonne sein müssen, sondern sich bevorzugt innerhalb des vorangehend genannten Rahmens der Bedingungen und Parameterbereiche von denen in der zweiten Reaktionskolonne unterscheiden. Vorzugsweise wird eine zur zweiten Reaktionskolonne zusätzliche Reaktionskolonne beispielsweise bei niedrigerem Druck betrieben als die zweite Reaktionskolonne; auch Rücklaufverhältnis und Sumpftemperatur können gegenüber denen in der zweiten Reaktionskolonne verändert sein. In einer bevorzugten Ausführungsform schließt sich im erfindungsgemäßen Verfahren an die erste Reaktionskolonne lediglich eine weitere Reaktionskolonne, d.h. die vorangehend genannte zweite Reaktionskolonne an. An die Reaktionskolonnen können sich jedoch weitere Kolonnen zur Aufreinigung und Trennung der Komponenten der entnommenen Ströme anschließen. Solche Kolonnen zur Aufreinigung und Trennung der Komponenten werden im Rahmen der Erfindung nicht als Reaktionskolonnen im Sinne der Erfindung verstanden, und als Destillationskolonnen bezeichnet.

Bei den Kondensatoren, mit denen wenigstens eine der Reaktionskolonne(n) ausgewählt aus der ersten oder der oder den weitere(n) Reaktionskolonne(n) ausgestattet ist und deren gewonnene Kondensationswärme direkt oder indirekt wieder in das Verfahren zurückgeführt wird, kann es sich sowohl um Kondensatoren am Kopf der Kolonnen - im Folgenden auch als Kopfkondensatoren bezeichnet - oder Kondensatoren im Verstärkungsteil der Kolonnen - im Folgenden auch als Zwischenkondensatoren bezeichnet - handeln.

Bevorzugt ist im erfindungsgemäßen Verfahren wenigstens ein Verstärkungsteil der ersten Reaktionskolonne mit wenigstens einem Zwischenkondensator ausgestattet und die durch Kondensation in diesem Zwischenkondensator gewonnene Kondensationswärme wird direkt oder indirekt wieder in das Verfahren zurückgeführt.

Weiterhin bevorzugt ist im erfmdungsgemäßen Verfahren die oder wenigstens eine der weitere(n) Reaktionskolonne(n) mit einem oder mehreren Kondensatoren am Kopf der Reaktionskolonne(n) ausgestattet und die durch Kondensation in diesen Kondensatoren gewonnene Kondensationswärme wird direkt oder indirekt wieder in das Verfahren zurückgeführt.

Die durch Kondensation in dem oder den Kondensator(en), bevorzugt Kopfkondensator(en) der zweiten oder den weiteren Reaktionskolonne(n), vorzugsweise der zweiten Reaktionskolonne, gewonnene Kondensationswärme wird erfindungsgemäß direkt oder indirekt, ganz oder teilweise wieder in das Verfahren zurückgeführt. Für den Fall, dass die erste Reaktionskolonne mit einem oder mehreren Zwischenkondensatoren ausgestattet ist, wird die durch Kondensation in dem oder den Zwischenkondensator(en) gewonnene Kondensationswärme erfindungsgemäß ebenfalls direkt oder indirekt, ganz oder teilweise wieder in das Verfahren zurückgeführt. Unter direkter Rückführung der Kondensationswärme in das Verfahren ist im Rahmen der Erfindung zu verstehen, dass diese Kondensationswärme ohne zwischengeschaltetes Heizmedium in das Verfahren zurückgeführt wird, z.B. zur Erwärmung entweder eines oder mehrerer Ströme oder zur Erwärmung eines oder mehrerer Kolonnenabschnitte innerhalb des Verfahrens. Dies kann beispielsweise in einem Wärmetauscher erfolgen. Vorzugsweise ist dabei ein solcher Wärmeaustauscher mit dem oder den Kondensatoren kombiniert. Unter indirekter Rückführung der Kondensationswärme in das Verfahren ist im Rahmen der Erfindung zu verstehen, dass mit der gewonnen Kondensationswärme zunächst ein Heizmedium erzeugt wird, welches zur Rückführung der Kondensationswärme in das Verfahren dient. Mit diesem Heizmedium können beispielsweise ein oder mehrere Ströme oder einer oder mehrere Kolonnenabschnitte innerhalb des Verfahrens erwärmt werden. Als Heizmedium kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl^{™}, Marlotherm^{®}). Besonders bevorzugte Heizmedien sind Wasser oder Wasserdampf.

Die durch Kondensation in dem oder den Kondensatoren, vorzugsweise Kopfkondensator(en), der weiteren Reaktionskolonne(n) und/oder dem oder den gegebenenfalls vorhandenen Zwischenkondensator(en) der ersten Reaktionskolonne gewonnene Kondensationswärme wird erfindungsgemäß direkt oder indirekt, ganz oder teilweise zur Trennung des Dialkylcarbonats vom während der Reaktion gebildeten Alkylalkohol verwendet. Bevorzugt wird die durch Kondensation in dem oder den Kondensatoren, vorzugsweise Kopfkondensator(en), der weiteren Reaktionskolonne(n) und/oder dem oder den gegebenenfalls vorhandenen Zwischenkondensator(en) der ersten Reaktionskolonne gewonnene Kondensationswärme direkt oder indirekt teilweise zur Trennung des Dialkylcarbonats vom während der Reaktion gebildeten Alkylalkohol und teilweise zur Verdampfung des in die erste Reaktionskolonne eingeleiteten Dialkylcarbonats verwendet. In bevorzugten Ausführungsformen des erfindungsgemäßen Verfahrens wird die durch Kondensation in dem oder den Kondensator(en) der weiteren Reaktionskolonne(n) gewonnene Kondensationswärme direkt oder indirekt, ganz oder teilweise zur Trennung des Dialkylcarbonats vom während der Reaktion gebildeten Alkylalkohol verwendet und die durch Kondensation in dem oder den Zwischenkondensator(en) der ersten Reaktionskolonne gewonnene Kondensationswärme direkt oder indirekt, ganz oder teilweise zur Verdampfung des in die erste Reaktionskolonne eingeleiteten Dialkylcarbonats verwendet.

Im erfindungsgemäßen Verfahren fallen bei der Umesterung und/oder Disproprotionierung in der ersten Reaktionskolonne und/oder der oder den weiteren Reaktionskolonne(n) Ströme enthaltend während der Reaktion gebildeten Alkylalkohol (Reaktionsalkohol) sowie nicht umgesetztes oder während der Reaktion gebildetes Dialkylcarbonat an und werden vorzugsweise in einem oder mehreren Strömen im Gemisch entnommen. Dieses in den Reaktionskolonnen nicht umgesetzte oder während der Reaktion gebildete Dialkylcarbonat wird erfindungsgemäß ganz oder teilweise in wenigstens einem weiteren Verfahrensschritt enthaltend wenigstens eine Destillationskolonne vom während der Reaktion gebildeten Alkylalkohol (Reaktionsalkohol) getrennt. Bevorzugt wird wenigstens ein Strom enthaltend nicht umgesetztes oder während der Reaktion gebildetes Dialkylcarbonat und während der Reaktion gebildeten Alkylalkohol am Kopf der ersten Reaktionskolonne entnommen und zur Auftrennung wenigstens einem weiteren Verfahrensschritt enthaltend wenigstens eine Destillationskolonne zugeführt.

Bevorzugt wird das am Kopf der ersten Reaktionskolonne entnommene Dampfgemisch enthaltend Dialkylcarbonat und während der Reaktion gebildetem Alkylalkohol nach Kondensation am Kopf der ersten Reaktionskolonne ganz oder teilweise wenigstens einem weiteren Verfahrensschritt enthaltend wenigstens eine Destillationskolonne zur Trennung von Dialkylcarbonat und Alkylalkohol - im Folgenden als Trenn-Destillationskolonne(n) bezeichnet - zugeführt. Besonders bevorzugt wird das dabei abgetrennte Dialkylcarbonat gegebenenfalls nach weiterer Aufreinigung wieder der ersten Reaktionskolonne zugeführt.

Die Trennung des Dialkylcarbonats und des Reaktionsalkohols erfolgt bevorzugt destillativ in einer oder mehreren Trenn-Destillationskolonnen oder in einer Kombination aus Destillation und Membrantrennung - im Folgenden als Hybridprozess bezeichnet.

Bilden Reaktionsalkohol und Dialkylcarbonat ein Azeotrop (z.B. Methanol und Dimethylcarbonat) so wird bevorzugt ein wenigstens zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet. Besonders bevorzugt wird das Zweidruckverfahren oder ein Hybridverfahren angewendet. Ganz besonders bevorzugt wird das Zweidruckverfahren angewendet. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Reaktionsalkohol und Dialkylcarbonat kein Azeotrop (z.B. Ethanol und Diethylcarbonat), so erfolgt die Trennung bevorzugt in einer einzelnen Trenn-Destillationskolonne.

Bilden Reaktionsalkohol und Dialkylcarbonat ein Azeotrop, so weist das Destillat einer ersten Trenn-Destillationskolonne des Verfahrensschritts zur Trennung von Dialkylcarbonat und Alkylalkohol (Reaktionsalkohol) vorzugsweise nahezu azeotrope Zusammensetzung auf. In diesem Fall wird dieses bevorzugt in einem Zweidruckverfahren wenigstens einer weiteren Trenn-Destillationskolonne zugeführt die bei einem Betriebsdruck arbeitet, der unter dem der ersten Trenn-Destillationskolonne liegt. Durch den unterschiedlichen Betriebsdruck verschiebt sich die Lage des Azeotrops hin zu geringeren Anteilen an Reaktionsalkohol. Man erhält als Sumpfprodukt dieser zweiten oder weiteren Trenn-Destillationskolonne(n) Reaktionsalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Trenn-Destillationskolonne(n) wird in ganz besonders bevorzugten Ausführungsformen vorzugsweise mit der Kondensationswärme des oder der Kopfkondensator(en) der ersten Trenn-Destillationskolonne betrieben.

Beim Zweidruckverfahren macht man sich die Druckabhängigkeit der azeotropen Zusammensetzung eines Zweistoffgemisches zunutze. Bei einem Gemisch aus Reaktionsalkohol (Alkylalkohol) und Dialkylcarbonat, wie beispielsweise Methanol und Dimethylcarbonat, verschiebt sich die azeotrope Zusammensetzung mit zunehmendem Druck zu höheren Reaktionsalkoholgehalten. Führt man ein Gemisch dieser beiden Komponenten einer ersten Trenn-Destillationskolonne (Dialkylcarbonatkolonne) zu, wobei der Reaktionsalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltene azeotrope Gemisch wird einer weiteren Trenn-Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Reaktionsalkoholgehalten. Hierdurch ist es möglich, das in der Dialkylcarbonatkolonne erhaltene azeotrope Gemisch in ein Destillat mit nahezu azeotroper Zusammensetzung und nahezu reinen Reaktionsalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 2 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

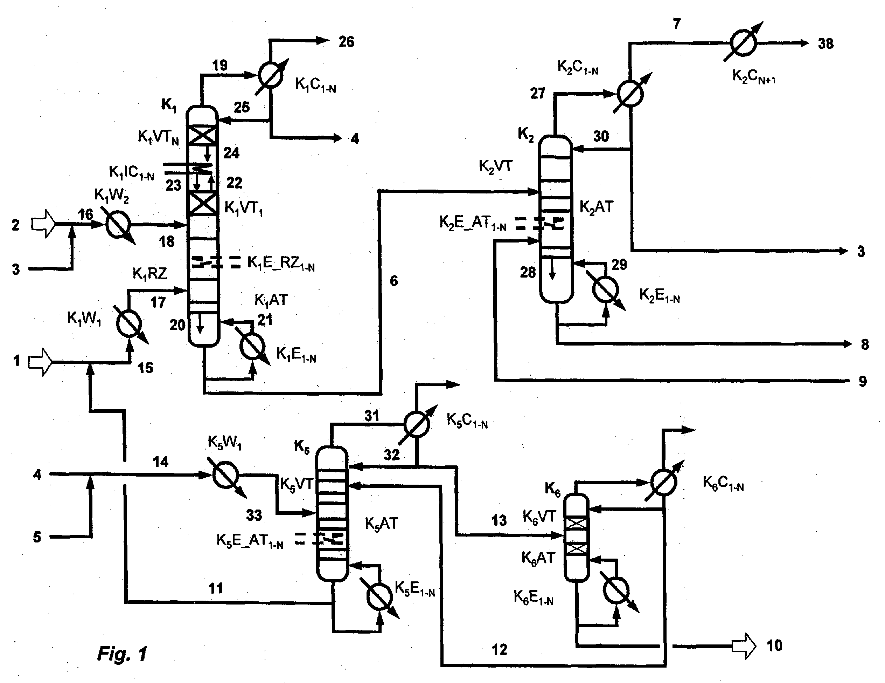

Eine beispielhafte Reaktionsführung bei der Trennung von Dialkylcarbonat und Reaktionsalkohol nach dem Zweidruckverfahren ist in Fig. 1 gezeigt.

Ein weiteres bevorzugtes Verfahren zur Trennung von Azeotropen aus Reaktionsalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Reaktionsalkohol und Dialkylcarbonat, wie beispielsweise Methanol und Dimethylcarbonat, erhält man bei Verwendung geeigneter Membranen mittels Pervaporation oder Dampfpermeation ein Reaktionsalkoholreiches Gemisch als Permeat und ein an Reaktionsalkohol verarmtes Gemisch als Retentat. Führt man ein Gemisch dieser beiden Komponenten einer Trenn-Destillationkolonne (Dialkylcarbonatkolonne) zu,
wobei der Reaktionsalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Reaktionsalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltene dampfförmige Gemisch wird gegebenenfalls nach Überhitzung einer Dampfpermation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Reaktionsalkoholreiche Fraktion mit einem Reaktionsalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Reaktionsalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltene Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Reaktionsalkoholreiche dampfförmige Fraktion mit einem Reaktionsalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Reaktionsalkoholanteil enthält, wird wieder der Trenn-Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervaporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Reaktionsalkohol erfolgt im Falle eines Hybridverfahrens besonders bevorzugt mittels einer Kombination aus Destillation und Dampfpermeation.

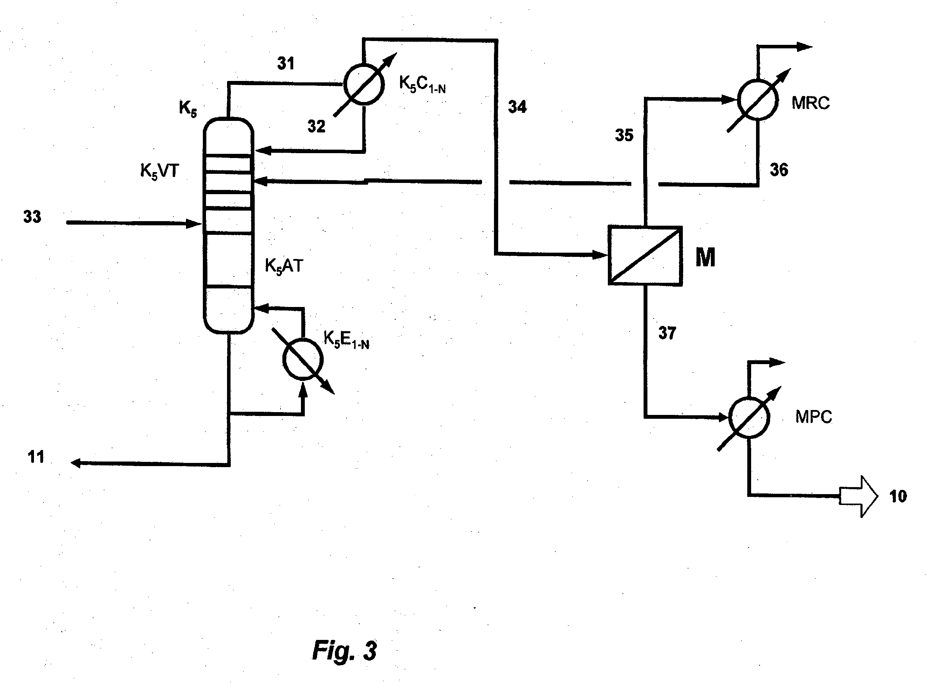

Eine beispielhafte Ausführung der Trennung von Dialkylcarbonat und Reaktionsalkohol nach dem Hybridverfahren mittels Dampfpermeation ist in Fig. 3 dargestellt.

Unabhängig vom gewählten Verfahren zur Trennung von Dialkylcarbonat und Reaktionsalkohol werden die Verfahrensbedingungen, wie Druck und Temperatur, erfindungsgemäß so gewählt, das die durch Kondensation in dem oder den Kondensator(en) der weiteren Reaktionskolonne(n) und/oder dem oder den gegebenenfalls vorhandenen Zwischenkondensator(en) der ersten Reaktionskolonne gewonnene Kondensationswärme effektiv genutzt werden kann.

Hierzu wird der Betriebsdruck und damit auch die Betriebstemperatur in der oder den Trenn-Destillationskolonne(n) des Verfahrensschritts zur Trennung von Dialkylcarbonat und Alkylalkohol so eingestellt, dass die Trenn-Destillationskolonne(n) ganz oder teilweise mit der Kondensationswärme in dem oder den Kondensator(en) der weiteren Reaktionskolonne(n) und/oder dem oder den gegebenenfalls vorhandenen Zwischenkondensator(en) der ersten Reaktionskolonne betrieben werden kann/können. Insbesondere wird hierzu der Betriebsdruck in der oder den Trenn-Destillationskolonne(n) des Verfahrensschritts zur Trennung von Dialkylcarbonat und Alkylalkohol so eingestellt, dass die Verdampungstemperatur im Sumpf der Trenn-Destillationskolonne(n) des Verfahrensschritts zur Trennung von Dialkylcarbonat und Alkylalkohol unter der Kondensationstemperatur in dem oder den Kondensator(en) der weiteren Reaktionskolonne(n) und/oder dem oder den gegebenenfalls vorhandenen Zwischenkondensator(en) der ersten Reaktionskolonne liegt.

Die zur Trennung von Reaktionsalkohol und Dialkylcarbonat erforderliche Wärme wird bei einer Temperatur zwischen 100 und 300 °C, vorzugsweise zwischen 100 und 230, und besonders bevorzugt zwischen 120 und 200 °C zugeführt. Um eine Wärmeintegration mit dem Zwischenkondensator der ersten Reaktionskolonne oder den Kondensatoren der zweiten Reaktionskolonne effizient zu ermöglichen, wird die Kondensation in dem oder den Kondensator(en) der weiteren Reaktionskolonne(n) und/oder dem oder den gegebenenfalls vorhandenen Zwischenkondensator(en) der ersten Reaktionskolonne bei einer um 1 bis 100 °C, bevorzugt 2 bis 50 °C und besonders bevorzugt 5 bis 40 °C erhöhten Temperatur durchgeführt.

Die Kondensationswärme aus dem oder den Kondensator(en) der weiteren Reaktionskolonne(n) und/oder dem oder den gegebenenfalls vorhandenen Zwischenkondensator(en) der ersten Reaktionskolonne kann dabei beispielsweise ganz oder teilweise zum Vorheizen von Zuströmen zu der oder den Trenn-Destillationskolonne(n) und/oder zur Erwärmung eines oder mehrerer Kolonnenabschnitte verwendet werden. In bevorzugten Ausführungsformen wird die Kondensationswärme aus dem oder den Kondensator(en) der weiteren Reaktionskolonne(n) und/oder dem oder den gegebenenfalls vorhandenen Zwischenkondensator(en) der ersten Reaktionskolonne teilweise zum Vorheizen des oder der Zuströme zu der oder den Trenn-Destillationskolonne(n) des Verfahrensschritts zur Trennung von Dialkylcarbonat und Alkylalkohol und teilweise zum Verdampfen des Sumpfes in der oder den Trenn-Destillationskolonne(n) verwendet. In einer ganz bevorzugten Ausführungsform des erfmdungsgemäßen Verfahrens, in der am Kopf der zweiten Reaktionskolonne eine Kaskade von wenigstens zwei, bevorzugt drei Kopfkondensatoren verwendet wird, dient die Kondensationswärme aus dem ersten Kondensator dieser Kaskade zur Verdampfung des Sumpfproduktes der oder der ersten Trenn-Destillationskolonne des Verfahrensschritts zur Trennung von Dialkylcarbonat und Alkylalkohol und die Kondensationswärme aus dem zweiten Kondensator der Kaskade zum Vorheizen des Zustroms zu der oder der ersten Trenn-Destillationskolonne des Verfahrensschritts zur Trennung von Dialkylcarbonat und Alkylalkohol.

Die Trenn-Destillationskolonne(n) verfügen bevorzugt über einen Verstärkungsteil mit 5 bis 40 theoretischen Stufen zur Aufkonzentrierung des Reaktionsalkohols und einen Abtriebsteil mit 5 bis 40 theoretischen Stufen zur Aufkonzentrierung des Dialkylcarbonates.

Durch die Nutzung der Kondensationswärme aus dem oder den Kondensator(en) der weiteren Reaktionskolonne(n) und gegebenenfalls dem oder den Zwischenkondensatoren der ersten Reaktionskolonne kann die Trennung des Reaktionsalkohol von überschüssigem Dialkylcarbonat bei deutlich reduziertem Energieverbrauch durchgeführt werden. Die Kühlleistung in den Umesterungsschritten kann dabei in gleichem Maße reduziert werden. Ein wesentlicher Vorteil des erfmdungsgemäßen Verfahrens gegenüber den Verfahren gemäß Stand der Technik liegt daher in der deutlichen Reduzierung des Energieverbrauchs bei der Herstellung von Diarylcarbonaten bzw. Alkylarylcarbonaten. Gleichzeitig kann das Verfahren mit einfachem apparativem Aufwand durchgeführt werden, da aufgrund der Verwendung von Kolonnenanordnungen keine komplizierte Reaktoranordnung mit mehreren getrennten und in Serie geschalteten Reaktionszonen erforderlich ist.

Das in der oder den weiteren Reaktionskolonne(n) aus Schritt (b) erhaltene Sumpfprodukt enthaltend Diarylcarbonat wird erfindungsgemäß wenigstens einem weiteren Verfahrensschritt zur Reinigung in wenigstens einer Destillationskolonne - im Folgenden auch erste Diarylcarbonat-Destillationskolonne genannt - enthaltend wenigstens einen Verstärkungsteil im oberen Teil der Kolonne und wenigstens einen Abtriebsteil im unteren Teil der Kolonne zugeführt. Bevorzugt wird dieser ersten Diarylcarbonat-Destillationskolonne ein Diarylcarbonat-haltiger Seitenstrom entnommen. Weiterhin bevorzugt enthält das in der oder den weiteren Reaktionskolonne(n) aus Schritt (b) erhaltene Sumpfprodukt enthaltend Diarylcarbonat Verbindungen mit einem Siedepunkt zwischen dem des Diarylcarbonats und dem des während der Herstellung des Diarylcarbonats als Zwischenprodukt gebildeten Alkylarylcarbonats als Verunreinigung, welche in einem weiteren Seitenstrom der Diarylcarbonat-Destillationskolonne entnommen und gegebenenfalls in die oder eine der weiteren Reaktionskolonne(n) aus Schritt (b) zurückgeführt werden.

Bevorzugt enthält das in der oder den weiteren Reaktionskolonne(n) aus Schritt (b) erhaltene Sumpfprodukt - auch als Rohdiarylcarbonat bezeichnet - 10 bis 90 Gew.-%, besonders bevorzugt 20 bis 80 Gew.-% und ganz besonders bevorzugt 40 bis 80 Gew.-% Diarylcarbonat sowie 5 bis 90 Gew.-%, besonders bevorzugt 5 bis 60 Gew.-% und ganz besonders bevorzugt 5 bis 40 Gew.-% Alkylarylcarbonat, 1 bis 90 Gew.-%, besonders bevorzugt 1 bis 50 Gew.-% und ganz besonders bevorzugt 1 bis 30 Gew.-% aromatische Hydroxyverbindung, 0 bis 5 Gew.-%, besonders bevorzugt 0 bis 2 Gew.-% und ganz besonders bevorzugt 0 bis 0,5 Gew.-% schwersiedende Nebenkomponenten, 0 bis 5 Gew.-%, besonders bevorzugt 0,0001 bis 2 Gew.-% und ganz besonders bevorzugt 0,0001 bis 1 Gew.-% mittelsiedende Nebenkomponenten und 0,01 bis 10 Gew.-%, besonders bevorzugt 0,1 bis 5 Gew.-% und ganz besonders bevorzugt 1 bis 5 Gew.-% Katalysator, wobei die Summe aller vorangehend genannten Komponenten im zu reinigenden Diarylcarbonat 100 Gew.-% ergibt. Die Gew.-%-Angaben sind jeweils bezogen auf das gesamte Gewicht des zu reinigenden Rohdiarylcarbonats.

Mit dem erfindungsgemäßen Verfahren können vorzugsweise Diarylcarbonate mit einer Reinheit von, d.h. einem Gehalt von reinem Diarylcarbonat von, 99 bis 100 Gew.-%, besonders bevorzugt 99,5 bis 100 Gew.-% und ganz besonders bevorzugt 99,9 bis 100 Gew.-%, bezogen auf das Gesamtgewicht des aufgereinigten Diarylcarbonats erhalten werden.

Das im Seitenstrom der ersten Diarylcarbonat-Destillationskolonne entnommene Diarylcarbonat kann flüssig oder dampfförmig entnommen werden. Bevorzugt wird das im Seitenstrom der ersten Diarylcarbonat-Destillationkolonne entnommene Diarylcarbonat dampfförmig entnommen. In bevorzugten Ausführungsformen kann jedoch die flüssige Entnahme des Diarylcarbonats im Seitenstrom insbesondere aufgrund konstruktiver Gegebenheiten bevorzugt sein.

Die erste Diarylcarbonat-Destillationskolonne weist mindestens zwei Sektionen, d.h. einen Verstärkungsteil im oberen Teil der Kolonne und einen Abtriebsteil im unteren Teil der Kolonne auf. Bevorzugt kann der Verstärkungsteil der ersten Diarylcarbonat-Destillationskolonne in einen unteren und einen oberen Verstärkungsteil unterteilt sein. Weiterhin bevorzugt kann der Abtriebsteil der ersten Diarylcarbonat-Destillationskolonne in einen unteren und einen oberen Abtriebsteil unterteilt sein.

In einer bevorzugten Ausführungsform des erfindungsgemäßen Verfahrens wird die Reinigung des in der oder den weiteren Reaktionskolonne(n) aus Schritt (b) erhaltene Sumpfprodukt enthaltend Diarylcarbonat in mindestens einer Diarylcarbonat-Destillationskolonne durchgeführt, welche wenigstens drei Sektionen aufweist. Bei diesen wenigstens drei Sektionen handelt es sich um wenigstens einen Verstärkungsteil und wenigstens einem Abtriebsteil, wobei der Abtriebsteil in einen unteren und einen oberen Abtriebsteil aufgeteilt ist. Besonders bevorzugt weist die erste Diarylcarbonat-Destillationskolonne mit einem Verstärkungsteil und einem Abtriebsteil, wobei der Abtriebsteil in einen unteren und einen oberen Abtriebsteil aufgeteilt ist, vier Sektionen auf, wobei auch der Verstärkungsteil in einen unteren und oberen Verstärkungsteil aufgeteilt ist.

Ingesamt weist die erste Diarylcarbonat-Destillationskolonne bevorzugt eine Gesamttrennleistung von 3 bis 160, besonders bevorzugt von 10 bis 90, ganz besonders bevorzugt von 13 bis 50 theoretische Stufen auf. Der obere Verstärkungsteil weist dabei bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der obere Abtriebsteil von bevorzugt 1 bis 40, besonders bevorzugt 2 bis 30, ganz besonders bevorzugt 5 bis 20 theoretische Stufen und der untere Abtriebsteil von bevorzugt 1 bis 40, besonders bevorzugt 2 bis 20, ganz besonders bevorzugt 2 bis 15 theoretische Stufen auf.

Die Verdampfung erfolgt vorzugsweise in einem Temperaturbereich von 100 bis 300°C, bevorzugt von 150 bis 240°C und besonders bevorzugt von 180 bis 230°C im Sumpf der Kolonne. Die Kondensation der Dämpfe am Kopf der Kolonne kann ein oder mehrstufig, bevorzugt ein- oder zweistufig, in einem Temperaturbereich von vorzugsweise 40 bis 250°C, bevorzugt 50 bis 200°C und besonders bevorzugt 60 bis 180°C erfolgen.

Die erste Diarylcarbonat-Destillationskolonne wird bevorzugt bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis beträgt dabei bevorzugt 0,1 bis 10, besonders bevorzugt 0,5 bis 5 und ganz besonders bevorzugt 0,5 bis 2.

In bevorzugten Ausführungsformen des erfindungsgemäßen Verfahrens kann es sich bei der ersten Diarylcarbonat-Destillationskolonne um eine Trennwandkolonne handeln.

Trennwandkolonnen eignen sich ebenfalls zur Trennung eines Gemisches in drei Fraktionen, d.h. Kopfprodukt, Sumpfprodukt und Seitenstrom mit hoher Reinheit. Die Trennwandkolonne verfügt über eine in der Regel vertikal angeordnete Trennwand, welche die Zulaufseite von der Entnahmeseite für den Seitenstrom voneinander trennt. Die Trennwand ist dabei bevorzugt nicht über die gesamte Länge der Kolonne durchgängig. Meist befindet sich oberhalb der Trennwand noch ein Verstärkungsteil und unterhalb der Trennwand noch ein Abtriebsteil. Im Bereich der Trennwand befinden sich sowohl auf der Zulauf- als auch auf der Entnahmeseite für den Seitenstrom bevorzugt mindestens 2 Sektionen. Auf der Zulaufseite dient eine obere Sektion zur Reduktion der im Zulauf enthaltenen Schwersieder und eine untere Sektion zur Reduktion der im Zulauf enthaltenen Leichtsieder. Auf der Entnahmeseite für den Seitenstrom befindet sich ebenfalls eine obere Sektion oberhalb der Entnahme, welche zur Reduzierung der Leichtsieder aus dem Verstärkungsteil dient. Eine untere Sektion, die unterhalb der Entnahme angeordnet ist dient der Reduzierung von Schwersiedern, welche aus dem Abtriebsteil unterhalb der Trennwand kommen.

Die Aufteilung der aus dem Verstärkungsteil ablaufenden Flüssigkeit richtet sich nach den Anforderungen an die spezielle Trennaufgabe und kann durch konstruktive als auch regelungstechnische Maßnahmen beeinflusst werden. Gleiches gilt für den, aus dem Abtriebsteil kommenden, Dampfstrom.

Trennwandkolonnen sind dem Fachmann bekannt und beispielsweise in DE-A 33 02 525 oder DE-A 199 14 966 beschrieben.

Bevorzugt weist die Trennwandkolonne zusätzlich zu wenigstens einem Verstärkungsteil im oberen Teil der Kolonne und wenigstens einem Abtriebsteil im unteren Teil der Kolonne jeweils eine obere und untere Sektion auf der Zulaufseite der Trennwand und auf der Entnahmeseite der Trennwand auf, wobei Zulauf und Entnahme jeweils zwischen der oberen und unteren Sektion erfolgen.

In besonders bevorzugten Ausführungsformen des erfindungsgemäßen Verfahrens weist der Verstärkungsteil der Trennwandkolonne zwei Sektionen, d.h. einen unteren Verstärkungsteil und einen oberen Verstärkungsteil, auf.

In ganz besonders bevorzugten Ausführungsformen des erfindungsgemäßen Verfahrens weist die Trennwandkolonne mindestens sieben Sektionen auf, umfassend mindestens einem Abtriebsteil im unteren Teil der Kolonne, jeweils eine obere und untere Sektion auf der Zulaufseite der Trennwand und auf der Entnahmeseite der Trennwand sowie einen oberen und unteren Verstärkungsteil im oberen Teil der Kolonne auf.

Die Trennwand in einer solchen Trennwandkolonne ist bevorzugt in Längsrichtung der Kolonne angeordnet. Sie verhindert sowohl dampfseitigen als auch flüssigkeitsseitigen Stoffaustausch zwischen Zulauf- und Entnahmeseite.

Die Entnahme des gereinigten Diarylcarbonats auf der Entnahmeseite der Trennwand kann flüssig oder dampfförmig erfolgen. Beim Kolonnendesign kann die Art der Entnahme die Anordnung der Trennwand innerhalb der Kolonne mitunter deutlich beeinflussen. Die Trennwand kann jeweils zur Entnahmeseite oder zur Zulaufseite verschoben in der Kolonne angeordnet sein und so den Querschnitt der jeweiligen Seite gegenüber der anderen verkleinern oder vergrößern. Im Falle dampfförmiger Entnahme des gereinigten Diarylcarbonats auf der Entnahmeseite der Trennwand ist der Querschnitt der Entnahmeseite der Kolonne bevorzugt größer als der Querschnitt der Zulaufseite, d.h. es gelangt mehr Dampf aus dem Abtriebsteil in die Entnahmeseite. Im Falle flüssiger Entnahme des gereinigten Diarylcarbonats auf der Entnahmeseite der Trennwand ist der Querschnitt der Zulaufseite der Kolonne bevorzugt identisch zum Querschnitt der Entnahmeseite.

Der obere Verstärkungsteil einer solchen Trennwandkolonne weist bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der Abtriebsteil von bevorzugt 1 bis 40, besonders bevorzugt 2 bis 20, ganz besonders bevorzugt 2 bis 15 theoretische Stufen auf. Die obere Sektion und untere Sektion auf der Zulaufseite der Trennwand sowie die obere Sektion und untere Sektion auf der Entnahmeseite der Trennwand weisen bevorzugt jeweils eine Trennleistung von 1 bis 40, besonders bevorzugt 2 bis 20, ganz besonders bevorzugt 5 bis 20 theoretische Stufen auf.

Das Sumpfprodukt der ersten Diarylcarbonat-Destillationskolonne hat einen Restgehalt an Diarylcarbonat von unter 95 Gew.-% bevorzugt unter 90 Gew.-% und besonders bevorzugt unter 75 Gew.-%.

Zur Vermeidung von Katalysatorverlusten kann das Sumpfprodukt der ersten Diarylcarbonat-Destillationskolonne ganz oder teilweise, bevorzugt zu mindestens 50 %, besonders bevorzugt zu mindestens 80 % und ganz besonders bevorzugt zu mindestens 90 % wieder in die Umesterung aus wenigstens einem Dialkylcarbonat und wenigstens einer aromatischen Hydroxyverbindung zurückführt werden. Der restliche Teil des Sumpfproduktes der ersten Diarylcarbonat-Destillationskolonne kann einer Aufarbeitungsstufe, im Folgenden als Rückstandsaufkonzentrierung bezeichnet, zwecks Aufkonzentrierung des Rückstands und teilweisen Rückgewinnung des im Sumpfprodukt der ersten Diarylcarbonat-Destillationskolonne noch enthaltenen Diarylcarbonats zugeführt werden. Das in der Rückstandsaufkonzentrierung zurückgewonnene Diarylcarbonat kann in einer besonderen Ausführungsform des Verfahrens flüssig oder dampfförmig, bevorzugt dampfförmig, wieder der ersten Diarylcarbonat-Destillationskolonne zugeführt. Der aufkonzentrierte Rückstand aus der Rückstandsaufkonzentrierung kann entweder aus dem Verfahren ausgeschleust oder einer weiteren Aufarbeitungsstufe zwecks Rückgewinnung des Katalysators zugeführt werden. Bevorzugt kann daher aus dem Sumpfprodukt wenigstens einer Diarylcarbonat-Destillationskolonne aus Verfahrensschritt (e) ein katalysatorhaltiger Strom erhalten werden, welcher ganz oder teilweise gegebenenfalls nach weiterer Aufreinigung wieder in das Verfahren, bevorzugt in Verfahrensschritt (a) zurückführt wird. Dadurch können sowohl Verluste teurer Katalysatoren als auch Verluste an gewünschten Diarylcarbonat vermieden und damit das erfindungsgemäße Verfahren zusätzlich wirtschaftlicher gestaltet werden.

In einer bevorzugten Ausführungsform des erfindungsgemäßen Verfahrens werden
(f) aus dem Sumpfprodukt wenigstens einer Diarylcarbonat-Destillationskolonne aus Verfahrensschritt (e) ein katalysatorhaltiger Strom erhalten, welcher ganz oder teilweise gegebenenfalls nach weiterer Aufreinigung wieder in das Verfahren, bevorzugt in Verfahrensschritt (a) zurückführt wird,
(g) aus wenigstens einer Diarylcarbonat-Destillationskolonne aus Verfahrensschritt (e) ein aromatische Hydroxyverbindundung(en) und Alkylarylcarbonat enthaltender Strom erhalten, welcher ganz oder teilweise wieder in das Verfahren, bevorzugt in Verfahrensschritt (a) oder (b) zurückführt wird, und
(h) aus wenigstens einer Diarylcarbonat-Destillationskolonne aus Verfahrensschritt (e) zusammen oder getrennt voneinander Verbindungen mit einem Siedepunkt oberhalb des Siedepunktes des Diarylcarbonats und Verbindungen, deren Siedepunkt zwischen dem des Dialkylcarbonats und dem des während der Herstellung des Diarylcarbonats gebildeten Alkylarylcarbonats liegt, ganz oder teilweise aus dem Verfahren ausgeschleust.

Die unter (h) beschriebene Ausschleusung kann vorzugsweise als flüssiger Seitenstrom aus dem Verstärkungsteil wenigstens einer Diarylcarbonat-Destillationskolonne und/oder eines Teilstrom des Destillats dieser Kolonne erfolgen.

Der am Kopf wenigstens einer Reaktionskolonne aus (b) gegebenenfalls nach Kondensation in wenigstens einem Kondensator entnommene aromatische Hydroxyverbindung(en) enthaltende Dampf wird erfindungsgemäß ganz oder teilweise wenigstens einem weiteren Verfahrensschritt enthaltend wenigstens eine Destillationskolonne zugeführt, wobei Verbindungen, deren Siedepunkt zwischen dem des Dialkylcarbonats und dem des während der Herstellung des Diarylcarbonats gebildeten Alkylarylcarbonats liegt - im Folgenden auch mittelsiedende Nebenverbindungen genannt - abgetrennt werden. Die in diesem Verfahrensschritt eingesetzte(n) Destillationskolonne(n) zur Abtrennung von Verbindungen, deren Siedepunkt zwischen dem des Dialkylcarbonats und dem des während der Herstellung des Diarylcarbonats gebildeten Alkylarylcarbonats liegt, werden im Folgenden auch als Zwischensieder-Destillationskolonnen bezeichnet.

In einer bevorzugten Ausführungsform des erfindungsgemäßen Verfahrens wird der am Kopf wenigstens einer Reaktionskolonne aus (b) gegebenenfalls nach Kondensation in wenigstens einem Kondensator entnommene aromatische Hydroxyverbindung(en) enthaltende Dampf wenigstens einem weiteren Verfahrensschritt enthaltend wenigstens zwei Zwischensieder-Destillationskolonnen zugeführt wird, wobei das Sumpfprodukt der ersten Zwischensieder-Destillationskolonne einer zweiten Zwischensieder-Destillationskolonne zugeführt wird.

Bevorzugt wird (werden) die aus dem oder den Verfahrensschritt(en) zur Abtrennung von Verbindungen, deren Siedepunkt zwischen dem des Dialkylcarbonats und dem des während der Herstellung des Diarylcarbonats gebildeten Alkylarylcarbonats liegt, aus dem am Kopf wenigstens einer Reaktionskolonne aus (b) gegebenenfalls nach Kondensation in wenigstens einem Kondensator ganz oder teilweise entnommenen aromatische Hydroxyverbindung(en) enthaltenden Dampf erhaltene(n) aromatische(n) Hydroxyverbindung(en) wieder der ersten Reaktionskolonne zugeführt. Dabei wird (werden) die nach der Abtrennung erhaltene(n) aromatische(n) Hydroxyverbindung(en) vorzugsweise einer ersten und einzigen Zwischensieder-Destillationskolonne als Sumpfprodukt oder einer zweiten oder weiteren Zwischensieder-Destillationskolonne als Seitenstrom oder Sumpfprodukt entnommen.

Bevorzugt enthält dabei das am Kopf der ersten Zwischensieder-Destillationskolonne entnommene Produkt Dialkylcarbonat und wird ganz oder teilweise dem Verfahrensschritt (c) enthaltend wenigstens eine Trenn-Destillationskolonne zur Abtrennung des Alkylalkohols zugeführt.

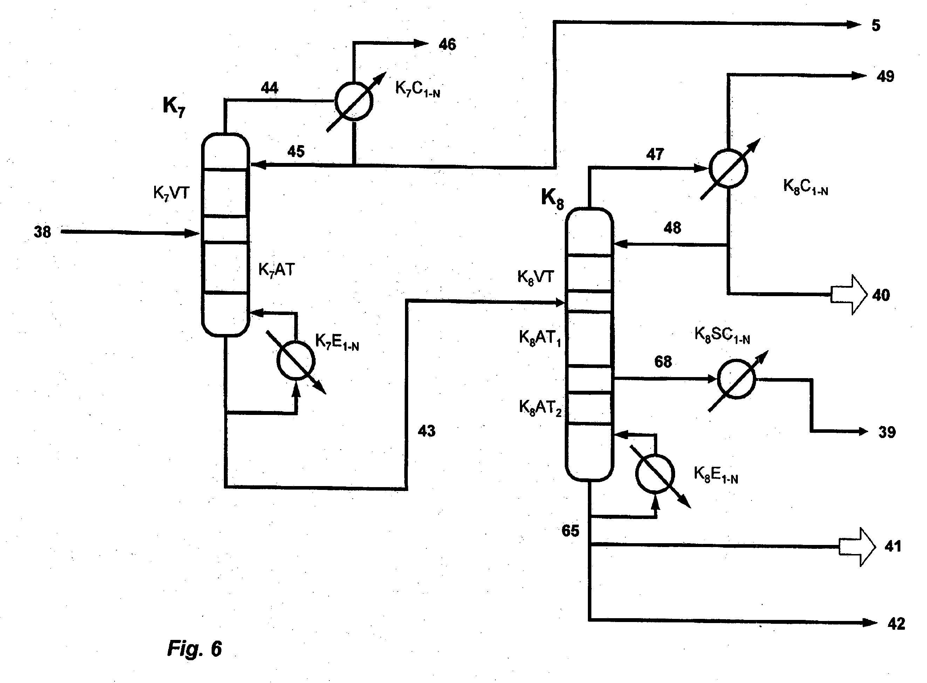

In einer bevorzugten Ausführungsform werden in der ersten Zwischensieder-Destillationskolonne(n) Reaktionsalkohol, Dialkylcarbonat und ggf. ein Teil der mittelsiedenden Nebenkomponenten als Kopfprodukt abgetrennt. (vgl. z.B. Fig. 6). In diesem Falle verfügt diese bevorzugt über einen Verstärkungsteil mit 5 bis 40 theoretischen Stufen zur Aufkonzentrierung des Reaktionsalkohols und des Dialkylcarbonats und einen Abtriebsteil mit 5 bis 40 theoretischen Stufen zur Aufkonzentrierung der mittelsiedenden Nebenverbindungen. Der Betriebsdruck liegt vorzugsweise zwischen 0,05 und 3 bar absolut, besonders bevorzugt zwischen 0,1 und 2 bar absolut und ganz besonders bevorzugt zwischen 0,5 und 1,5 bar absolut. Das Rücklaufverhältnis beträgt vorzugsweise 0,1 bis 10, besonders bevorzugt 0,5 bis 5 und ganz besonders bevorzugt 0,5 bis 2.

Im Falle der oben genannten bevorzugten Ausführungsform der ersten Zwischensieder-Destillationskolonne wird die aromatische Hydroxyverbindung als Sumpfprodukt oder Seitenstrom, besonders bevorzugt als Seitenstrom, mittelsiedende Nebenverbindungen mit einem Siedepunkt oberhalb der aromatischen Hydroxyverbindung am Sumpf und mittelsiedende Nebenverbindungen mit einem Siedepunkt unterhalb der aromatischen Hydroxyverbindung als Destillat entnommen. Im Falle dieser bevorzugten Ausführung verfügt die Kolonne vorzugsweise über einen Verstärkungsteil mit mindestens einer Sektion und einer Trennleistung von 5 bis 40 theoretischen Stufen, einem Abtriebsteil mit bevorzugt mindestens einer, besonders bevorzugt mit mindestens 2 Sektionen mit einer Trennleistung von 5 bis 60 theoretischen Stufen. Der Betriebsdruck liegt vorzugsweise zwischen 0,05 und 3 bar absolut, besonders bevorzugt zwischen 0,1 und 2 bar absolut und ganz besonders bevorzugt zwischen 0,5 und 1,5 bar absolut. Das Rücklaufverhältnis beträgt vorzugsweise 1 bis 1000, besonders bevorzugt 10 bis 500 und ganz besonders bevorzugt 50 bis 200.

Im Falle einer besonders bevorzugten Ausführungsform der zweiten Zwischensieder-Destillationskolonne wird die aromatische Hydroxyverbindung als dampfförmiger Seitenstrom entnommen. Die bei der Kondensation des dampfförmigen Seitenstroms anfallende Wärme kann entweder zur Erzeugung eines Wärmeträgermediums oder direkt zur Beheizung anderer Verfahrensabschnitte zur Herstellung von Diarylcarbonaten genutzt werden.

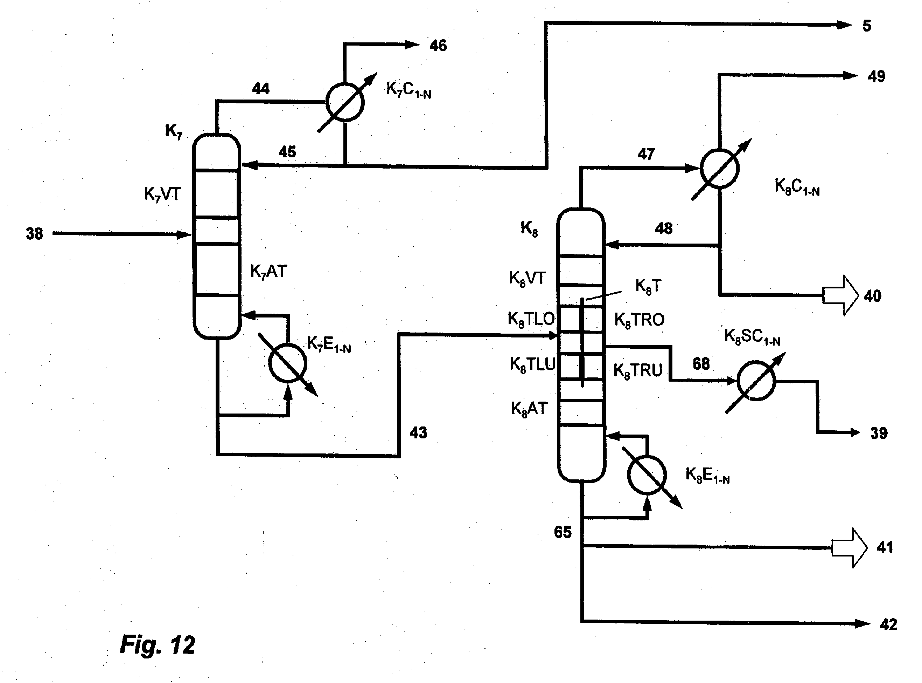

In einer weiteren bevorzugten Ausführungsform erfolgt die Abtrennung der aromatischen Hydroxyverbindung in der zweiten Zwischensieder-Destillationskolonne mit einer Trennwandkolonne (vgl. z.B. Fig. 12). In Falle einer solchen Ausführungsform des erfindungsgemäßen Verfahrens weist die Trennwandkolonne bevorzugt mindestens sechs Sektionen auf, umfassend mindestens einem Abtriebsteil im unteren Teil der Kolonne, jeweils eine obere und untere Sektion auf der Zulaufseite der Trennwand und auf der Entnahmeseite der Trennwand sowie einen Verstärkungsteil im oberen Teil der Kolonne auf. Die Sektionen haben vorzugsweise eine Trennleistung von 5 bis 40 Stufen, wobei die Sektionen hinsichtlich ihrer Trennleistung unterschiedlich ausgeführt sein können.

Die Trennwand in einer solchen Trennwandkolonne ist bevorzugt in Längsrichtung der Kolonne angeordnet. Sie verhindert sowohl dampfseitigen als auch flüssigkeitsseitigen Stoffaustausch zwischen Zulauf und Entnahmeseite.

Bilden Dialkylcarbonat und aromatische Hydroxyverbindung ein Maximumazeotrop, so kann für die Abtrennung der mittelsiedenden Nebenverbindungen eine andere Trennsequenz vorteilhaft sein. In einer solchen weiteren Ausführungsform der ersten Zwischensieder-Destillationskolonne werden Reaktionsalkohol, Dialkylcarbonat und mittelsiedende Nebenverbindungen als Destillat und ein azeotropes Gemisch aus Dialkylcarbonat und aromatischer Hydroxyverbindung als Sumpfprodukt entnommen.

Im Falle dieser Ausführung verfügt die erste Zwischensieder-Destillationskolonne vorzugsweise über einen Verstärkungsteil mit mindestens einer Sektion und einer Trennleistung von 5 bis 40 theoretischen Stufen, einem Abtriebsteil mit mindestens einer Sektion und einer Trennleistung von 5 bis 40 theoretischen Stufen. Der Betriebsdruck liegt vorzugsweise zwischen 0,05 und 3 bar absolut, besonders bevorzugt zwischen 0,1 und 2 bar absolut und ganz besonders bevorzugt zwischen 0,1 und 1,5 bar absolut. Das Rücklaufverhältnis beträgt vorzugsweise 0,1 bis 10, bevorzugt 0,2 bis 5 und besonders bevorzugt zwischen 0,4 und 2.

Im Falle einer Abtrennung von Dialkylcarbonat, Reaktionsalkohol und mittelsiedenden Nebenverbindungen als Destillat der ersten Zwischensieder-Destillationskolonne erfolgt die Abtrennung der mittelsiedenden Nebenverbindungen bevorzugt in einer zweiten Zwischensieder-Destillationskolonne als Sumpfprodukt. In diesem Falle wird als Destillat der zweiten Zwischensieder-Destillationskolonne ein Gemisch aus Reaktionsalkhol und Dialkylcarbonat erhalten. Im Falle dieser Ausführungsform verfügt die zweite Zwischensieder-Destillationskolonne vorzugsweise ebenfalls über einen Verstärkungsteil mit mindestens einer Sektion und einer Trennleistung von 5 bis 40 theoretischen Stufen, einem Abtriebsteil mit mindestens einer Sektion und einer Trennleistung von 5 bis 40 theoretischen Stufen. Der Betriebsdruck liegt vorzugsweise zwischen 0,05 und 10 bar absolut, besonders bevorzugt zwischen 0,05 und 2 bar absolut und ganz besonders bevorzugt zwischen 0,08 und 1 bar absolut. Das Rücklaufverhältnis beträgt vorzugsweise 0,1 bis 10, bevorzugt 0,2 bis 5 und besonders bevorzugt zwischen 0,4 und 2.

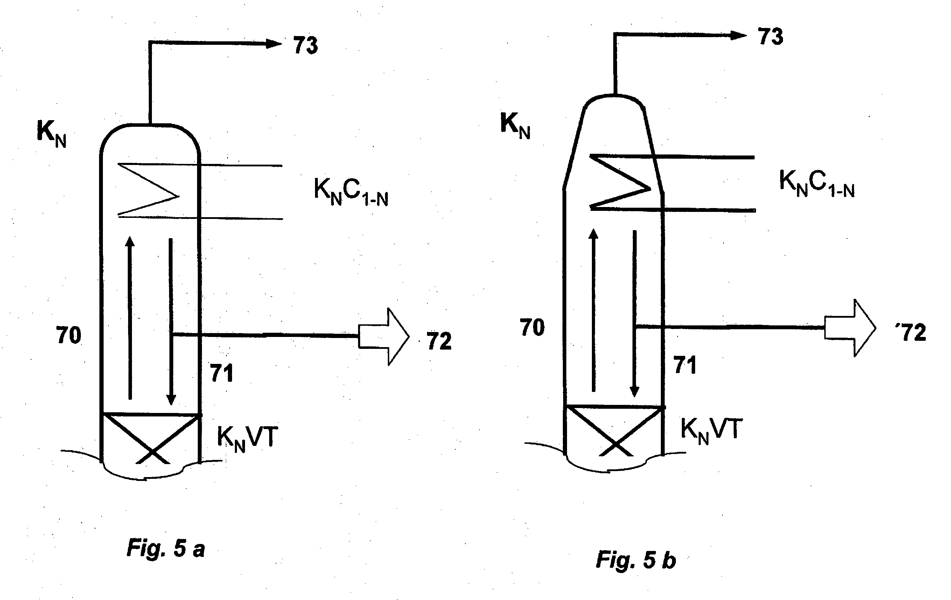

In bevorzugten Ausführungsformen des erfindungsgemäßen Verfahrens kann wenigstens eine der im Verfahren eingesetzten Reaktionskolonnen und/oder wenigstens eine der im Verfahren eingesetzten Destillationskolonnen einen oder mehrere Kopfkondensatoren aufweisen, welche in die Kolonne integriert sind, wobei das Verhältnis d/D von Durchmesser der Dampfleitung von der Kolonne zu dem oder den Kopfkondensator(en) (d) zu Kolonnendurchmesser der Kolonne (D) im Bereich von 0,2 bis 1, bevorzugt im Bereich von 0,5 bis 1 liegt. In einer besonders bevorzugten Ausführungsform kann der Kopfkondensator in die Destillationskolonne integriert sein, so dass zwischen Destillationskolonne und Kopfkondensator keine Dampfleitung erforderlich ist. Das Verhältnis d/D beträgt in diesem Fall 1. Dabei kann der Kolonnenquerschnitt nach Eintritt in den Kopfkondensator unter Umständen auch dem Kondensationsfortschritt angepasst werden. Solche bevorzugten Ausführungsformen sind ausschnittsweise und beispielhaft für eine Diarylcarbonat-Destillationskolonne in den Fig. 5a und 5b dargestellt. Entsprechende Anordnungen sind auch für die anderen im Verfahren eingesetzten Destillationskolonnen und/oder Reaktionskolonnen möglich. Bevorzugt weisen mehrere der im Verfahren eingesetzten Reaktionskolonnen und/oder Destillationskolonnen einen der vorangehend genannten Kopfkondensatoren auf.

Bei der in Fig. 5a dargestellten Ausführungsform bleibt der Kolonnendurchmesser im Bereich der Kondensation unverändert. Die aus dem Verstärkungsteil, falls vorhanden bevorzugt dem oberen Verstärkungsteil K_{N}VT, aufsteigenden Dämpfe (70) werden in dem oder den integrierten Kopfkondensator(en) K_{N}C_{1-N} kondensiert. Ein Teil des Kondensates wird als Rücklauf (71) wieder auf die obere Kolonnensektion aufgegeben. Der restliche Teil des Kondensats wird als Destillat (72) aus der Kolonne ausgeschleust. Inerte und/oder nicht kondensierte Dämpfe (73) werden am Kopf der Kolonne entnommen.

Bei einigen Kondensatorformen kann es von Vorteil sein, den Kolonnenquerschnitt variabel zu gestalten. Ist die Führung der zu kondensierenden Dämpfe beispielsweise von unten nach oben, so nimmt die Dampfmenge nach oben hin ab. Durch eine Reduktion des Kolonnendurchmessers in Richtung Kolonnenkopf, wird der für den Dampf verfügbare Kolonnenquerschnitt der nach oben hin abnehmenden Dampfmenge angepasst. Eine solche Ausführungsform ist beispielhaft in Fig. 5b dargestellt. Dabei muss die Entnahme der nicht kondensierten Dämpfe nicht zwangsläufig oben erfolgen. Wird beispielsweise eine Konstruktion gewählt, bei der ein Platten- oder Rohrbündel von oben in die Kolonne einfügt wird, so kann sich die Entnahme der nicht kondensierten Dämpfe auch seitlich befinden.

In bevorzugten Ausführungsformen des erfindungsgemäßen Verfahrens können Leitungen und Aggregate, die Gemische führen, welche einen Festpunkt von mehr als 30°C, bevorzugt mehr als 40°C aufweisen, auf Temperaturen oberhalb dieses Festpunktes, bevorzugt auf Temperaturen von mehr als 1°C oberhalb dieses Festpunktes, besonders bevorzugt auf Temperaturen von mehr als 5°C oberhalb dieses Festpunktes beheizt werden. Dadurch werden Ausfällungen von Feststoffen innerhalb dieser Leitungen und Aggregate vermieden und die Wiederinbetriebnahme der entsprechenden Anlagen nach Stillständen erheblich erleichtert.

Das erfindungsgemäße Verfahren wird bevorzugt kontinuierlich durchgeführt.

Das erfindungsgemäße Verfahren wird beispielhaft anhand der Figuren 1 bis 14 beschrieben.
Fig. 1 beschreibt einen ersten Umesterungsschritt mittels Reaktivrektifikation in einer ersten Reaktionskolonne mit Zwischenkondensator im Allgemeinen, einen zweiten Reaktionsschritt zur Umesterung bzw. zur Disproportionierung von Alkylarylcarbonat in einer zweiten Reaktionskolonne und eine Trennung des in der ersten Reaktionskolonne als Kopfprodukt anfallenden Gemisches enthaltend Dialkylcarbonat und Reaktionsalkohol in einem weiteren Verfahrensschritt enthaltend wenigstens eine Trenn-Destillationskolonne.
Fig. 2 beschreibt eine besonders bevorzugte Ausführungsform einer ersten Reaktionskolonne (Reaktivrektifikationskolonne) mit externer Anordnung eines Zwischenkondensators und Kombination mit der Verdampfung des Dialkylcarbonats zur Rückführung der anfallenden Kondensationswärme.
Fig. 3 beschreibt eine bevorzugte Ausführungsform der Trennung von Dialkylcarbonat und Reaktionsalkohol nach dem Hybridverfahren.
Fig. 4 beschreibt eine bevorzugte Ausführungsform des Verfahrensabschnitts (e) zur Reinigung des in einer zweistufigen Reaktion erhaltenen Diarylcarbonats
Fig. 5a beschreibt ausschnittweise die Kondensation am Kopf einer im Verfahren verwendeten Reaktions- oder Destillationskolonne, wobei der Kolonnendurchmesser im Bereich der Kondensation unverändert bleibt.
Fig. 5b beschreibt ausschnittweise die Kondensation am Kopf einer im Verfahren verwendeten Reaktions- oder Destillationskolonne, mit einer Reduktion des Kolonnendurchmessers in Richtung Kolonnenkopf.
Fig. 6 beschreibt den Verfahrensabschnitt (d) zur Abtrennung von mittelsiedenden Nebenverbindungen, deren Siedepunkt zwischen dem des eingesetzten Dialkylcarbonats und dem des in der Reaktion gebildeten Alkylarylcarbonats liegt.
Fig. 7 beschreibt eine bevorzugte Ausführungsform des Verfahrensabschnitts (e) zur Reinigung des in einer wenigstens zweistufigen Reaktion erhaltenen Diarylcarbonats, wobei Verfahrensabschnitt (e) nur eine einzige Destillationskolonne aufweist.
Fig. 8 beschreibt eine weitere bevorzugte Ausführungsform des Verfahrensabschnitts (e) zur Reinigung des in einer wenigstens zweistufigen Reaktion erhaltenen Diarylcarbonats mit integrierter Seitenstromkolonne.
Fig. 9 beschreibt eine weitere bevorzugte Ausführungsform des Verfahrensabschnitts (e) zur Reinigung des in einer wenigstens zweistufigen Reaktion erhaltenen Diarylcarbonats mit Seitenstromkolonne, wobei die Seitenstromkolonne sowohl über einen Verstärkungsteil als auch einen Abtriebsteil verfügt.
Fig. 10 beschreibt eine weitere bevorzugte Ausführungsform des Verfahrensabschnitts (e) zur Reinigung des in einer wenigstens zweistufigen Reaktion erhaltenen Diarylcarbonats wobei Destillationskolonne als Trennwandkolonne mit flüssiger Seitenstromentnahme ausgeführt ist.
Fig. 11 beschreibt eine weitere bevorzugte Ausführungsform des Verfahrensabschnitts (e) zur Reinigung des in einer wenigstens zweistufigen Reaktion erhaltenen Diarylcarbonats wobei Destillationskolonne als Trennwandkolonne mit dampfförmiger Seitenstromentnahme ausgeführt ist.
Fig. 12: beschreibt eine bevorzugte Ausführungsform des Verfahrensabschnitts (d) zur Abtrennung von mittelsiedenden Nebenverbindungen, deren Siedepunkt zwischen dem des eingesetzten Dialkylcarbonats und dem des in der Reaktion gebildeten Alkylarylcarbonats liegt, bei der die zweite Zwischensieder-Destillationskolonne als Trennwandkolonne ausgeführt ist.
Fig. 13 beschreibt ausschnittsweise eine weitere bevorzugte Ausführungsform der Kondensation am Kopf einer im Verfahren verwendeten Reaktions- oder Destillationskolonne, wobei die Kondensation in einer weiteren Kolonnensektion und die Abführung der Kondensationswärme über einen externen Wärmetauscher erfolgt.
Fig. 14 beschreibt eine bevorzugte Ausführungsform des gesamten Verfahrens mit Verfahrensschritten (a) bis (e).

Die Figuren dienen der beispielhaften Erläuterung der Erfindung und sind nicht als Beschränkung aufzufassen.

In den Fig. 1 bis 14 bedeuten:
- K₁: Alkylarylcarbonat-Reaktionskolonne (erste Reaktionskolonne)
- K₁C_{1-N}: Kopfkondensator(en) 1 bis N der K₁
- K₁E_{1-N}: Verdampfer 1 bis N der K₁
- K₁IC_{1-N}: Zwischenkondensator(en) 1 bis N der K₁
- K₁VT₁: unterer Verstärkungsteil der K₁

- K₁VT_{N}: oberer Verstärkungsteil der K₁
- K₁W₁: VorwärmerNerdampfer/LJberhitzer der K₁ für Dialkylcarbonat enthaltenden Strom
- K₁W₂: Vorwärmer/Verdampfer der K₁ für Eduktstrom mit aromatischer Hydroxyverbindung
- K₁RZ: Reaktionszone der K₁
- K₁AT: Abtriebsteil der K₁
- K₁E_RZ_{1-N}: Zwischenverdampfer 1 bis N im Bereich der Reaktionszone der \K₁

- K₂: Diarylcarbonat-Reaktionskolonne (zweite Reaktionskolonne)
- K₂C_{1-N}: Kopfkondensator(en) 1 bis N der K₂
- K₂C_{N+1}: Kondensator für Restbrüdenstrom aus K₂C_{1-N} enthaltend mittelsiedende Nebenverbindungen
- K₂E_{1-N}: Verdampfer 1 bis N der K₂
- K₂VT: Verstärkungsteil der K₂
- K₂AT: Abtriebsteil und Reaktionszone der K₂
- K₂E_AT_{1-N}: Zwischenverdampfer 1 -N im Abtriebsteil der K₂

- K₃: Diarylcarbonat-Feindestillationskolonne (erste Diarylcarbonat-Destillationskolonne)
- K₃C_{1-N}: gegebenenfalls mehrstufige(r) Kopfkondensator(en) 1 bis N der K₃
- K₃E_{1-N}: gegebenenfalls mehrstufige(r) Verdampfer 1 bis N der K₃
- K₃E_{N+1}: Zusätzlicher Verdampfer zur Aufkonzentrierung des nicht in das Verfahren zurückgeführten Sumpfproduktstromes aus dem Sumpf der Kolonne K₃ und/oder der/des Kolonnenverdampfer/s K₃E_{1-N}
- K₃SC_{1-N}: gegebenenfalls mehrstufige(r) Kondensator(en) 1 bis N für dampfförmigen Seitenstrom der K₃
- K₃VT₁: oberer Verstärkungsteil der K₃
- K₃VT₂: unterer Verstärkungsteil der K₃
- K₃AT₁: oberer Abtriebsteil der K₃
- K₃AT₂: unterer Abtriebsteil der K₃
- K₃T: Trennwand der K₃
- K₃TLO: Verstärkungsteil auf der Zulaufseite der Trennwand der K₃
- K₃TLU: Abtriebsteil auf der Zulaufseite der Trennwand der K₃
- K₃TRU: Verstärkungsteil auf der Entnahmeseite der Trennwand der K₃
- K₃TRO: Abtriebsteil auf der Entnahmeseite der Trennwand der K₃

- K₄: Diarylcarbonat-Seitenstromkolonne (zweite Diarylcarbonat-Destillationskolonne)
- K₄C_{1-N}: gegebenenfalls mehrstufige(r) Kopfkondensator(en) 1 bis N der K₄

- K₄VT: Verstärkungsteil der K₄
- K₄AT: Abtriebsteil der K₄
- K₄E_{1-N}: gegebenenfalls mehrstufige(r) Verdampfer 1 bis N der K₄

- K₅: Dialkylcarbonat-Destillationskolonne
- K₅VT: Verstärkungsteil der K₅
- K₅AT: Abtriebsteil der K₅
- K₅W₁: Vorwärmer/Verdampfer der K₅ für Reaktionsalkohol und Dialkylcarbonat enthaltenden Strom
- K₅C_{1-N}: Kopflcondensator(en) 1 bis N der K₅
- K₅E_{1-N}: Verdampfer 1 bis N der K₅
- K₅E_AT_{1-N}: Zwischenverdampfer 1 bis N im Abtriebsteil der K₅

- K₆: Reaktionsalkohol-Destillationskolonne
- K₆C_{1-N}: Kopfkondensator(en) 1 bis N der K₆
- K₆E_{1-N}: Verdampfer 1 bis N der K₆
- K₆VT: Verstärkungsteil der K₆
- K₆AT: Abtriebsteil der K₆

- M: Membrantrennung (Dampfpermeation oder Pervaporation)
- MRC: Kondensator für Retentat nach der Membrantrennung
- MPC: Kondensator für Permeat nach der Membrantrennung
- K₇: Alkylarylether-Vorkonzentrierungskolonne (erste Zwischensiederkolonne)
- K₇C _{1-N}: gegebenenfalls mehrstufige(r) Kopfkondensator(en) 1 bis N der K₇
- K₇E_{1-N}: gegebenenfalls mehrstufige(r) Verdampfer 1 bis N der K₇
- K₇VT: Verstärkungsteil der K₇
- K₇AT: Abtriebsteil der K₇

- K₈: Alkylarylether-Kolonne (zweite Zwischensiederkolonne)
- K₈C _{1-N}: gegebenenfalls mehrstufige(r) Kopfkondensator(en) 1 bis N der K₈
- K₈SC _{1-N}: gegebenenfalls mehrstufige(r) Kondensator(en) 1 bis N für dampfförmigen Seitenstrom der K₈
- K₈E_{1-N}: gegebenenfalls mehrstufige(r) Verdampfer 1 bis N der K₈
- K_{B}VT: Verstärkungsteil der K₈
- K₈AT₁: oberer Abtriebsteil der K₈
- K₈AT₂: unterer Abtriebsteil der K₈
- K₈T: Trennwand der K₈

- K₈TLO: Verstärkungsteil auf der Zulaufseite der Trennwand der K₈
- K₈TLU: Abtriebsteil auf der Zulaufseite der Trennwand der K₈
- K₈TRU: Verstärkungsteil auf der Entnahmeseite der Trennwand der K₈
- K₈TRO: Abtriebsteil auf der Entnahmeseite der Trennwand der K₈

- K_{N}: eine der Reaktions- oder Destillationskolonnen K₁ bis K₈
- K_{N}C _{1-N}: gegebenenfalls mehrstufige(r) Kopfkondensator(en) 1 bis N der K_{N}
- K_{N}VT: Verstärkungsteil der K_{N}
- K_{N}CS _{1-N}: Kolonnensegment(e) 1 bis N der K_{N} mit direkter Kondensation
- K_{N}W_{1-N}: Wärmetauscher 1 bis N zur Kühlung eines Umlaufstromes für die Kondensation in K_{N}CS_{1-N} der K_{N}

Weiterhin sind in den Fig. 1 bis 14 die folgenden Stoffströme benannt
- 1: Eduktzustrom enthaltend Dialkylcarbonat
- 2: Eduktzustrom enthaltend aromatische Hydroxyverbindung
- 3: Destillat der K₂
- 4: Destillat der K₁
- 5: Strom enthaltend Dialkylcarbonat und Reaktionsalkohol
- 6: Sumpfprodukt der K₁
- 7: Zwischensiederpurge
- 8: Sumpfprodukt der K₂
- 9: Strom enthaltend Alkylarylcarbonat und aromatische Hydroxyverbindung
- 10: Ausschleusung Reaktionsalkohol aus K₆
- 11: Dialkylcarbonat enthaltender Strom aus K₅
- 12: Destillat der K₆
- 13: Destillat der K₅
- 14: Strom enthaltend Dialkylcarbonat und Reaktionsalkohol
- 15: Dialkylcarbonat enthaltender Strom zur K₁
- 16: Aromatische Hydroxyverbindung enthaltender Strom zur K₁
- 17: Dialkylcarbonat enthaltender Strom nach Verdampfung
- 18: Strom mit aromatischer Hydroxyverbindung nach Erhitzung
- 19: Dampfstrom am Kopf der K₁
- 20: Flüssiger Ablauf aus dem Abtriebsteil der K₁
- 21: Dampf-Flüssigkeitsgemisch aus Sumpfverdampfer der K₁
- 22: Dampfgemisch aus unterem Verstärkungsteil der K₁
- 23: Kondensat des oder der Zwischenkondensatoren der K₁
- 24: Ablauf Flüssigkeitsgemisch aus oberem Verstärkungsteil der K₁

- 25: Rücklauf der K₁
- 26: Restliches Dampfgemisch aus Kondensation der K₁
- 27: Dampfstrom am Kopf der K₁
- 28: Ablauf Flüssigkeitsgemisch aus Reaktionszone oder gegebenenfalls Abtriebsteil der K₂
- 29: Dampf-Flüssigkeitsgemisch aus Sumpfverdampfer der K₂
- 30: Rücklauf der K₂
- 31: Dampfstrom am Kopf der K₅
- 32: Rücklauf der K₅
- 33: Zulaufgemisch zur K₅
- 34: Destillat K₅ zur Membrantrennung (M)
- 35: Retentat Membrantrennung (M) zum Kondensator (MRC)
- 36: Flüssiges Retentat zur K₅
- 37: Permeat der Membrantrennung (M) zum Kondensator (MPC)
- 38: Strom enthaltend mittelsiedende Nebenverbindungen
- 39: Strom enthaltend aromatische Hydroxyverbindung aus K₈
- 40: Destillatstrom enthaltend Zwischensieder
- 41: Sumpfprodukt enthaltend Zwischensieder zur Entsorgung
- 42: Sumpfprodukt der K₈ zur K₁ oder K₂
- 43: Sumpfprodukt enthaltend aromatische Hydroxyverbindung und mittelsiedende Nebenverbindungen
- 44: Dampfstrom am Kopf der K₇
- 45: Rücklauf der K₇
- 46: Restlicher Dampfstrom nach Kondensation in K₇C_{1-N}
- 47: Dampfstrom am Kopf der Kg
- 48: Rücklauf der K₈
- 49: Restlicher Dampfstrom nach Kondensation in K₈C_{1-N}
- 50: Strom enthaltend aromatische Hydroxyverbindung und Katalysator
- 51: Strom enthaltend Diarylcarbonat mit hoher Reinheit
- 52: Strom enthaltend schwersiedende Nebenverbindungen und Katalysator
- 53: Strom enthaltend mittelsiedende Nebenverbindungen
- 54: Strom enthaltend schwersiedende Nebenverbindungen und Katalysator zur Entsorgung
- 55: Strom enthaltend schwersiedende Nebnverbindungen und Katalysator
- 56: Brüdenstrom enthaltend Diarylcarbonat zur K₃
- 57: Brüdenstrom enthaltend Diarylcarbonat zur K₄
- 58: Brüdenstrom der K₃
- 59: Restlicher Dampfstrom nach Kondensation in K₃C_{1-N}
- 60: Rücklauf der K₃

- 61: Sumpfprodukt der K₄
- 62: Brüdenstrom der K₄
- 63: Rücklauf der K₄
- 64: Ablauf Flüssigkeit aus dem unteren Abtriebsteil der K₃
- 65: Sumpfprodukt der K₈
- 66: Restbrüdenstrom nach Kondensation in K₄C_{1-N}
- 67: Purgestrom aus dem Sumpfprodukt derK₄
- 68: Seitenstrom enthaltend aromatische Hydroxyverbindung aus K₈
- 69: Brüdenstrom aus Abtriebsteil der K₃ zur Zulaufseite (Ausführung als Trennwandkolonne)
- 70: Brüdenstrom aus Verstärkungsteil der K_{N} zur Kondensation
- 71: Rücklauf auf Verstärkungsteil der K_{N}
- 72: Destillat
- 73: Restbrüdenstrom nach Kondensation
- 74: Externer Kreislauf bei Kondensation in einem Kolonnensegment vor Kühlung
- 75: Externer Kreislauf bei Kondensation in einem Kolonnensegment nach Kühlung
- 76: Brüdenstrom aus Abtriebsteil der K₃ zur Entnahmeseite (Ausführung als Trennwandkolonne)
- 77: Flüssigkeit aus Verstärkungsteil der K₃ zur Zulaufseite (Ausführung als Trennwandkolonne)
- 78: Flüssigkeit aus Abtriebsteil der K₃ zur Entnahmeseite (Ausführung als Trennwandkolonne)

Fig. 1 zeigt unter anderem eine erste Reaktionskolonne K₁, in die die beiden Eduktströme, d.h. ein die aromatische Hydroxyverbindung enthaltender Strom 16 und ein das Dialkylcarbonat enthaltender Strom 15 im Bereich einer Reaktionszone RZ im Sinne eine Gegenstromumesterung gegeneinander geführt und zu Alkylarylcarbonaten und geringen Anteilen Diarylcarbonaten umgesetzt werden.

Der das Dialkylcarbonat enthaltende Strom 15 kann - insbesondere bei kontinuierlich geführten Verfahren - neben dem Dialkylcarbonat auch Teile der aromatischen Hydroxyverbindung, die bei der Reaktion anfallende aliphatische Hydroxyverbindung R¹-OH und/oder R²-OH (Reaktionsalkohol), sehr geringe Mengen des bei der Umesterung anfallenden Alkylarylcarbonats und/oder Diarylcarbonats und bei der Reaktion entstehende unerwünschte Nebenkomponenten enthalten. Der das Dialkylcarbonat enthaltende Strom 15 kann beispielsweise 0 bis 5 Gew.-%, bevorzugt 0.05 bis 3 Gew: % und besonders bevorzugt 0.05 bis 2 Gew: % des Reaktionsalkohols, 0 bis 40 Gew.-%, bevorzugt 0 bis 10 Gew.-%, besonders bevorzugt 0 bis 5 Gew.-% der aromatischen Hydroxyverbindung, 0 bis 5 Gew.-% Alkylarylcarbonat, 0 bis 5 Gew.-% Diarylcarbonat und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dialkylcarbonat enthaltenden Stromes, aufweisen. Vorzugsweise enthält der das Dialkylcarbonat enthaltende Strom 15 50 bis 100 Gew.-% Dialkylcarbonat, bezogen auf das Gesamtgewicht des Dialkylcarbonat enthaltenden Stromes, wobei sich die Anteile der einzelnen vorangehend genannten Komponenten zu 100 Gew.-% addieren. Der die aromatische Hydroxyverbindung enthaltende Strom 16 kann - insbesondere bei kontinuierlich geführten Verfahren - neben der aromatischen Hydroxyverbindung auch Teile des Dialkylcarbonats, das bei der Umesterung anfallende Alkylarylcarbonat und/oder Diarylcarbonat, in sehr geringen Mengen den Reaktionsalkohol und bei der Reaktion entstehende unerwünschte Nebenprodukte enthalten. Beispielsweise kann der Gehalt des Dialkylcarbonats 0 bis 50 Gew.-%, der Gehalt des Reaktionsalkohols 0 bis 10 Gew.-%, bevorzugt 0 bis 5 Gew.-%, der Gehalt des Alkylarylcarbonats und des Diarylcarbonats jeweils 0 bis 10 Gew.-%, bevorzugt 0 bis 5 Gew.-% und der Gehalt der unerwünschten Nebenprodukte 0 bis 5 Gew.-%, bevorzugt 0 bis 1 Gew.-%, jeweils bezogen auf das Gesamtgewicht des die aromatische Hydroxyverbindung enthaltenden Stromes, betragen. Mit dem die aromatische Hydroxyverbindung enthaltenden Strom 16 kann zudem der Katalysator in die Reaktionskolonne eingespeist werden. In diesem Fall beträgt der Gehalt an Katalysator bevorzugt 0 bis 5 Gew.-%, bezogen auf das Gesamtgewicht des die aromatische Hydroxyverbindung enthaltenden Stromes. Vorzugsweise enthält der die aromatische Hydroxyverbindung enthaltende Strom 16 50 bis 100 Gew.-% aromatische Hydroxyverbindung, bezogen auf das Gesamtgewicht des die aromatische Hydroxyverbindung enthaltenden Stromes, wobei sich die Anteile der einzelnen vorangehend genannten Komponenten zu 100 Gew.-% addieren.

Der das Dialkylcarbonat enthaltende Strom 15 wird vor Einleiten in die Kolonne K₁ teilweise oder vollständig verdampft und gegebenenfalls überhitzt. Der die aromatische Hydroxyverbindung enthaltende Strom 16 wird vor Einleiten in die Kolonne K₁ erhitzt und dabei gegebenenfalls teilweise verdampft. Die Eduktströme 17 und 18, jeweils nach Verdampfung und gegebenenfalls Überhitzung bzw. nach Erhitzen, werden in der Reaktionszone K₁RZ im Gegenstrom zueinander geführt, d.h. der die aromatische Hydroxyverbindung enthaltende Strom 18 wird am oberen Ende der Reaktionszone K₁RZ in erhitzter, vorwiegend flüssiger Form und der das Dialkylcarbonat enthaltende Strom 17 wird am unteren Ende der Reaktionszone K₁RZ vorwiegend gasförmig oder gegebenenfalls leicht überhitzt zugeführt. Die bei der Reaktion anfallende aliphatische Hydroxyverbindung R¹-OH und/oder R²-OH wird zusammen mit noch nicht umgesetzten Dialkylcarbonat am Kopf der Kolonne dampfförmig abgezogen (19) und das schwerer flüchtige Alkylarylcarbonat zusammen mit noch nicht umgesetzten Mengen der aromatischen Hydroxyverbindung, Diarylcarbonat und gegebenenfalls weiteren schwerflüchtigen Verbindungen als flüssiges Sumpfprodukt der K₁ entnommen (6). Die zur Einstellung des gewünschten Temperaturprofils erforderliche Energie kann unter Anderem am Sumpf der Kolonne durch einen oder mehrere Verdampfer K₁E_{1-N} erzeugt werden. Hierzu wird das aus dem Abtriebsteil K₁AT, bzw. bei nicht vorhandenem Abtriebsteil aus der Reaktionszone K₁RZ, ablaufende flüssige Gemisch (20) teilweise verdampft. Je nach Verdampferausführung erhält man am Austritt des Verdampfers nur Dampf oder ein Dampf-Flüssigkeitsgemisch (Strom 21). Der im Strom 21 enthaltene Dampf wird dem Abtriebsteil (K₁AT) von unten, oder falls kein Abtriebsteil vorhanden ist, der Reaktionszone K₁RZ von unten zugeführt. Im Bereich der Reaktionszone kann durch zusätzliche Zwischenverdampfer K₁E_RZ₁-_{N} Wärme zugeführt werden. Im zwischen Reaktionszone K₁RZ und Verdampfer K₁E_{1-N} angebrachten Abtriebsteil K₁AT erfolgt eine Aufkonzentrierung des gebildeten Alkylarylcarbonats und des Diarylcarbonats, wobei bereits in diesem Teil der Kolonne K₁ durch die Verarmung an Dialkylcarbonat die Disproportionierungsreaktion von Alkylarylcarbonat zu Diarylcarbonat in verstärktem Maße einsetzt.

In einem oder mehreren zwischen dem/den Kondensator/en K₁C_{1-N} und Reaktionszone K₁RZ befindlichen Verstärkungsteil(en) erfolgt die Aufkonzentrierung der bei der Reaktion gebildeten aliphatischen Hydroxyverbindung (Reaktionsalkohol) und des überschüssigen Dialkylcarbonats. Dabei soll ein Gehalt an aromatischer Hydroxyverbindung(en) im Destillat 4 von 0 bis 40 Gew.-% bevorzugt 0 bis 10 Gew.-%, besonders bevorzugt von 0 bis 5 Gew.-%, bezogen auf Gesamtgewicht des Destillats 4, eingestellt werden. Der Verstärkungsteil ist in mindestens zwei Sektionen, den oberen und den unteren Verstärkungsteil, unterteilt, wobei sich zwischen dem oberen Verstärkungsteil K₁VT_{N} und dem unteren Verstärkungsteil K₁VT₁ ein oder mehrere Zwischenkondensator(en) K₁IC_{1-N}, bevorzugt wenigstens ein Zwischenkondensator K₁IC₁, befindet. Diese(r) Zwischenkondensator(en) K₁IC_{1-N} bzw. dieser Zwischenkondensator K₁IC₁ kondensiert einen Teil der aus dem unteren Verstärkungsteil K₁VT₁ aufsteigenden Dämpfe 22. Das in den oder die Zwischenkondensator(en) K₁IC_{1-N}, bevorzugt wenigstens einen Zwischenkondensator K₁IC₁, eintretende dampfförmige Gemisch 22 enthält vorzugsweise 10 bis 80 Gew.-% an aromatischer Hydroxyverbindung. Die Kondensationstemperatur in dem oder den Zwischenkondensator(en) K₁IC_{1-N} ist daher aufgrund verhältnismäßig hoher Mengen an aromatischer Hydroxyverbindung deutlich höher im Vergleich zur Kondensationstemperatur im Kopfkondensator K₁C_{1-N} (N: Kondensator ist gegebenenfalls mehrstufig). Je nach Betriebsdruck und Lage des Konzentrationsprofils kann die Kondensationstemperatur in dem oder den Zwischenkondensator(en) bevorzugt im Bereich von 100 bis 300°C, besonders bevorzugt von 120 bis 250°C, ganz besonders bevorzugt von 150 bis 240°C und im Kopfkondensator bevorzugt im Bereich von 0 bis 250°C, besonders bevorzugt von 40 bis 200°C liegen. Das in dem oder den Zwischenkondensator(en) K₁IC_{1-N} anfallende Kondensat 23 sowie die aus dem darüber liegenden oberen Verstärkungsteil K₁VT_{N} ablaufende Flüssigkeit 24 wird auf den unteren Verstärkungsteil K₁VT₁ geleitet. Das dampfförmige Gemisch nach dem oder den Zwischenkondensator(en) gelangt in den oberen Verstärkungsteil K₁VT_{N}. Der aus dem oberen Verstärkungsteil K₁VT_{N} kommende Dampf 19 wird in dem oder den Kondensator(en) K₁C_{1-N} weitestgehend kondensiert, wobei das Kondensat teilweise wieder als Rücklauf dem oberen Verstärkungsteil K₁VT_{N} zugeführt wird (25) und teilweise als Destillatstrom 4 entnommen wird. Der Destillatstrom 4 enthält im Wesentlichen das im Überschuss eingesetzte Dialkylcarbonat und den entsprechenden bei der Reaktion entstehenden Alkylalkohol R¹-OH und/oder R²-OH (Reaktionsalkohol) und gegebenenfalls geringe Mengen der aromatischen Hydroxyverbindung. Das restliche Dampfgemisch aus dem oder den Kondensator(en) K₁C_{1-N} wird als Dampfstrom 26 entnommen.

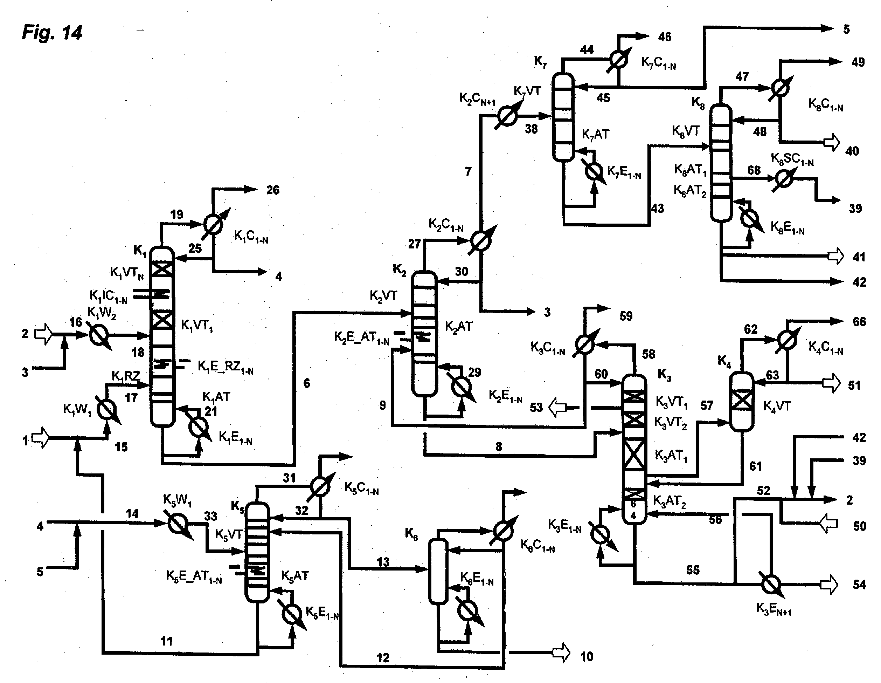

Die in dem oder den Zwischenkondensator(en) K₁IC_{1-N}, bevorzugt wenigstens dem Zwischenkondensator K₁IC₁, frei werdende Kondensationswärme kann wie vorangehend für das erfindungsgemäße Verfahren beschrieben direkt oder indirekt wieder in das Verfahren zurückgeführt werden (In Fig. 1 und 14 nicht gezeigt).

In einer bevorzugten Ausführungsform des erfindungsgemäßen Verfahrens wird die in dem oder den Zwischenkondensator(en) K₁IC_{1-N}, bevorzugt wenigstens dem Zwischenkondensator K₁IC₁, anfallende Kondensationswärme zur Erhitzung eines Wärmeträgermediums verwendet. Dieses wiederum wird zur Verdampfung und Überhitzung des bei der Gegenstromumesterung in der Reaktionskolonne K₁ eingesetzten Dialkylcarbonat enthaltenden Stromes 15 verwendet. Bei dieser bevorzugten Ausführungsform handelt es sich um eine indirekte Nutzung der Kondensationswärme.

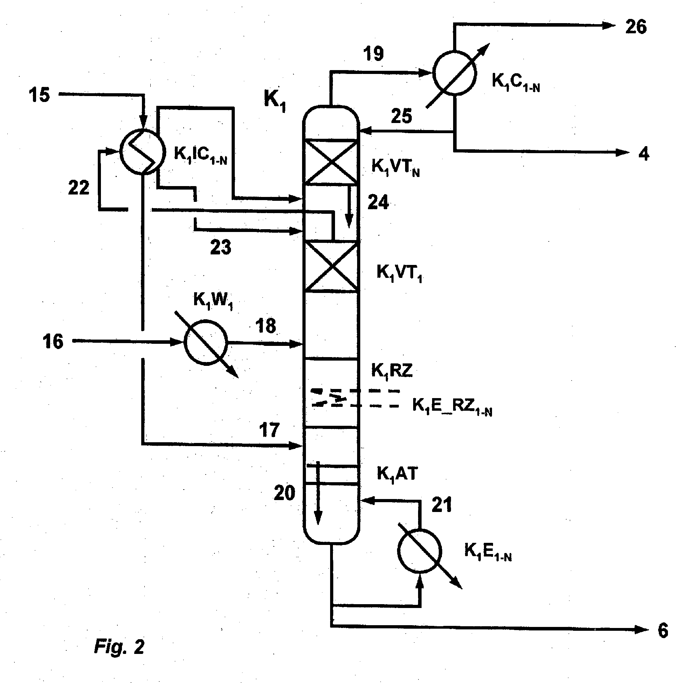

Eine andere bevorzugte Ausführungsform der Umesterung in der ersten Reaktionskolonne in Gegenwart wenigstens eines Zwischenkondensators wird in Fig. 2 gezeigt. Hierbei werden der oder die Zwischenkondensator(en) außerhalb der ersten Reaktionskolonne ausgeführt. Die Erhitzung, Verdampfung und gegebenenfalls Überhitzung des Dialkylcarbonat enthaltenden Stromes 15 erfolgt ebenfalls im Zwischenkondensator. Dabei wird das dampfförmige Gemisch 22 des unteren Verstärkungsteils K₁VT₁ zu dem oder den Zwischenkondensator(en) K₁IC_{1-N}, bevorzugt zu wenigstens einem Zwischenkondensator K₁IC₁, geleitet, wo dieses teilweise kondensiert. Das dabei anfallende Kondensat 23 wird wieder dem unteren Verstärkungsteil K₁VT₁ zugeführt und die nicht kondensierten Dämpfe in den oberen Verstärkungsteil K₁VT_{N} geleitet. Ansonsten entspricht das in Fig. 2 gezeigte Verfahren dem in Fig. 1 dargestellten. Die vorangehenden Erläuterungen für Fig. 1 gelten daher analog.

Gemäß Fig. 1 wird das Sumpfprodukt 6 der ersten Reaktionskolonne K₁ einer zweiten Reaktionskolonne K₂ zugeführt. Dieses kann 0 bis 60 Gew.-% Diarylcarbonat, 5 bis 80 Gew.-% Alkylarylcarbonat, 5 bis 95 Gew.-% der aromatischen Hydroxyverbindung, 1 bis 80 Gew.-% Dialkylcarbonat, 0 bis 5 Gew.-% Katalysator und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Sumpfproduktstromes 6, aufweisen. Die Prozentangaben beziehen sich auf das Gesamtgewicht des Sumpfproduktstromes 6, wobei sich die Anteile der einzelnen vorangehend genannten Komponenten zu 100 Gew.-% addieren.

Zusätzlich zum Sumpfprodukt der ersten Reaktionskolonne können darüber hinaus mindestens noch ein weiterer Alkylarylcarbonat enthaltender Strom 9 der zweiten Reaktionskolonne zugeführt werden. Dieser Strom 9 kann beispielsweise aus einem Aufarbeitungsschritt zur Aufreinigung des Diarylcarbonats, wie z.B. einer Diarylcarbonat-Destillationskolonne K₃, stammen.

Dieser kann 0 bis 10 Gew.-% Diarylcarbonat, 10 bis 100 Gew.-% Alkylarylcarbonat, 0 bis 90 Gew.-% der aromatischen Hydroxyverbindung, 0 bis 20 Gew.-% Dialkylcarbonat und 0 bis 20 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dialkylcarbonat enthaltenden Stromes, aufweisen. Die Prozentangaben beziehen sich auf das Gesamtgewicht des Stromes 9, wobei sich die Anteile der einzelnen vorangehend genannten Komponenten zu 100 Gew.-% addieren.

Die Ströme 6 und 9 werden der Reaktionszone K₂AT der zweiten Reaktionskolonne zugeführt.

Die bei der Umesterung anfallende Reaktionsalkohol R¹-OH und/oder R²-OH wird zusammen mit noch nicht umgesetzten oder bei der Disproportionierung freigesetztem Dialkylcarbonat und nicht umgesetzter aromatischer Hydroxyverbindung am Kopf der Kolonne K₂ dampfförmig abgezogen (27) und das schwerer flüchtige Diarylcarbonat zusammen mit noch nicht umgesetzten Mengen der aromatischen Hydroxyverbindung, Alkylarylcarbonat und gegebenenfalls weiteren schwerflüchtigen Verbindungen als flüssiger Strom am Fuß der zweiten Reaktionskolonne K₂ entnommen (8).

Die zur Einstellung des gewünschten Temperaturprofils erforderliche Energie kann unter anderem am Sumpf der Kolonne durch einen oder mehrere Verdampfer K₂E_{1-N} erfolgen. Hierzu wird das aus der Reaktionszone, ablaufende flüssige Gemisch (28) teilweise verdampft. Je nach Verdampferausführung erhält man am Austritt des Verdampfers nur Dampf oder ein Dampf-Flüssigkeitsgemisch (Strom 29). Der im Strom 29 enthaltene Dampf wird dem Abtriebsteil (K₂AT), welcher gleichzeitig auch als Reaktionszone fungiert und aus mehreren Sektionen besteht von unten zugeführt. Im Bereich der Reaktionszone kann durch zusätzliche Zwischenverdampfer K₂E_AT_{I}-_{N} Wärme zugeführt werden. In der Reaktionszone K₂AT und im Verdampfer K₂E_{1-N} erfolgt sowohl Reaktion (Umesterung und/oder bevorzugt Disproportionierung) als auch Abtrennung der gebildeten leichtsiedenden Reaktionsprodukte (Reaktionsalkohol und Dialkylcarbonat) und der aromatischen Hydroxyverbindung.

In einem zwischen dem/den Kondensator/en K₂C_{1-N} und Reaktionszone K₂AT befindlichen Verstärkungsteil K₂VT wird der Gehalt an schwersiedenden Verbindungen wie beispielsweise Alkylarylcarbonat oder Diarylcarbonat reduziert. Dabei wird vorzugsweise ein Gehalt an Alkylarylcarbonat im Destillat 3 von 0 bis 20 Gew.-%, bevorzugt 0 bis 5 Gew.-%, besonders bevorzugt von 0 bis 2 Gew.-%, bezogen auf Gesamtgewicht des Destillats 3, eingestellt. Der Verstärkungsteil kann analog zur ersten Reaktionskolonne mit einem oder mehreren Zwischenkondensatoren ausgeführt werden. In der in Fig. 1 und Fig. 14 dargestellten bevorzugten Ausführungsform wird der Verstärkungsteil der K₂ jedoch ohne Zwischenkondensator(en) ausgeführt.

Der oder die Kondensator(en) K₂C_{1-N}, in einer ganz besonders bevorzugten Ausführungsform eine Kaskade von Kondensatoren, am Kopf der K₂ kondensieren einen Teil der aus dem Verstärkungsteil K₂VT aufsteigenden Dämpfe 27. Das in den oder die Kondensator(en) K₂C_{1-N} eintretende dampfförmige Gemisch 27 enthält vorzugsweise 10 bis 90 Gew.-% an aromatischer Hydroxyverbindung. Die Kondensationstemperatur in dem oder den Kondensator(en) K₂C_{1-N} ist daher aufgrund verhältnismäßig hoher Mengen an aromatischer Hydroxyverbindung hoch. Je nach Betriebsdruck und Zusammensetzung des dampfförmigen Gemisches 27 kann die Kondensationstemperatur in dem oder den Kondensator(en) bevorzugt im Bereich von 100 bis 300°C, besonders bevorzugt von 120 bis 250°C, ganz besonders bevorzugt von 150 bis 240°C liegen. Das Kondensat wird teilweise wieder als Rücklauf 30 dem Verstärkungsteil K₂VT zugeführt und teilweise als Destillatstrom 3 entnommen.

Der Destillatstrom 3 enthält im Wesentlichen aromatische Hydroxyverbindungen und geringe Mengen an Reaktionsalkohol, bevorzugt 0 bis 5 Gew.-% und kann dem Eduktstrom der ersten Reaktionskolonne wieder zugeführt werden.

Das Destillat der ersten Reaktionskolonne (4) wird gegebenenfalls zusammen mit weiteren, Reaktionsalkohol und Dialkylcarbonat enhaltenden Strömen (5 und/oder 12) gegebenenfalls nach Erhitzung und/oder teilweiser Verdampfung einer Destillationskolonne K₅ (Dialkylcarbonat-Destillationskolonne) zur Trennung des Dialkylcarbonats vom gebildeten Reaktionsalkohols zugeführt, wobei der erhaltene Dialkylcarbonat enthaltende Strom 11 wieder dem Dialkylcarbonat enthaltenden Zustrom 15 der ersten Reaktionskolonne zugeführt und der abgetrennte Reaktionsalkohol aus dem Verfahren ausgeschleust wird (10). Strom 5 kann dabei beispielsweise aus weiteren Reinigungs- bzw. Nebenproduktabtrennungsschritten, wie z.B. der Abtrennung mittelsiedender Nebenverbindungen in K₇, stammen.

Bilden Reaktionsalkohol und Dialkylcarbonat ein Azeotrop, so erhält man als Destillat (13) der Destillationskolonne K₅ vorzugsweise ein annähernd azeotropes Gemisch. Für eine vollständige Trennung von Reaktionsalkohol und Dialkylcarbonat ist daher mindestens ein weiterer Trennschritt erforderlich.

Bilden Reaktionsalkohol und Dialkylcarbonat kein Azeotrop, so erhält man als Destillat vorzugsweise Reaktionsalkohol mit einem Gehalt von 95 bis 100 Gew.-%.

Als Sumpfprodukt der Destillationskolonne K₅ wird ein Gemisch enthaltend Dialkylcarbonat mit weniger als 5 Gew.-% Reaktionsalkohol entnommen.

Die Dialkylcarbonat-Destillationskolonne K₅ verfügt über einen Verstärkungsteil mit bevorzugt 5 bis 40 theoretischen Stufen zur Aufkonzentrierung des Reaktionsalkohols und einen Abtriebsteil mit bevorzugt 5 bis 40 theoretischen Stufen zur Aufkonzentrierung des Dialkylcarbonates.

Die für die Destillation in der Dialkylcarbonat-Destillationskolonne erforderliche Energie kann unter anderem am Sumpf der Kolonne durch einen oder mehrere Verdampfer K₅E_{1-N} erzeugt werden. Im Bereich des Abtriebsteils K₅AT kann durch zusätzliche Zwischenverdampfer K₅E_AT_{1-N} Wärme zugeführt werden.

Der oder die Kondensator(en) K₅C_{1-N} kondensieren die aus dem Verstärkungsteil K₅VT aufsteigenden Dämpfe 31. Das Kondensat wird teilweise wieder als Rücklauf 32 dem Verstärkungsteil K₅VT zugeführt und teilweise als Destillatstrom 13 entnommen.

Der Destillatstrom 13 enthält Reaktionsalkohol und Dialkylcarbonat in nahezu azeotroper Zusammensetzung. Bilden Reaktionsalkohol und Dialkylcarbonat kein Azeotrop, so erhält man als Destillat nahezu reinen Reaktionsalkohol.

Der Betriebsdruck in der Dialkylcarbonat-Destillationskolonne (K₅) wird so eingestellt, dass die Kolonne mit Abwärme aus dem Umesterungsprozess betrieben werden kann. Bevorzugt wird hierzu die Kondensationswärme aus dem Zwischenkondensator der ersten Reaktionskolonne und/oder dem oder den Kondensator(en) der zweiten Reaktionskolonne genutzt. Bevorzugt wird der Betriebsdruck in der Kolonne K₅ so eingestellt, dass die Verdampungstemperatur im Sumpf der Kolonne K₅ unter der Kondensationstemperatur im Zwischenkondensator der ersten Reaktionskolonne und/oder dem oder den Kondensatoren der zweiten Reaktionskolonne liegt.

Bilden Reaktionsalkohol und Dialkylcarbonat unter den Bedingungen in der Destillationskolonne K₅ ein Azeotrop, so kann man dieses mittels Schleppmittel- oder Extraktivrektifikation, nach dem Zweidruckverfahren oder mittels einer Kombination aus Rektifikation und Membrantrennung getrennt werden. Besonders bevorzugt wird das Zweidruckverfahren zur Trennung des Reaktionsalkohol und des Dialkylcarbonat eingesetzt, welches anhand von Fig. 1 ebenfalls beispielhaft erläutert wird.

Hat das Destillat der Destillationskolonne K₅ eine azeotrope Zusammensetzung, so wird dieses einer weiteren Kolonne (Reaktionsalkohol-Destillationskolonne; K₆ in Fig. 1 und 14) zugeführt, die bei einem Betriebsdruck arbeitet, der unter dem der Destillationskolonne K₅ liegt. Durch den unterschiedlichen Betriebsdruck verschiebt sich die Lage des Azeotrops hin zu geringeren Anteilen an Reaktionsalkohol. Man erhält als Sumpfprodukt 10 der Destillationskolonne K₆ Reaktionsalkohol mit einer Reinheit von 90 bis 100 Gew.-% und als Destillat der Kolonne K₆ ein nahezu azeotropes Gemisch. Die bei geringerem Betriebsdruck arbeitende Kolonne K₆ wird in einer besonders bevorzugten Ausführungsform mit der Kondensationswärme des oder der Kopflcondensator(en) der Kolonne K₅ betrieben.

Die Reaktionsalkohol-Destillationskolonne K₆ verfügt über einen Verstärkungsteil K₆VT mit 5 bis 40 theoretischen Stufen zur Aufkonzentrierung des Reaktionsalkohols und einen Abtriebsteil K₆AT mit 5 bis 40 theoretischen Stufen zur Aufkonzentrierung des Dialkylcarbonates.

Weiterhin bevorzugt kann das Azeotrop aus Reaktionsalkohol und Dialkylcarbonat auch mittels eines Hybridverfahrens in Form einer Kombination aus Rektifikation und Membrantrennung getrennt werden (vgl. Fig. 3). Dabei wird das Destillat der K₅ einer Membrantrennung M zugeführt, welche in ihren unterschiedlichen Ausführungsformen bereits vorangehend beschrieben wurde. Dabei wird auf der Permeatseite eine Reaktionsalkoholreiche Fraktion 37 mit einem Reaktionsalkoholgehalt von mindestens 70 Gew.-%, bevorzugt mindestens 90 Gew.-%, bezogen auf das Gesamtgewicht der Fraktion erhalten und im Kondensator MPC kondensiert. Das Retentat 35, welches einen im Vergleich zum Destillat der Kolonne K₅ reduzierten Reaktionsalkoholanteil enthält, wird im Kondensator MRC kondensiert und vorzugsweise wieder der Destillationskolonne K₅ zugeführt (36).

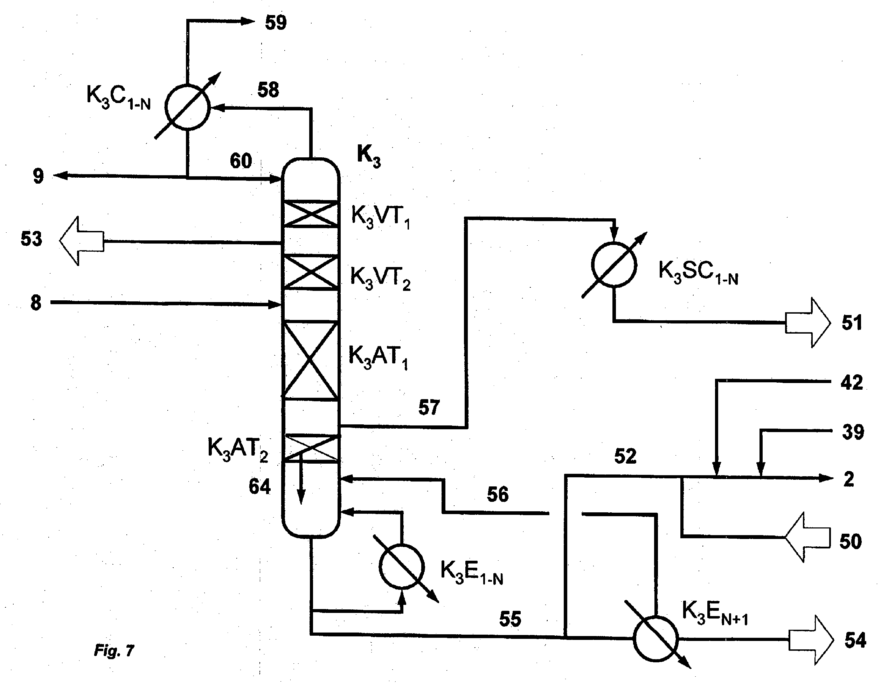

Fig. 7 zeigt beispielhaft eine besonders bevorzugte Variante des erfindungsgemäßen Verfahrens zur Reinigung des in der oder den weiteren Reaktionskolonne(n) aus Schritt (b) erhaltenen Sumpfproduktes enthaltend Diarylcarbonat. Die erste Diarylcarbonat-Destillationskolonne K₃ dieser bevorzugten Ausführungsform weist vier Sektionen, einen unteren Abtriebsteil (K₃AT₂) und einen oberen Abtriebsteil (K₃AT₁) sowie einen unteren Verstärkungsteil (K₃VT₂) und einen oberen Verstärkungsteil (K₃VT₁) auf. Das Sumpfprodukt der zweiten Reaktionskolonne (8) wird zwischen unterem Verstärkungsteil K₃VT₂ und oberem Abtriebsteil K₃AT₁ der Kolonne zugeführt.

Die erste Diarylcarbonat-Destillationskolonne weist zudem einen ein- oder mehrstufigen (N-Stufigen) Kopfkondensator K₃C_{1-N} sowie einen ein- oder mehrstufigen (N-Stufigen) Verdampfer K₃E_{1-N} für das Sumpfprodukt auf. Bei Kondensation oder Verdampfung in mehreren Apparaten (Kondensatoren bzw. Verdampfern) sind sowohl Parallel- und/oder Reihenschaltungen als auch Kombinationen aus Parallel- und Reihenschaltung möglich.

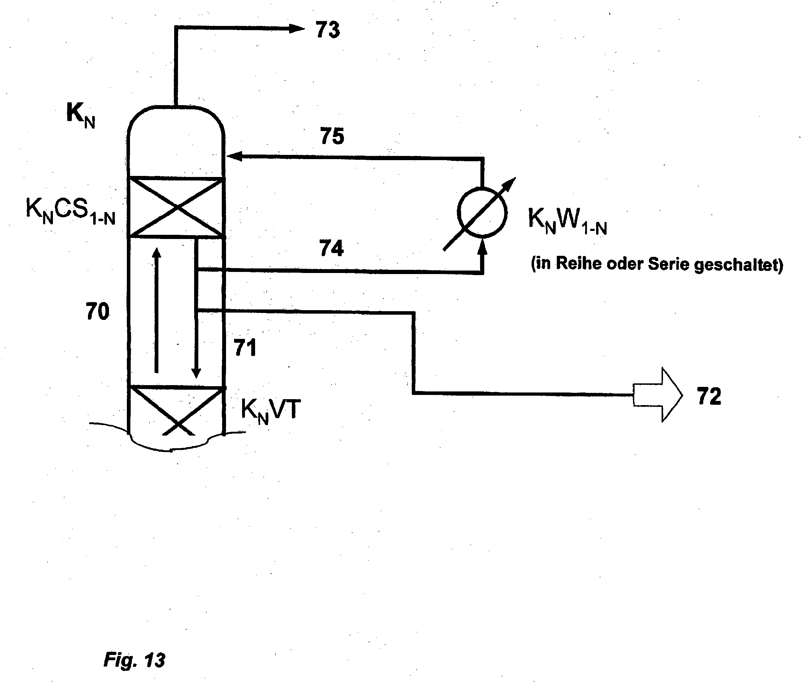

Hinsichtlich der Kondensation im Kopfkondensator sind unterschiedliche Ausführungsformen denkbar. Neben den in Fig. 5a und 5b dargestellten Ausführungsformen des integrierten Kopfkondensators zeigt Fig. 13 eine weiter bevorzugte Ausführungsform eines Kopfkondensators, welche ebenfalls auch in anderen Verfahrensabschnitten des erfindungsgemäßen Verfahrens verwendet werden kann. Dabei erfolgt die Kondensation der aus dem Verstärkungsteil aufsteigenden Dämpfe (70) in einer oder mehreren zusätzlichen Kolonnensektion(en) (K_{N}CS_{1-N}) mit einem in einem externen Kreislauf gekühlten Kondensat. Die am unteren Ende dieser Kolonnensektion austretende Flüssigkeit wird dabei teilweise entnommen (71) und einem oder mehreren externen Kühler(n) K_{N}W_{1-N}, die sowohl in Reihe als auch parallel geschaltet sein können, zur Abfuhr der anfallenden Kondensationswärme zugeführt. Die restliche Flüssigkeit wird entweder als Destillat (72) ausgeschleust oder als Rücklauf (71) auf den Verstärkungsteil K_{N}VT, aufgegeben. Nach der Kühlung wird die Flüssigkeit (75) oberhalb der zusätzlichen Kolonnensektion(en) K_{N}CS_{1-N} wieder der Destillationskolonne zugeführt. Bei der Kondensation in der Kolonne können dabei die bereits vorangehend beschriebenen Kolonnenböden, Füllkörper oder strukturierten Packungen verwendet werden. Die Entnahme der nicht kondensierten Dämpfe oder Inerte (73) erfolgt oberhalb der Kolonnensektion(en) K_{N}CS_{1-N}.

Die aus dem unteren Abtriebsteil K₃AT₂ in der beispielhaften Darstellung in Fig. 7 ablaufende Flüssigkeit 64 wird in einer ein- oder mehrstufigen (N-stufigen) Verdampfung eingedampft, wobei die Dämpfe des dabei anfallenden Dampf-/Flüssigkeitsgemisches wieder dem unteren Abtriebsteil K₃AT₂ zugeführt werden. Die Verdampfung erfolgt vorzugsweise in einem Temperaturbereich von 150 bis 300°C, bevorzugt von 160 bis 240°C und besonders bevorzugt von 180 bis 230°C im Sumpf der Kolonne. Die Temperatur am Kopf der Kolonne beträgt vorzugsweise 40 bis 250°C, bevorzugt 50 bis 200°C und besonders bevorzugt 60 bis 180°C. Man erhält ein Sumpfprodukt (55) mit einem Restgehalt von Diarylcarbonat von unter 95 Gew.-%, bevorzugt unter 90 Gew.-% und besonders bevorzugt unter 75 Gew.-%.

Bevorzugt wird das gereinigte Diarylcarbonat als dampfförmiger Seitenstrom (57) oberhalb des unteren Abtriebsteils K₃AT₂ entnommen und anschließend in einem ein- oder mehrstufigen (N-stufigen) Kondensator K₃SC_{1-N} kondensiert und als Flüssigkeit (51) abgeführt.

Die bei der Kondensation in dem oder den Kondensator(en) K₃SC_{1-N} anfallende Kondensationswärme kann bevorzugt zur Erzeugung von Heizdampf oder zur Beheizung anderer Verfahrensabschnitte, wie z.B. solchen bei der Herstellung von Diarylcarbonaten, verwendet werden.

Die erste Diarylcarbonat-Destillationskolonne K₃ wird bevorzugt bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis wird vorzugsweise dabei so eingestellt, dass der Diarylcarbonat-Gehalt im Destillat 10 bevorzugt kleiner als 10 Gew.-%, besonders bevorzugt kleiner als 5 Gew.-% und ganz besonders bevorzugt kleiner 1 Gew.-%, bezogen auf das Gesamtgewicht des Destillats, beträgt. Hierzu wird bevorzugt ein Rücklaufverhältnis von 0,2 bis 5, besonders bevorzugt 0,2 bis 2 und ganz besonders bevorzugt von 0,3 bis 1,6 eingestellt.

Enthält das Rohdiarylcarbonat Verbindungen mit einem Siedepunkt zwischen dem des Diarylcarbonats und dem des während der Herstellung des Diarylcarbonats als Nebenprodukt gebildeten Alkylarylcarbonats als Verunreinigung, können diese erfindungsgemäß in einem weiteren Seitenstrom (53) der ersten Destillationskolonne entnommen werden.

Wie in Fig. 7 dargestellt kann das Sumpfprodukt der ersten Diarylcarbonat-Destillationskolonne (55) zur Vermeidung von Katalysatorverlusten zu mindestens 50 %, bevorzugt mindestens 80 % und besonders bevorzugt mindestens 90 % wieder in die Umesterung aus wenigstens einem Dialkylcarbonat und wenigstens einer aromatischen Hydroxyverbindung zur Herstellung des Diraylcarbonats zurückgeführt werden (52). Der restliche Teil (55) des Sumpfproduktes wird in einer besonders bevorzugten Ausführungsform einem oder mehreren in Reihe oder parallel geschalteter Verdampfer(n) K₃E_{N+1} zwecks Aufkonzentrierung des Rückstands und teilweiser Rückgewinnung des im Sumpfprodukt der ersten Diarylcarbonat-Destillationskolonne noch enthaltenen Diarylcarbonats zugeführt. Das in der Rückstandsaufkonzentrierung zurück gewonnene Diarylcarbonat (56) kann flüssig oder dampfförmig, bevorzugt dampfförmig wieder der ersten Destillationskolonne zugeführt werden. Der aufkonzentrierte Rückstand (54) kann entweder aus dem Verfahren ausgeschleust oder einer weiteren Aufarbeitungsstufe zwecks Rückgewinnung des Katalysators zugeführt werden.

Im Falle einer Ausschleusung des aufkonzentrierten Rückstands (54) beträgt der Verlust an Diarylcarbonat weniger als 5 %, bevorzugt weniger als 2%, besonders bevorzugt weniger als 1 % und ganz besonders bevorzugt weniger als 0,5 % bezogen auf die Mengen an aufgereinigtem Diarylcarbonat.

Die vorangehend beschriebene Aufarbeitung des Sumpfproduktes der ersten Diarylcarbonat-Destillationskolonne kann optional auch bei allen weiteren im Folgenden vorgestellten Ausführungsformen durchgeführt werden.

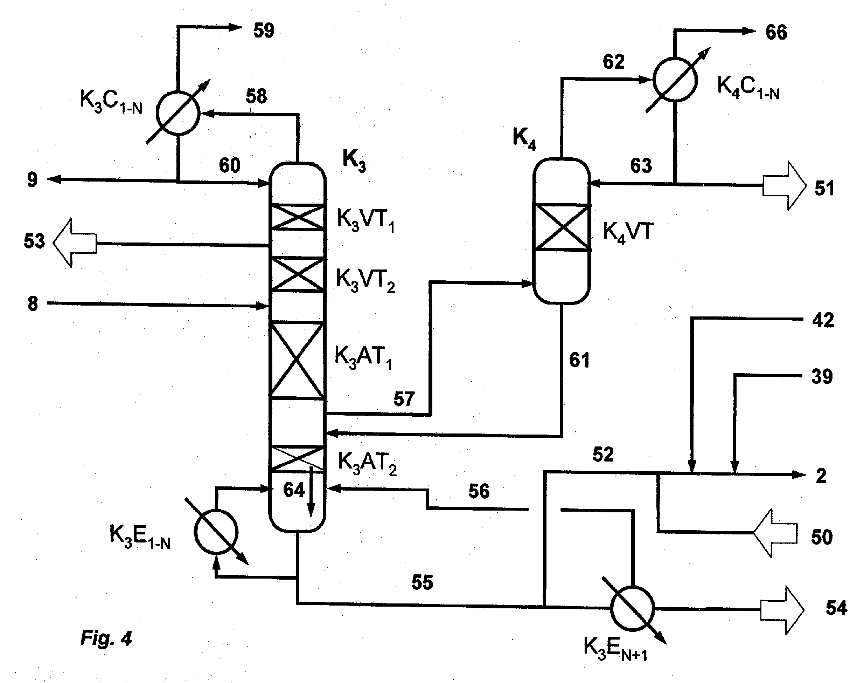

In einer weiteren besonders bevorzugten Ausführungsform des erfindungsgemäßen Verfahrens wird das im Seitenstrom der ersten Diarylcarbonat-Destillationskolonne entnommene Diarylcarbonat (57) in wenigstens einer, bevorzugt in einer zweiten Diarylcarbonat-Destillationskolonne aufgereinigt. In einer besonders bevorzugten Variante dieser bevorzugten Ausführungsform ist diese zweite Diarylcarbonat-Destillationskolonne ohne Abtriebsteil ausgeführt. Eine solche besonders bevorzugte Variante dieser bevorzugten Ausführungsform ist beispielhaft in Fig. 4 dargestellt.

In dieser besonders bevorzugten Variante wird das Diarylcarbonat in einer ersten Diarylcarbonat-Destillationskolonne K₃ - wie sie beispielhaft bereits im Zusammenhang mit Fig. 7 beschrieben wurde - und einer zusätzlichen Seitenstromkolonne K₄ aufgereinigt. Der dampfförmige Seitenstrom 57 wird der zweiten Diarylcarbonat-Destillationskolonne K₄, bevorzugt deren unterem Teil zugeführt. Gegenüber der Ausführung in Fig. 7 weist die Destillationskolonne K₃ einen zusätzlichen Zulauf (61) oberhalb des unteren Abtriebsteils K₃AT₂ auf, über den das flüssige Sumpfprodukt aus der Kolonne K₄ wieder in die K₃ zurückgeführt wird.

Die Kolonne K₄ weist bevorzugt mindestens eine Sektion auf. Besonders bevorzugt wird sie wie in Fig. 4 dargestellt als reiner Verstärkungsteil K₄VT₁ betrieben und besitzt vorzugsweise eine Trennleistung von 1 bis 50, besonders bevorzugt von 2 bis 30 und ganz besonders bevorzugt von 5 bis 20 theoretischen Stufen.

Die zweite Diarylcarbonat-Destillationskolonne K₄ wird bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Die Kolonne K₄ wird vorzugsweise bei einem Rücklaufverhältnis von 0,1 bis 10, besonders bevorzugt von 0,2 bis 5 und ganz besonders bevorzugt von 0,2 bis 2 betrieben.

Die Kondensation der Dämpfe (62) am Kopf der Kolonne K₄ kann ein- oder mehrstufig in einem Kopfkondensator K₄C_{1-N} erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf (63) auf die Kolonne K₄ aufgegeben. Der restliche Teil des Kondensats wird als Destillat (51) (aufgereinigtes Diarylcarbonat) entnommen. Inerte und/oder nicht kondensierte Dämpfe (66) werden ausgeschleust.

Hinsichtlich der Kondensation im Kondensator K₄C_{1-N} sind die gleichen Ausführungsformen, wie bereits für die Kondensation am Kopf der ersten Diarylcarbonat-Destillationskolonne K₃ beschrieben (K₃C_{1-N}) geeignet.

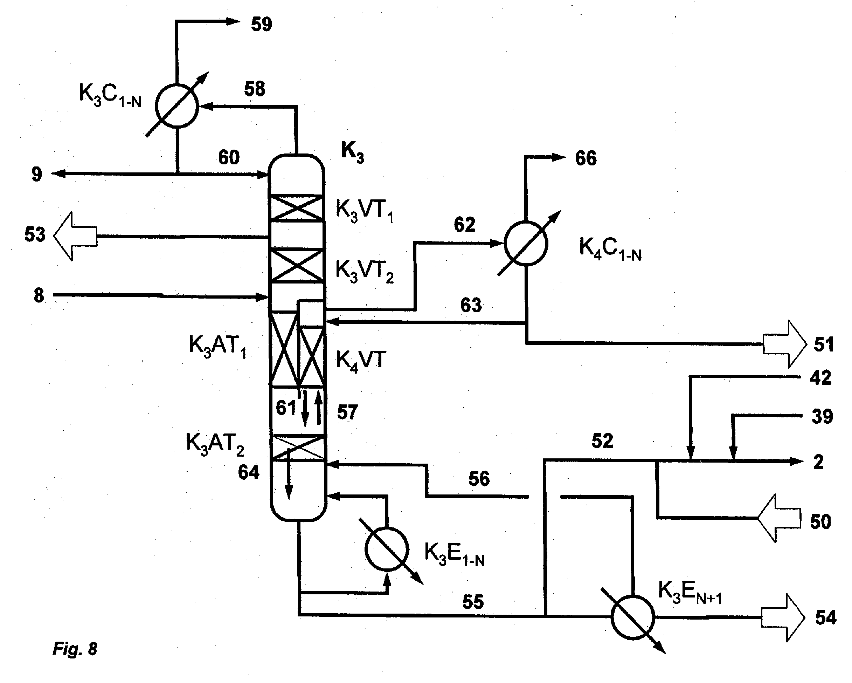

Eine weitere besonders bevorzugten Ausführungsform der Reinigung des in der oder den weiteren Reaktionskolonne(n) aus Schritt (b) erhaltenen Sumpfproduktes enthaltend Diarylcarbonat wird in Fig. 8 dargestellt. Für den Fall der besonders bevorzugten Variante, dass die zweite Diarylcarbonat-Destillationskolonne ohne Abtriebsteil ausgeführt wird, kann der Verstärkungsteil dieser zweite Diarylcarbonat-Destillationskolonne in die erste Diarylcarbonat-Destillationskolonne (K₃) integriert sein. Dabei gelangt ein Teil der aus dem unteren Abtriebsteil der ersten Destillationskolonne (K₃AT₂) kommenden Dämpfe (57) in einen integrierten Verstärkungsteil (K₄VT₁), um den Gehalt an Hochsiedern zu reduzieren. Die am Kopf dieser integrierten Seitenstromkolonne austretenden Dämpfe (62) werden in dem oder den externen Kondensator(en) K₄C_{1-N} kondensiert und teilweise als Rücklauf (63) wieder auf den Kopf der zweiten Diarylcarbonat-Destillationskolonne zurückgeführt. Der restliche Teil des Kondensats wird als Destillat (51) (gereinigtes Diarylcarbonat) entnommen. Nicht kondensierte Dämpfe (66) werden ausgeschleust.

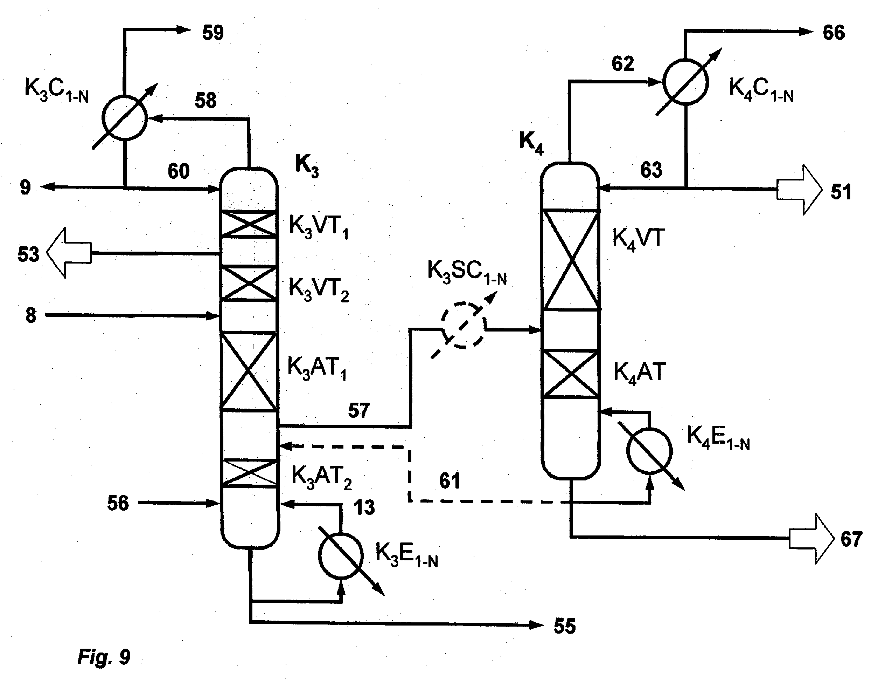

In einer weiteren besonders bevorzugten Variante der besonders bevorzugten Ausführungsform des erfindungsgemäßen Verfahrens unter Verwendung einer zweiten Diarylcarbonat-Destillationskolonne wird diese zweite Diarylcarbonat-Destillationskolonne sowohl mit wenigstens einem Verstärkungsteil als auch mit wenigstens einem Abtriebsteil ausgeführt. Eine solche besonders bevorzugte Variante dieser bevorzugten Ausfiihrungsform ist beispielhaft in Fig. 9 dargestellt.

Die in Fig. 9 dargestellte zweite Diarylcarbonat-Destillationskolonne K₄ weist sowohl einen Abtriebsteil K₄AT, als auch einen Verstärkungsteil K₄AT₂ auf. Der dampfförmige Seitenstrom 57 der ersten Diarylcarbonat-Destillationskolonne K₃ kann dabei zunächst in einem ein- oder mehrstufigen Seitenstromkondensator K₃SC_{1-N} kondensiert und anschließend der Kolonne K₄ zugeführt werden. Die zweite Diarylcarbonat-Destillationskolonne K₄ wird bevorzugt bei einem Kopfdruck von 1 bis 1000 mbar (absolut), bevorzugt 1 bis 100 mbar (absolut) und besonders bevorzugt 5 bis 50 mbar (absolut) betrieben. Dabei ergibt sich im Sumpf eine Temperatur von 150 bis 300 °C, bevorzugt von 160 bis 240 °C und besonders bevorzugt 180 bis 230°C °C.

Die zweite Diarylcarbonat-Destillationskolonne K₄ gemäß Fig. 9 weist vorzugsweise eine Gesamttrennleistung von 5 bis 100 Stufen, bevorzugt 10 bis 80 Stufen besonders bevorzugt 30 bis 80 Stufen, wobei der Verstärkungsteil davon eine Trennleistung von 1 bis 99, bevorzugt von 1 bis 79 und besonders bevorzugt von 2 bis 79 aufweist. Die Seitenstromkolonne K₄ wird bevorzugt bei einem Rücklaufverhältnis von 0,5 bis 20, bevorzugt 1 bis 10 und besonders bevorzugt 1 bis 5 betrieben.

Die Kondensation der Dämpfe (62) am Kopf der K₄ kann ein- oder mehrstufig in einem Kopfkondensator K₄C_{1-N} erfolgen. Sie erfolgt bevorzugt ein oder zweistufig in einem Temperaturbereich von 70 bis 250°C, besonders bevorzugt von 90 bis 230°C und ganz besonders bevorzugt von 90 bis 210°C. Die bei der Kondensation anfallende Abwärme kann bevorzugt zur Erzeugung von Heizdampf oder zur Beheizung anderer Verfahrensabschnitte, wie z.B. solchen bei der Herstellung von Diarylcarbonaten, verwendet werden. Das bei der Kondensation anfallende Kondensat wird teilweise wieder als Rücklauf (63) auf die zweite Diarylcarbonat-Destillationskolonne aufgegeben. Der restliche Teil des Kondensats wird als Destillat (51) (aufgereinigtes Diarylcarbonat) entnommen. Nicht kondensierte Dämpfe (66) werden ausgeschleust.

Die Verdampfung der aus dem Abtriebsteil K₄AT₁ der zweiten Diarylcarbonat-Destillationskolonne ablaufenden Flüssigkeit kann ebenfalls ein- oder mehrstufig in einem Verdampfer K₄E_{1-N} erfolgen.

Das Sumpfprodukt (67) der zweiten Diarylcarbonat-Destillationskolonne K₄ kann anschließend ganz oder teilweise aus dem Verfahren ausgeschleust werden und/oder ganz oder teilweise wieder der ersten Diarylcarbonat-Destillationskolonne K₃ zugeführt (61) werden.

Die vorangehend beschriebene besonders bevorzugte Ausführungsform des erfindungsgemäßen Verfahrens unter Verwendung einer zweiten Diarylcarbonat-Destillationskolonne eignet sich insbesondere für die Reinigung von Diarylcarbonaten mit erhöhten Anforderungen bezüglich deren Qualität. Solche erhöhten Anforderungen können beispielsweise in einem reduzierten Anteil hochsiedender Nebenkomponenten liegen, wobei deren Anteil im Diarylcarbonat um 10 bis 100 Gew.-%, bevorzugt 20 bis 90 Gew.-% und besonders bevorzugt 25 bis 80 Gew.-% gegenüber dem Verfahren mit nur einer Destillationskolonne reduziert werden kann.

In einer weiteren besonders bevorzugten Ausführungsform des erfindungsgemäßen Verfahrens kann die erste Diarylcarbonat-Destillationskolonne als Trennwandkolonne ausgeführt sein.

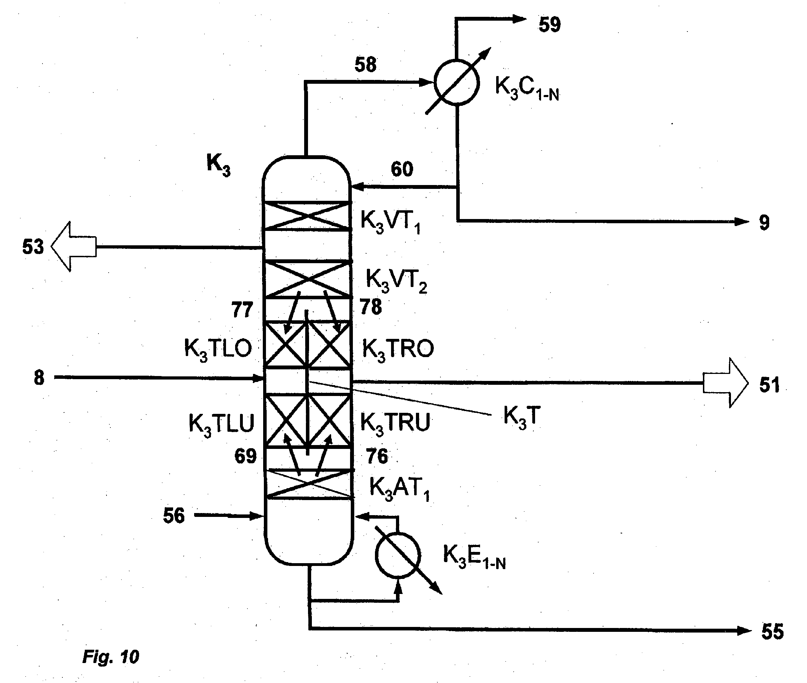

Eine solche Ausführungsform mit einer ersten Diarylcarbonat-Destillationskolonne als Trennwandkolonne mit sieben Sektionen ist beispielhaft in Fig. 10 dargestellt. Die Trennwandkolonne K₃ in Fig. 10 weist einen Abtriebsteil K₃AT₁ im unteren Teil der Kolonne K₃, jeweils eine obere Sektion K₃TLO und untere Sektion K₃TLU auf der Zulaufseite der Trennwand T und eine obere Sektion K₃TRO und untere Sektion K₃TRU auf der Entnahmeseite der Trennwand T sowie einen oberen Verstärkungsteil K₃VT₁ und unteren Verstärkungsteil K₃VT₂ im oberen Teil der Kolonne auf. Das Rohdiarylcarbonat (8) wird zwischen der oberen Sektion K₃TLO und der unteren Sektion K₃TLU auf der Zulaufseite der Trennwand T der Kolonne zugeführt, das aufgereinigte Diarylcarbonat (51) wird zwischen der oberen Sektion K₃TRO und der unteren Sektion K₃TRU auf der Entnahmeseite der Trennwand T der Kolonne entnommen.

Die auf der Zulaufseite der Trennwand befindliche obere Sektion K₃TLO dient zur Abtrennung von Schwersiedern, die im Zulauf enthalten sind. Die untere Sektion K₃TLU auf der Zulaufseite der Trennwand dient zur Abtrennung von Leichtsiedern, die im Rohdiarylcarbonat bzw. im Sumpf der zweiten Reaktionskolonne (8) enthalten sind. Die auf der Entnahmeseite der Trennwand befindliche obere Sektion K₃TRO dient zur Abtrennung von Leichtsiedern, die im aus dem Verstärkungsteil K₃VT₂ im oberen Teil der Kolonne austretenden Flüssigkeitsstrom (78) enthalten sind. Die untere Sektion der Entnahmeseite K₃TRU dient zur Abtrennung von Schwersiedern, die im aus dem Abtriebsteil K₃AT₁ austretenden Dampfstrom (76) enthalten sind.

Die Entnahme des gereinigten Diarylcarbonats auf der Entnahmeseite der Trennwand kann flüssig oder dampfförmig erfolgen. Beim Kolonnendesign kann die Art der Entnahme die Anordnung der Trennwand innerhalb der Kolonne mitunter deutlich beeinflussen. Die Trennwand kann jeweils zur Entnahmeseite oder zur Zulaufseite verschoben in der Kolonne angeordnet sein und so den Querschnitt der jeweiligen Seite gegenüber der anderen verkleinern oder vergrößern. Im Falle dampfförmiger Entnahme des gereinigten Diarylcarbonats auf der Entnahmeseite der Trennwand ist der Querschnitt der Entnahmeseite der Kolonne bevorzugt größer als der Querschnitt der Zulaufseite, d.h. es gelangt mehr Dampf aus dem Abtriebsteil in die Entnahmeseite. Im Falle flüssiger Entnahme des gereinigten Diarylcarbonats auf der Entnahmeseite der Trennwand ist der Querschnitt der Zulaufseite der Kolonne bevorzugt identisch zum Querschnitt der Entnahmeseite.

Im Falle der flüssigen Entnahme des gereinigten Diarylcarbonats, die beispielhaft in Fig. 10 dargestellt ist, werden 10 bis 90 %, bevorzugt 20 bis 90 % besonders bevorzugt 50 bis 85 % der aus dem Abtriebsteil K₃TRO der Entnahmeseite ablaufenden Flüssigkeit als Seitenstrom 51 entnommen. Die restliche Flüssigkeit wird der unteren Sektion K₃TRU der Entnahmeseite zugeführt. Die aus dem Verstärkungsteil K₃VT₂ ablaufende Flüssigkeit wird zu 5 bis 50 %, bevorzugt zu 10 bis 50 % und besonders bevorzugt 10 bis 40 % auf die Zulaufseite der Trennwand, also oberhalb von K₃TLO, aufgegeben (77). Die restliche Flüssigkeit wird am oberen Ende der Entnahmeseite der Trennwand, also auf K₃TRO aufgegeben (78). Der aus dem Abtriebsteil K₃AT₁ aufsteigende Dampf wird zu 5 bis 90 %, bevorzugt zu 10 bis 80 und besonders bevorzugt zu 20 bis 75 % der Zulaufseite der Trennwand zugeführt (69).

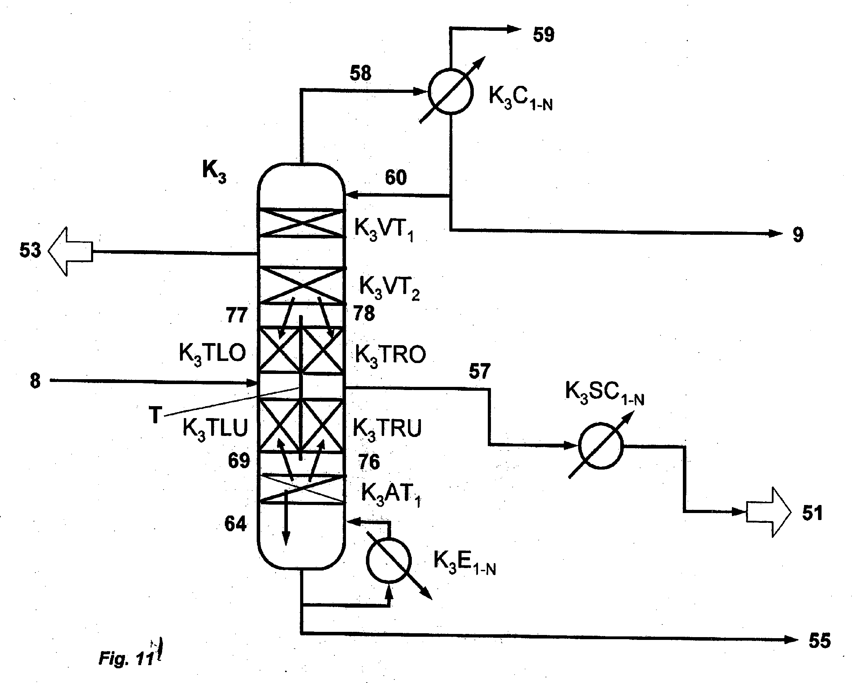

Im Falle der dampfförmigen Entnahme des gereinigten Diarylcarbonats, die beispielhaft in Fig. 11 dargestellt ist, werden 10 bis 90 %, bevorzugt 20 bis 90 % besonders bevorzugt 50 bis 85 % des aus der unteren Sektion K₃TRU der Entnahmeseite austretenden Dampfes als Seitenstrom (57) entnommen. Der restliche Dampf wird der oberen Sektion K₃TRO der Entnahmeseite zugeführt. Die aus dem Verstärkungsteil K₃VT₂ ablaufende Flüssigkeit wird zu 5 bis 90 %, bevorzugt zu 10 bis 80 % und besonders bevorzugt 20 bis 60 % auf die Zulaufseite der Trennwand, also oberhalb von K₃TLO, aufgegeben (77). Die restliche Flüssigkeit wird am oberen Ende der Entnahmeseite der Trennwand, also auf K₃TRO aufgegeben (78). Der aus dem Abtriebsteil K₃AT₁ aufsteigende Dampf wird zu 5 bis 90 %, bevorzugt zu 10 bis 80 und besonders bevorzugt zu 20 bis 60 % der Zulaufseite der Trennwand zugeführt (69). Im Falle der dampfförmigen Entnahme wird der dampfförmig entnommene Seitenstrom (57) bevorzugt in einem oder mehreren Seitenstromkondensator(en) K₃SC_{1-N} kondensiert und als flüssiger Diarylcarbonatstrom (51) abgeführt.

Der obere Verstärkungsteil einer solchen Trennwandkolonne weist bevorzugt eine Trennleistung von 0 bis 40, besonders bevorzugt 1 bis 20, ganz besonders bevorzugt 1 bis 10 theoretische Stufen, der untere Verstärkungsteil von bevorzugt 1 bis 40, besonders bevorzugt 5 bis 20, ganz besonders bevorzugt 5 bis 15 theoretische Stufen, der Abtriebsteil von bevorzugt 1 bis 40, besonders bevorzugt 2 bis 20, ganz besonders bevorzugt 2 bis 15 theoretische Stufen auf. Die obere Sektion K₃TLO und untere Sektion K₃TLU auf der Zulaufseite der Trennwand sowie die obere Sektion K₃TRO und untere Sektion K₃TRU auf der Entnahmeseite der Trennwand weisen bevorzugt jeweils eine Trennleistung von 1 bis 40, besonders bevorzugt 2 bis 20, ganz besonders bevorzugt 5 bis 20 theoretische Stufen auf.

Die Trennwandkolonne weist zudem einen ein- oder mehrstufigen (N-Stufigen) Kopfkondensator K₃C_{1-N} sowie einen ein- oder mehrstufigen (N-Stufigen) Verdampfer K₃E_{1-N} für das Sumpfprodukt auf.

Die Kondensation der Dämpfe am Kopf der Trennwandkolonne kann ein oder mehrstufig, bevorzugt ein- oder zweistufig, in einem Temperaturbereich von 40 bis 250°C, bevorzugt von 50 bis 200°C und besonders bevorzugt von 60 bis 180°C erfolgen. Hinsichtlich der Kondensation im Kopfkondensator sind die vorangehend bereits für die Destillationskolonnen genannten unterschiedlichen Ausführungsformen der Kondensatoren möglich.

Die aus dem Abtriebsteil K₃AT₁ ablaufende Flüssigkeit (64) wird in einer ein- oder mehrstufigen (N-stufigen) Verdampfung eingedampft, wobei die Dämpfe des dabei anfallenden Dampf-/Flüssigkeitsgemisches wieder dem unteren Abtriebsteil K₃AT₁ zugeführt werden. Die Verdampfung erfolgt vorzugsweise in einem Temperaturbereich von 100 bis 250°C, bevorzugt von 150 bis 240°C und besonders bevorzugt von 180 bis 220°C.

Die Trennwandkolonne wird bei einem Kopfdruck von 1 bis 1000 mbar (absolut), besonders bevorzugt von 1 bis 100 mbar (absolut) und ganz besonders bevorzugt von 5 bis 50 mbar (absolut) betrieben. Das Rücklaufverhältnis wird dabei so eingestellt, dass der Diarylcarbonat-Gehalt im Destillat 10 bevorzugt kleiner als 10 Gew.-%, besonders bevorzugt kleiner als 5 Gew.-% und ganz besonders bevorzugt kleiner 1 Gew.-%, bezogen auf das Gesamtgewicht des Destillats, beträgt. Hierzu wird bevorzugt ein Rücklaufverhältnis von 0,2 bis 5, besonders bevorzugt 0,2 bis 2 und ganz besonders bevorzugt von 0,3 bis 1,6 eingestellt, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführte Kondensat entspricht.

Enthält das zu reinigende Diarylcarbonat Verbindungen mit einem Siedepunkt zwischen dem des Diarylcarbonats und dem des während der Herstellung des Diarylcarbonats als Zwischenprodukt gebildeten Alkylarylcarbonats als Verunreinigung, können diese erfindungsgemäß in einem weiteren Seitenstrom (53) der Trennwandkolonne entnommen werden.

In einer bevorzugten Ausführungsform des erfindungsgemäßen Verfahrens erfolgt die Abtrennung mittelsiedender Nebenverbindungen in zwei Destillationskolonnen. Eine solche bevorzugte Ausführungsform ist in Fig. 6 dargestellt.

Die in Fig. 6 dargestellte erste Zwischensiederkolonne (Alkylarylether-Vorkonzentrierungskolonne) K₇ weist einen Abtriebsteil K₇AT mit 5 bis 40 theoretischen Stufen als auch einen Verstärkungsteil K₇VT mit 5 bis 40 theoretischen Stufen auf. Der bevorzugt flüssig zugeführt Strom aus der zweiten Reaktionskolonne K₂ (38) wird der Kolonne K₇ oberhalb des Abtriebsteils zugeführt. Die erste Zwischensiederkolonne K₇ wird vorzugsweise bei einem Kopfdruck von 50 bis 3000 mbar (absolut), bevorzugt 100 bis 2000 mbar (absolut) und besonders bevorzugt 500 bis 1500 mbar (absolut) betrieben.

Die erste Zwischensiederkolonne K₇ wird weiterhin vorzugsweise bei einem Rücklaufverhältnis von 0,1 bis 10, bevorzugt 0,5 bis 5 und besonders bevorzugt 0,5 bis 2 betrieben.

Die Kondensation der Dämpfe (44) am Kopf der K₇ kann ein- oder mehrstufig in einem Kopfkondensator K₇C_{1-N} erfolgen. Das bei der Kondensation anfallende Kondensat wird teilweise wieder als Rücklauf (45) auf die erste Zwischensiederkolonne K₇ aufgegeben. Der restliche Teil des Kondensats wird als Destillat (5), enthaltend Reaktionsalkohol und Dialkylcarbonat, entnommen. Nicht kondensierte Dämpfe (46) werden ausgeschleust.

Die Verdampfung der aus dem Abtriebsteil K₇AT der ersten Zwischensiederkolonne K₇ ablaufenden Flüssigkeit kann ein- oder mehrstufig in einem Verdampfer K₇E_{1-N} erfolgen.

Das Sumpfprodukt (43) der ersten Zwischensiederkolonne K₇, enthaltend aromatische Hydroxyverbindung und mittelsiedende Nebenverbindungen wird in Falle der in Fig. 6 dargestellten besonderen Ausführungsform einer zweiten Zwischensiederkolonne K₈ (Alkylarylether-Kolonne) zugeführt.

Die zweite Zwischensiederkolonne K₈ weist einen Abtriebsteil mit 2 Sektionen (K₈AT₁ und K₈AT₂), die jeweils eine Trennleistung von 5 bis 40 theoretischen Stufen haben als auch einen Verstärkungsteil K₈VT mit 5 bis 40 theoretischen Stufen auf. Das Sumpfprodukt der ersten Zwischensiederkolonne K₇ wird der zweiten Zwischensiederkolonne K₈ oberhalb des oberen Abtriebsteils K₈AT₁ zugeführt. Die zweite Zwischensiederkolonne K₈ wird vorzugsweise bei einem Kopfdruck von 50 bis 3000 mbar (absolut), bevorzugt 100 bis 2000 mbar (absolut) und besonders bevorzugt 500 bis 1500 mbar (absolut) betrieben.

Die zweite Zwischensiederkolonne K₈ wird weiterhin vorzugsweise bei einem Rücklaufverhältnis von 1 bis 1000, bevorzugt 10 bis 500 und besonders bevorzugt 50 bis 200 betrieben.

Die Kondensation der Dämpfe (47) am Kopf der K₈ kann ein- oder mehrstufig in einem Kopfkondensator K₈C_{1-N} erfolgen. Das bei der Kondensation anfallende Kondensat wird teilweise wieder als Rücklauf (48) auf die zweite Zwischensiederkolonne K₈ aufgegeben. Der restliche Teil des Kondensats wird als Destillat (40), enthaltend mittelsiedende Nebenverbindungen mit einem Siedepunkt unterhalb dem der aromatischen Hydroxyverbindung, entnommen. Nicht kondensierte Dämpfe (49) werden ausgeschleust.

Die Verdampfung der aus dem Abtriebsteil K₈AT der zweiten Zwischensiederkolonne K₈ ablaufenden Flüssigkeit kann ein- oder mehrstufig in einem Verdampfer K₈E_{1-N} erfolgen.

Das Sumpfprodukt (65) der zweiten Zwischensiederkolonne K₈, enthaltend aromatische mittelsiedende Nebenverbindungen und Alkylarylcarbonat, wird abhängig vom Gehalt an mittelsiedenden Nebenverbindungen mit einem Siedepunkt oberhalb dem der aromatischen Hydroxyverbindunge aus dem Verfahren ausgeschleust (41) oder wieder zurückgeführt (42).

Die aromatische Hydroxyverbindung wird bevorzugt als Seitenstrom, besonders bevorzugt als dampfförmiger Seitenstrom oberhalb des unteren Abtriebsteils K₈AT₂ entnommen. Im Falle der dampfförmigen Seitenstromentnahme wird der Strom (68) im Kondensator K₈SC_{1-N} kondensiert und wieder der Reaktion in der ersten Reaktionskolonne zugeführt (39). Die bei der Kondensation des dampfförmigen Seitenstroms (68) anfallende Kondensationswärme kann zur Erzeugung eines Wärmeträgers oder zur Energieintegration im Prozess verwendet werden.

In einer weiteren bevorzugten Ausführungsform kann die Abtrennung mittelsiedender Nebenverbindungen in zwei Destillationskolonnen erfolgen, wobei die zweite Zwischensiederkolonne als Trennwandkolonne ausgeführt ist. Eine solche besondere Ausführungsform ist in Fig. 12 dargestellt.

In diesem Falle wird der Seitenstrom (68) dampfförmig oder flüssig, besonders bevorzugt dampfförmig entnommen.

Die Trennwandkolonne K₈ weist vorzugsweise mindestens sechs Sektionen auf, einen Abtriebsteil K₈AT im unteren Teil der Kolonne K₈, jeweils eine obere Sektion K₈TLO und untere Sektion K₈TLU auf der Zulaufseite der Trennwand T und eine obere Sektion K₈TRO und untere Sektion K₈TRU auf der Entnahmeseite der Trennwand T sowie einen Verstärkungsteil K₈VT im oberen Teil der Kolonne mit jeweils 5 bis 40 theoretischen Stufen.

Die Erläuterungen zur ersten Zwischensiederkolonne K₇ sowie betreffend die weiteren Ströme und Kondensatoren und Verdampfer der zweiten Zwischensiederkolonne K₈ gemäß in Fig. 6 dargestellter bevorzugter Ausführungsform gelten für die bevorzugte Ausführungsform gemäß Fig. 12 analog.

Das folgende Beispiel dient der beispielhaften Erläuterung der Erfindung und sind nicht als Beschränkung aufzufassen.

### Beispiele

### Beispiel 1 (erfindungsgemäß)

Für das Beispiel kann ein Gesamtaufbau, wie in Fig. 14 gezeigt, verwendet werden.

In eine erste Reaktionskolonne (K₁) enthaltend einen oberen Verstärkungsteil (K₁VT₂) mit 4 theoretischen Stufen, einen Zwischenkondensator (K₁IC₁), einen unteren Verstärkungsteil (K₁VT₁) mit 4 theoretischen Stufen, eine Reaktionszone (K₁RZ) mit 30 Reaktionsböden (Hold-up: 12 1), wobei 3 Böden mit Heizelementen (K₁E_RZ₁₋₃) ausgestattet sind und einen Abtriebsteil K₁AT mit 6 Böden (Hold-up: 12 1) werden 399,3 kg/h eines Gemisches (18) aus 85.4 Gew.-% Phenol, 9.2 Gew.-% Dimethylcarbonat, 3.2 Gew.-% Diphenylcarbonat, 1.5 Gew.-% Titantetraphenolat, 0.3 Gew.-% Anisol, 0.3 Gew.-% Methylphenylcarbonat und 0.1 Gew.% Methanol am oberen Ende der Reaktionszone zudosiert. Am unteren Ende der Reaktionszone (K₁RZ) werden 539,6 kg/h eines um 5 °C überhitzten Dampfgemisches (17) aus 98.8 Gew.-% Dimethylcarbonat, 0.9 Gew.-% Phenol, 0.2 Gew.-% Anisol und 0.1 Gew.% Methanol zugeführt.

Dabei erhält man am Sumpf der Kolonne 452.4 kg/h eines Produktgemisches (6) aus 49.8 Gew.-% Phenol, 28.2 Gew.-% Methylphenylcarbonat, 12.3 Gew.-% Diphenylcarbonat, 8.1 Gew.-% Dimethylcarbonat, 0.2 Gew.-% Anisol und 1.4 Gew.-% Titantetraphenolat.

Die K₁ wird bei einem Kopfdruck (oberhalb von K₁VT₂) von 3.6 bar und einem Rücklaufverhältnis von 1.15 betrieben. Dabei wird im Sumpf der Kolonne eine Temperatur von 230 °C und in der Reaktionszone (K₁RZ) eine mittlere Reaktionstemperatur von 215 °C eingestellt. Ein Sumpfverdampfer K₁E₁ und drei Zwischenverdampfer (K₁E_RZ₁-₃) in der Reaktionszone werden mit Heizdampf bei einem Dampfdruck von 35 bar betrieben, wobei als Sumpfverdampfer (K₁E₁) ein Naturumlaufverdampfer verwendet wird und als Zwischenverdampfer auf den Reaktionsböden integrierte Heizregister verwendet werden. Die Eintrittstemperatur in den Zwischenkondensator (oberhalb von K₁IC₁) beträgt 205°C, die Austrittstemperatur 193 °C und die Kühlleistung 57 kW. Die bei der Zwischenkondensation anfallende Kondensationswärme kann zur Erzeugung von Heizdampf mit einem Heizdampfdruck von 8 bar (Tautemperatur: 170.4 °C) verwendet werden. Die erforderliche Heizleistung zur Verdampfung des Dimethylcarbonat enthaltenden Stromes (15) beträgt 52 kW. Die Verdampfung und Überhitzung des Dimethylcarbonates erfolgt bei einer Temperatur von 135 bis 152 °C, wozu der im Zwischenkondensator verwendete Dampf problemlos verwendet werden kann.

Das Sumpfprodukt (6) der ersten Reaktionskolonne (K₁) wird einer zweiten Reaktionskolonne (K₂), enthaltend einen Verstärkungsteil (K₂VT) mit 10 theoretischen Stufen und einen Abtriebsteil (K₂AT) mit 22 theoretischen Stufen zugeführt.

Zusätzlich werden 81,9 kg/h eines Gemisches (9) aus 69,9 Gew.-% Methylphenylcarbonat, 28.3 Gew.-% Phenol, 1.2 Gew.-% Dimethylcarbonat, 0,5 Gew.-% Diphenylether und 0,1 Gew.-% Diphenylcarbonat, aus der Diphenylcarbonat-Feindestillation (K₃) im Bereich des Abtriebsteils (K₂AT) zudosiert.

Dabei werden am Sumpf der zweiten Reaktionskolonne (K₂) 236,6 kg/h eines Produktgemisches (8) aus 62,8 Gew.-% Diphenylcarbonat, 24.2 Gew.-% Methylphenylcarbonat, 9.8 Gew.-% Phenol, 0.4 Gew.-% Dimethylcarbonat, 2.6 Gew.-% Titantetraphenolat und 0.2 Gew.-% Diphenylether erhalten.

Ferner werden 238.2 kg/h flüssiges Destillat (3) mit 83.5 Gew.-% Phenol, 15.5 Gew.-% Dimethylcarbonat, 0.6 Gew.-% Methylphenylcarbonat, 0.3 Gew.-% Anisol und 0.1 Gew.-% Methanol entnommen.

Das aus der zweiten Reaktionskolonne (K₂) kommende Dampfgemisch wird nur teilweise kondensiert, so dass auch 60 kg/h eines dampfförmigen Produktstromes (7) nach der Kondensation (K₂C₁₋₃) , zwecks Ausschleusung von mittelsiedenden Nebenverbindungen, insbesondere Anisol, entnommen werden. Dieser dampfförmige Produktstrom enthält 59.8 Gew.-% Dimethylcarbonat, 38.2 Gew.-% Phenol, 1.6 Gew.-% Methanol, 0.3 Gew.-% Anisol und 0,1 Gew.-% Methylphenylcarbonat.

Die zweite Reaktionskolonne (K₂) wird bei einem Kopfdruck (oberhalb von K₂VT) von 1 bar und einem Rücklaufverhältnis von 0.65 betrieben. Durch die Verwendung strukturierter Packungen in Verstärkungs- und Abtriebsteil liegt der Druckverlust in der Kolonne unter 50 mbar. Das aus Abtriebsteil (K₂AT) ablaufende Gemisch hat eine Temperatur von 198 °C und wird in einer zweistufigen Verdampfung zugeführt. Die Austrittstemperatur nach der ersten Verdampfungsstufe (K₂E₁) beträgt 209 und nach der zweiten Verdampferstufe (K₂E₂) 230 °C. Als Verdampfer werden in der ersten Stufe ein Naturumlaufverdampfer und in der zweiten Stufe ein Kettle-type-Verdampfer verwendet. Die gesamte Verdampferleistung beträgt 66.4 kW. Da der Katalysator nicht flüchtig ist beschränkt sich die Reaktion auf den Abtriebsteil, den Kolonnesumpf und die Verdampfer. Aufgrund der vergleichsweise geringen Temperaturen im Abtriebsteil (188 -198 °C) findet die Reaktion vorwiegend im Kolonnensumpf und den Verdampfern statt.

Die Kondensation des am Kopf der zweiten Reaktionskolonne entnommenen Dampfgemisches (27) erfolgt 3-stufig und zwar in der 1. Stufe bei 174 - 165 °C (45 kW) der 2. Stufe bei 165 - 155 °C (17 kW) und der 3. Stufe bei 155 - 154 °C (1KW). Die Kondensationswärme der 1. und 2. Stufe wird zur Trennung eines Gemisches aus Dimethylcarbonat und Methanol verwendet.

Das Destillat (4) der ersten Reaktionskolonne K₁ mit einer Gesamtmenge von 486.6 kg/h enthält 90.6 Gew.-% Dimethylcarbonat, 8.2 Gew.-% Methanol, 1 Gew.-% Phenol und 0.2 Gew.-% Anisol und wird zusammen mit 36,6 kg/h eines weiteren Stromes (5), enthaltend 97.3 Gew.-% Dimethylcarbonat und 2.7 Gew.-% Methanol, einer Aufarbeitung in zwei Destillationskolonnen K₅ und K₆ zwecks Abtrennung des Methanols und Rückgewinnung des Dimethylcarbonat zugeführt.

Dabei erhält man 482 kg/h einer Dimethylcarbonat-Fraktion (11) mit 98.75 Gew.-% Dimethylcarbonat, 1 Gew.-% Phenol, 0.2 Gew.-% Anisol und 0.05 Gew.-% Methanol und 41 kg/h einer Methanol-Fraktion (10) mit 99.5 Gew.-% Methanol und 0.5 Gew.-% Dimethylcarbonat erhalten.

Da Methanol und Dimethylcarbonat ein Azeotrop bilden, wird die Trennung des Gemisches unter Anwendung des Zweidruckverfahrens durchgeführt. Dabei wird das Gemisch zunächst in einem Vorwärmer auf 137 °C erhitzt und dabei auch teilweise verdampft, anschließend in einer Dimethylcarbonat-Destillationskolonne K₅ zunächst in die vorangehend genannte Dimethylcarbonat-Fraktion als Sumpfprodukt (11) und 113.4 kg/h einer Fraktion mit nahezu azeotroper Zusammensetzung (13) mit 76.1 Gew.-% Methanol und 23.9 Gew.-% Dimethylcarbonat als Destillat zerlegt.

Die Dimethylcarbonat-Destillationskolonne (K₅) arbeitet bei einem Kopfdruck von 5,5 bar und einem Rücklaufverhältnis von 1,2 und hat einen Verstärkungsteil (K₅VT) mit 16 theoretischen Stufen und einen Abtriebsteil (K₅AT) mit 7 theoretischen Stufen.

Dabei ergibt im Sumpf der Kolonne sich eine Temperatur von 155,8 °C. Die erforderliche Verdampfungswärme beträgt 58,2 kW. Die Verdampfung des Sumpfproduktes erfolgt in zwei Naturumlaufverdampfern (K₅E₁₋₂), wobei der größte Teil der Wärme (45 kW) in einem Umlaufverdampfer, der gleichzeitig als erster Kondensator der zweiten Reaktionskolonne fungiert, ausgetauscht wird. Die restliche Verdampfungswärme wird in einem zweiten Umlaufverdampfer mittels Heizdampf bereitgestellt.

Der Wärmetauscher zur Vorwärmung des Zustroms der Dimethylcarbonat-Destillationskolonne (K₅W₁) fungiert gleichzeitig als zweiter Kondensator der zweiten Reaktionskolonne, wobei die übertragene Wärmemenge 17 kW beträgt.

In einer Methanol-Destillationskolonne (K₆), die bei einem Kopfdruck von 600 mbar und einem Rücklaufverhältnis von 2,3 arbeitet, wird Methanol als Sumpfprodukt (41 kg/h; methanol/Dimethylcarbonat 99,5/0,5 Gew.-%) abgetrennt. Das Destillat (12) mit einer Gesamtmenge von 72.3 kg/h , 62.4 Gew.-% Methanol und 37.6 Gew.-% Dimethylcarbonat wird erneut der Dimethylcarbonat-Destillationskolonne zugeführt.

Die Methanol-Destillationskolonne (K₆) hat eine Trennleistung von 30 theoretischen Stufen, die sich zu gleichen Teilen auf Verstärkungs- und Abtriebsteil aufteilt.

Die im Verdampfer der Methanol-Destillationskolonne erforderliche Wärme (56 kW) wird durch die Kondensation der Dämpfe aus der Dimethylcarbonat-Destillationskolonne bereitgestellt. Der Kondensator der Dimethylcarbonat-Destillationskolonne fungiert somit gleichzeitig als Verdampfer der Methanol-Destillationskolonne.

Das in der zweiten Reaktionskolonne (K₂) erhaltene Sumpfgemisch (8) mit 62.7/24.2/9.8/0.4/2.6/0,03 Gew.-% Diphenylcarbonat / Methylphenylcarbonat / Phenol /Dimethylcarbonat/Titantetraphenolat/Salol und einer Gesamtmenge von 236,6 kg/h wird einer destillativen Aufarbeitung zwecks Isolierung des Diphenylcarbonat, Abtrennung von Hochsiedern und Katalysator und leichtsiedenden Verbindungen zugeführt. Diese besteht aus einer Diphenylcarbonat-Feindestillationskolonne K₃ und einer Diphenylcarbonat-Seitenstromkolonne K₄ gemäß Fig. 4.

Die Diphenylcarbonat-Feindestillationskolonne (K₃) besteht aus vier Sektionen, einem oberen Verstärkungsteil (K₃VT₁) mit 5, einem unteren Verstärkungsteil (K₃VT₂) mit 3, einem oberen Abtriebsteil (K₃AT₁) mit 16 und einem unteren Abtriebsteil (K₃AT₂) mit 9 theoretischen Stufen. Die Kondensation der am Kopf der Kolonne austretenden Dämpfe im Kopfkondensator (K₃C₁) und die teilweise Verdampfung der aus dem unteren Abtriebsteil (K₃AT₂) ablaufenden Flüssigkeit im Verdampfer (K₃E₁) für das Sumpfprodukt erfolgt jeweils einstufig.

Die Diphenylcarbonat-Feindestillationskolonne (K₃) wird bei einem Kopfdruck von 15 mbar und einem Rücklaufverhältnis von 0,7 betrieben.

Dabei erhält man als Destillat (9) einen Strom mit 69,9/28,3/1,2/0,5 Gew.-% Methylphenylcarbonat/Phenol/Dimethylcarbonat/DPE. Unterhalb des oberen Verstärkungsteils (K₃VT₁) werden 0,02 kg/h Flüssigkeit zwecks Ausschleusung von Zwischensiedern im Seitenstrom (53) entnommen. Ferner werden unterhalb des oberen Verstärkungsteils (K₃VT₁) 201 kg/h eines dampfförmigen Seitenstroms (57) mit 99,9 Gew.-% Diphenylcarbonat entnommen. Als Sumpfprodukt (55) erhält man 20,6 kg/h eines Gemisches mit 70/29,8/0,2 Gew.-% Diphenylcarbonat/Titantetraphenolat/Salol.

Der dampfförmige Seitenstrom (57) wird einer Seitenstromkolonne (K₄) zugeführt. Diese verfügt lediglich über einen Verstärkungsteil (K₄VT) mit 9 theoretischen Stufen.

Die Seitenstromkolonne (K₄) wird unter gleichen Druckbedingungen wie die Diphenylcarbonat-Feindestillationskolonne (K₃) und bei einem Rücklaufverhältnis von 0,5 betrieben.

Die am Kopf der Seitenstromkolonne (K₄) austretenden Dämpfe (62) werden in einer zweistufigen Kondensation in den Kondensatoren (K₄C₁₋₂)kondensiert, wobei die Kondensationswärme entweder zur Erzeugung von Dampf oder zur Erwärmung anderer Prozessabschnitte der Diphenylcarbonat-Herstellung verwendet wird.

Dabei erhält man ein Destillat (51) mit 99,96 Gew.-% Diphenylcarbonat und nur 300 ppm Salol. Die am Sumpf der Seitenstromkolonne ablaufende Flüssigkeit (61) wird der Diphenylcarbonat-Feindestillationskolonne (K₃) oberhalb des unteren Abtriebsteils (K₃AT₂) zugeführt.

Das nach der Kondensation der zweiten Reaktionskolonne (K₂C₁₋₃) verbleibende Brüdengemisch wird gegebenenfalls zunächst kondensiert (K₂C₄) und anschließend einer weiteren Aufarbeitung zur Abtrennung von mittelsiedenden Nebenverbindungen, insbesondere Anisol, zugeführt.

Die Aufarbeitung erfolgt in zwei Destillationskolonnen. In einer Anisol-Vorkonzentrierungskolonne (K₇) wird eine Leichtsiederfraktion als Kopfprodukt entnommen. Diese enthält 97.3 Gew.-% Dialkylcarbonat, und 2.7 Gew.-% Reaktionsalkohol und hat eine Gesamtmenge von 36.6 kg/h. Als Sumpfprodukt werden 23 kg/h eines Gemisches enthaltend 99.0/0.1/0.65 Gew.-% Phenol/Dimethylcarbonat/Anisol entnommen.

Die Anisol-Vorkonzentierungskolonne (K₇) weist einen Verstärkungsteil (K₇VT) mit 8 theoretischen Stufen und einen Abtriebsteil (K₇AT) mit 14 theoretischen Stufen auf. Die Kondensation (K₇C₁) der am Kopf der Kolonne entnommenen Brüden (44) erfolgt einstufig bei einer Kondensationstemperatur von 88 bis 80 °C. Die Kolonne wird bei einem Kopfdruck von 1bar und einem Rücklaufverhältnis von 0,8 betrieben. Die Energiezufuhr im Sumpf der Kolonne erfolgt über Naturumlaufverdampfer (K₇E₁).

Das Sumpfprodukt (43) der Anisol-Vorkonzentierungskolonne (K₇) wird der Anisolkolonne (K₈) zugeführt. Als Destillat der Anisolabtrennung (5) werden 0.2 kg/h einer Mischung 46.4/41.3/9.8/2.5 Gew.-% Phenol/Anisol/Dimethylcarbonat/sonstige mittelsiedende Nebenverbindungen entnommen. Die Seitenstromentnahme (68) erfolgt dampfförmig. Der entnommene Dampfstrom enthält Phenol mit einer Reinheit von 99,7 Gew.-%. Dieser wird kondensiert und wieder der ersten Reaktionskolonne (K₁), nach Vermischung mit weiteren, das Phenol und gegebenenfalls den Katalysator enthaltenden Strömen, zugeführt. Die bei der Kondensation (K₈SC₁) anfallende Kondensationswärme wird zur Erzeugung von Heizdampf mit einem Heizdampfdruck von 7 bar absolut verwendet.

Im Sumpf der Anisolabtrennung werden 0,1 kg/h (41) entnommen und vollständig aus dem Verfahren ausgeschleust.

Die Anisolkolonne (K₈) weist einen Verstärkungsteil (K₈VT) mit 14 theoretischen Stufen und einen Abtriebsteil (K₈AT) mit 14 theoretischen Stufen auf, wobei der Abtriebsteil aus einem oberen (K₈AT₁) mit 10 und einem unteren Abtriebsteil (K₈AT₂) mit 4 theoretischen Stufen besteht. Die Entnahme des Seitenstroms erfolgt oberhalb des unteren Abtriebsteils (K₈AT₂). Die Kondensation (K₈C₁) der am Kopf der Kolonne entnommenen Brüden (47) erfolgt einstufig. Die Kolonne wird unter Rückflussbedingungen bei einem Rücklaufverhältnis von 84 betrieben. Die Energiezufuhr im Sumpf der Kolonne erfolgt über einen Fallfilmverdampfer (K₈E₁). Der Kopfdruck der Kolonne beträgt 1 bar absolut.

Das Beispiel zeigt in eindrucksvoller Weise, wie der Energieverbrauch bei der Herstellung von Diphenylcarbonat durch effiziente Wärmeintegration deutlich reduziert werden kann.

So wird in der ersten Reaktionskolonne durch die Verwendung eines Zwischenkondensators der Wärmebedarf einschließlich der Erhitzung und Verdampfung der Edukte, Verdampfung im Sumpf der Kolonne und Beheizung der Reaktionszone von 183.3 auf 131.3 KW, also um 28.4 % reduziert. Gleichzeitig reduziert sich der Kühlmittelverbrauch von 183.2 auf 126.2 kW, somit um 31.1 %.

Durch die Wärmeintegration der zweiten Reaktionskolonne mit der Trennung des Methanol/Dimethylcarbonat-Gemisches wird der Heizmittelbedarf zur Trennung von Methanol und Dimethylcarbonat von 76 kW auf 13 kW also um 83 % reduziert werden. Gleichzeitig reduziert sich der Kühlmittelbedarf der zweiten Reaktionskolonne von 64 auf 1 KW also um 98,4 %.

## Patentansprüche

1. Verfahren zur Herstellung von wenigstens einem Diarylcarbonat aus wenigstens einem Dialkylcarbonat und wenigstens einer aromatischen Hydroxyverbindung, wobei
(a) das oder die Dialkylcarbonat(e) in Gegenwart wenigstens eines Umesterungskatalysators mit der oder den aromatischen Hydroxyverbindung(en) in einer ersten Reaktionskolonne enthaltend wenigstens einen Verstärkungsteil im oberen Teil der Kolonne und wenigstens eine Reaktionszone unterhalb des Verstärkungsteils, welches mindestens zwei Sektionen aufweist, umgesetzt werden,
(b) das Sumpfprodukt der ersten Reaktionskolonne wenigstens einer weiteren Reaktionskolonne enthaltend wenigstens einen Verstärkungsteil im oberen Teil der Kolonne und wenigstens eine Reaktionszone unterhalb des Verstärkungsteils zugeführt und in dieser weiter umgesetzt wird,
(c) das in den Reaktionskolonnen der Schritte (a) und/oder (b) nicht umgesetzte oder während der Reaktion gebildete Dialkylcarbonat ganz oder teilweise in wenigstens einem weiteren Verfahrensschritt enthaltend wenigstens eine Destillationskolonne vom während der Reaktion gebildeten Alkylalkohol getrennt wird,
(d) der am Kopf wenigstens einer Reaktionskolonne aus (b) gegebenenfalls nach Kondensation in wenigstens einem Kondensator entnommene aromatische Hydroxyverbindung(en) enthaltende Dampf ganz oder teilweise wenigstens einem weiteren Verfahrensschritt enthaltend wenigstens eine Destillationskolonne zugeführt wird, wobei Verbindungen, deren Siedepunkt zwischen dem des Dialkylcarbonats und dem des während der Herstellung des Diarylcarbonats gebildeten Alkylarylcarbonats liegt, abgetrennt werden, und
(e) das in der oder den weiteren Reaktionskolonne(n) aus Schritt (b) erhaltene Sumpfprodukt enthaltend Diarylcarbonat wenigstens einem weiteren Verfahrensschritt zur Reinigung in wenigstens einer Destillationskolonne enthaltend wenigstens einen Verstärkungsteil im oberen Teil der Kolonne und wenigstens einen Abtriebsteil im unteren Teil der Kolonne zugeführt wird,
wobei wenigstens eine der Reaktionskolonne(n) ausgewählt aus der ersten oder der oder den weitere(n) Reaktionskolonne(n) mit einem oder mehreren Kondensatoren ausgestattet ist und die durch Kondensation in diesen Kondensatoren gewonnene Kondensationswärme direkt oder indirekt wieder in das Verfahren zurückgeführt wird,
wobei gegebenenfalls wenigstens ein Verstärkungsteil der ersten Reaktionskolonne mit wenigstens einem Zwischenkondensator ausgestattet ist und die durch Kondensation in diesem Zwischenkondensator gewonnene Kondensationswärme direkt oder indirekt wieder in das Verfahren zurückgeführt wird,
und wobei der Betriebsdruck in der oder den Trenn-Destillationskolonne(n) des Verfahrensschritts zur Trennung von Dialkylcarbonat und Alkylalkohol so eingestellt wird, dass die Verdampfungstemperatur im Sumpf der Trenn-Destillationskolonne(n) des Verfahrensschritts zur Trennung von Dialkylcarbonat und Alkylalkohol unter der Kondensationstemperatur in dem oder den Kondensator(en) der weiteren Reaktionskolonne(n) und/oder dem oder den gegebenenfalls vorhandenen Zwischenkondensator(en) der ersten Reaktionskolonne liegt, und die durch Kondensation in diesen Kondensator(en) gewonnene Kondensationswärme direkt oder indirekt, ganz oder teilweise zur Trennung des Dialkylcarbonats vom während der Reaktion gebildeten Alkylalkohol verwendet wird.

2. Verfahren gemäß Anspruch 1, **dadurch gekennzeichnet, dass** wenigstens ein Verstärkungsteil der ersten Reaktionskolonne mit wenigstens einem Zwischenkondensator ausgestattet ist und die durch Kondensation in diesem Zwischenkondensator gewonnene Kondensationswärme direkt oder indirekt wieder in das Verfahren zurückgeführt wird.

3. Verfahren gemäß Anspruch 1 oder 2, **dadurch gekennzeichnet, dass** die weitere(n) Reaktionskolonne(n) mit einem oder mehreren Kondensatoren am Kopf der weiteren Reaktionskolonne(n) ausgestattet sind und die durch Kondensation in diesen Kondensatoren gewonnene Kondensationswärme direkt oder indirekt wieder in das Verfahren zurückgeführt wird.

4. Verfahren gemäß wenigstens einem der Ansprüche 1 bis 3, **dadurch gekennzeichnet, dass** die durch Kondensation in dem oder den Kondensator(en) der weiteren Reaktionskolonne(n) und/oder dem oder den gegebenenfalls vorhandenen Zwischenkondensator(en) der ersten Reaktionskolonne gewonnene Kondensationswärme direkt oder indirekt teilweise zur Trennung des Dialkylcarbonats vom während der Reaktion gebildeten Alkylalkohol und teilweise zur Verdampfung des in die erste Reaktionskolonne eingeleiteten Dialkylcarbonats verwendet wird.

5. Verfahren gemäß wenigstens einem der Ansprüche 1 bis 4, **dadurch gekennzeichnet, dass** die durch Kondensation in dem oder den Kondensator(en) der weiteren Reaktionskolonne(n) gewonnene Kondensationswärme direkt oder indirekt, ganz oder teilweise zur Trennung des Dialkylcarbonats vom während der Reaktion gebildeten Alkylalkohol verwendet wird und die durch Kondensation in dem oder den Zwischenkondensator(en) der ersten Reaktionskolonne gewonnene Kondensationswärme direkt oder indirekt, ganz oder teilweise zur Verdampfung des in die erste Reaktionskolonne eingeleiteten Dialkylcarbonats verwendet wird.

6. Verfahren gemäß wenigstens einem der Ansprüche 1 bis 5, **dadurch gekennzeichnet, dass** das in der oder den weiteren Reaktionskolonne(n) aus Schritt (b) erhaltene Sumpfprodukt enthaltend Diarylcarbonat Umesterungskatalysator aus der Herstellung des Diarylcarbonats enthält.

7. Verfahren gemäß wenigstens einem der Ansprüche 1 bis 6, **dadurch gekennzeichnet, dass** das in der oder den weiteren Reaktionskolonne(n) aus Schritt (b) erhaltene Sumpfprodukt enthaltend Diarylcarbonat wenigstens einem weiteren Verfahrensschritt zur Reinigung zugeführt wird, welcher wenigstens eine Destillationskolonne aufweist, wobei dieser Destillationskolonne ein Diarylcarbonat-haltiger Seitenstrom entnommen wird.

8. Verfahren gemäß Anspruch 7, **dadurch gekennzeichnet, dass** das in der oder den weiteren Reaktionskolonne(n) aus Schritt (b) erhaltene Sumpfprodukt enthaltend Diarylcarbonat Verbindungen mit einem Siedepunkt zwischen dem des Diarylcarbonats und dem des während der Herstellung des Diarylcarbonats als Nebenprodukt gebildeten Alkylarylcarbonats als Verunreinigung enthält, welche in einem weiteren Seitenstrom der Destillationskolonne entnommen und gegebenenfalls in die oder eine der weiteren Reaktionskolonne(n) aus Schritt (b) zurückgeführt werden.

9. Verfahren gemäß Anspruch 7 oder 8, **dadurch gekennzeichnet, dass** es sich bei der Destillationskolonne um eine Trennwandkolonne handelt.

10. Verfahren gemäß wenigstens einem der Ansprüche 1 bis 9, **dadurch gekennzeichnet, dass** das unter (c) abgetrennte Dialkylcarbonat gegebenenfalls nach weiterer Aufreinigung wieder der ersten Reaktionskolonne zugeführt wird.

11. Verfahren gemäß wenigstens einem der Ansprüche 1 bis 10, **dadurch gekennzeichnet, dass** die in dem oder den Verfahrensschritt(en) zur Abtrennung von Verbindungen, deren Siedepunkt zwischen dem des Dialkylcarbonats und dem des während der Herstellung des Diarylcarbonats gebildeten Alkylarylcarbonats liegt, aus dem am Kopf wenigstens einer Reaktionskolonne aus (b) gegebenenfalls nach Kondensation in wenigstens einem Kondensator ganz oder teilweise entnommenen aromatische Hydroxyverbindung(en) enthaltenden Dampf erhaltene(n) aromatische(n) Hydroxyverbindung(en) wieder der ersten Reaktionskolonne zugeführt wird.

12. Verfahren gemäß wenigstens einem der Ansprüche 1 bis 11, **dadurch gekennzeichnet, dass** der am Kopf wenigstens einer Reaktionskolonne aus (b) gegebenenfalls nach Kondensation in wenigstens einem Kondensator entnommene aromatische Hydroxyverbindung(en) enthaltende Dampf wenigstens einem weiteren Verfahrensschritt enthaltend wenigstens zwei Destillationskolonnen zugeführt wird, wobei das Sumpfprodukt der ersten Destillationskolonne einer zweiten Destillationskolonne zugeführt wird.

13. Verfahren gemäß Anspruch 11 oder 12, **dadurch gekennzeichnet, dass** die in dem oder den Verfahrensschritt(en) zur Abtrennung von Verbindungen, deren Siedepunkt zwischen dem des Dialkylcarbonats und dem des während der Herstellung des Diarylcarbonats gebildeten Alkylarylcarbonats liegt, erhaltene(n) aromatische(n) Hydroxyverbindung(en) einer ersten und einzigen Destillationskolonne als Sumpfprodukt oder einer zweiten oder weiteren Destillationskolonne als Seitenstrom entnommen werden.

14. Verfahren gemäß wenigstens einem der Ansprüche 11 bis 13, **dadurch gekennzeichnet, dass** das am Kopf der ersten Destillationskolonne entnommene Produkt Dialkylcarbonat enthält und ganz oder teilweise dem weiteren Verfahrensschritt (c) enthaltend wenigstens eine Destillationskolonne zur Abtrennung des Alkylalkohols zugeführt wird.

15. Verfahren gemäß wenigstens einem der Ansprüche 1 bis 14, **dadurch gekennzeichnet, dass** wenigstens eine der im Verfahren eingesetzten Reaktionskolonnen und/oder wenigstens eine der im Verfahren eingesetzten Destillationskolonnen einen oder mehrere Kopfkondensatoren aufweist, welche in die Kolonne integriert sind, wobei das Verhältnis d/D von Durchmesser der Dampfleitung von der Kolonne zu dem oder den Kopfkondensator(en) (d) zu Kolonnendurchmesser der Kolonne (D) im Bereich von 0,2 bis 1 liegt.

16. Verfahren nach wenigstens einem der Ansprüche 1 bis 15, **dadurch gekennzeichnet, dass** die Leitungen und Aggregate, die Gemische führen, welche einen Festpunkt von mehr als 30°C, bevorzugt mehr als 40°C aufweisen, auf Temperaturen oberhalb dieses Festpunktes beheizt werden.

17. Verfahren nach wenigstens einem der Ansprüche 1 bis 16, **dadurch gekennzeichnet, dass**
a. aus dem Sumpfprodukt wenigstens einer Destillationskolonne aus Verfahrensschritt (e) ein katalysatorhaltiger Strom erhalten wird, welcher ganz oder teilweise gegebenenfalls nach weiterer Aufreinigung wieder in das Verfahren, bevorzugt in Verfahrensschritt (a) zurückführt wird,
b. aus wenigstens einer Destillationskolonne aus Verfahrensschritt (e) ein aromatische Hydroxyverbindung(en) und Alkylarylcarbonat enthaltender Strom erhalten wird, welcher ganz oder teilweise wieder in das Verfahren, bevorzugt in Verfahrensschritt (a) oder (b) zurückführt wird, und
c. aus wenigstens einer Destillationskolonne aus Verfahrensschritt (e) zusammen oder getrennt voneinander Verbindungen mit einem Siedepunkt oberhalb des Siedepunktes des Diarylcarbonats und Verbindungen, deren Siedepunkt zwischen dem des Dialkylcarbonats und dem des während der Herstellung des Diarylcarbonats gebildeten Alkylarylcarbonats liegt, ganz oder teilweise aus dem Verfahren ausgeschleust werden.

## Claims

1. Process for preparing at least one diaryl carbonate from at least one dialkyl carbonate and at least one aromatic hydroxyl compound, by
(a) reacting the dialkyl carbonate(s) in the presence of at least one transesterification catalyst with the aromatic hydroxyl compound(s) in a first reaction column comprising at least one rectifying section in the upper part of the column and at least one reaction zone below the rectifying section which has at least two sectors,
(b) feeding the bottom product of the first reaction column to at least one further reaction column comprising at least one rectifying section in the upper part of the column and at least one reaction zone below the rectifying section, and converting it further therein,
(c) separating the dialkyl carbonate unconverted in the reaction columns of steps (a) and/or (b) or formed during the reaction fully or partly from the alkyl alcohol formed during the reaction in at least one further process step comprising at least one distillation column,
(d) feeding the vapour containing aromatic hydroxyl compound(s) withdrawn at the top of at least one reaction column from (b), optionally after condensation in at least one condenser, fully or partly to at least one further process step comprising at least one distillation column to remove compounds whose boiling point is between that of the dialkyl carbonate and that of the alkyl aryl carbonate formed during the preparation of the diaryl carbonate, and
(e) feeding the bottom product which comprises diaryl carbonate and is obtained in the further reaction column (s) from step (b) to at least one further process step for purification in at least one distillation column comprising at least one rectifying section in the upper part of the column and at least one stripping section in the lower part of the column,
wherein at least one of the reaction column(s) selected from the first reaction column or the further reaction column(s) is equipped with one or more condensers and the heat of condensation obtained by condensation in these condensers is recycled directly or indirectly back into the process, wherein at least one rectifying section of the first reaction column is optionally equipped with at least one intermediate condenser, and the heat of condensation obtained by condensation in this intermediate condenser is recycled directly or indirectly back into the process, and wherein the operating pressure in the separating distillation column(s) of the process step for separation of dialkyl carbonate and alkyl alcohol is adjusted such that the evaporation temperature in the bottom of the separating distillation column(s) of the process step for separation of dialkyl carbonate and alkyl alcohol is below the condensation temperature in the condenser(s) of the further reaction column(s) and/or any intermediate condenser(s) present in the first reaction column, and the heat of condensation obtained by condensation in this (these) condenser(s) is used directly or indirectly, fully or partly, to separate the dialkyl carbonate from the alkyl alcohol formed during the reaction.

2. Process according to Claim 1, **characterized in that** at least one rectifying section of the first reaction column is equipped with at least one intermediate condenser and the heat of condensation obtained by condensation in this intermediate condenser is recycled directly or indirectly back into the process.

3. Process according to Claim 1 or 2, **characterized in that** the further reaction column(s) is/are equipped with one or more condensers at the top of the further reaction column (s), and the heat of condensation obtained by condensation in these condensers is recycled directly or indirectly back into the process.

4. Process according to at least one of Claims 1 to 3, **characterized in that** the heat of condensation obtained by condensation in the condenser(s) of the further reaction column(s) and/or any intermediate condenser(s) present in the first reaction column is used directly or indirectly partly to separate the dialkyl carbonate from the alkyl alcohol formed during the reaction and partly to evaporate the dialkyl carbonate introduced into the first reaction column.

5. Process according to at least one of Claims 1 to 4, **characterized in that** the heat of condensation obtained by condensation in the condenser(s) of the further reaction column(s) is used directly or indirectly, fully or partly, to separate the dialkyl carbonate from the alkyl alcohol formed during the reaction, and the heat of condensation obtained by condensation in the intermediate condenser(s) of the first reaction column is used directly or indirectly, fully or partly, to evaporate the dialkyl carbonate introduced into the first reaction column.

6. Process according to at least one of Claims 1 to 5, **characterized in that** the bottom product which comprises diaryl carbonate and is obtained in the further reaction column(s) from step (b) comprises transesterification catalyst from the preparation of the diaryl carbonate.

7. Process according to at least one of Claims 1 to 6, **characterized in that** the bottom product which comprises diaryl carbonate and is obtained in the further reaction column(s) from step (b) is fed to at least one further process step for purification, which has at least one distillation column, a diaryl carbonate-containing sidestream being withdrawn from this distillation column.

8. Process according to Claim 7, **characterized in that** the bottom product which comprises diaryl carbonate and is obtained in the further reaction column(s) from step (b) comprises compounds having a boiling point between that of the diaryl carbonate and that of the alkyl aryl carbonate formed as a by-product during the preparation of the diaryl carbonate as an impurity, which are withdrawn from the distillation column in a further sidestream and optionally recycled into the reaction column or one of the further reaction column(s) from step (b).

9. Process according to Claim 7 or 8, **characterized in that** the distillation column is a dividing wall column.

10. Process according to at least one of Claims 1 to 9, **characterized in that** the dialkyl carbonate removed in (c) is fed back to the first reaction column, optionally after further purification.

11. Process according to at least one of Claims 1 to 10, **characterized in that** the aromatic hydroxyl compound(s) obtained from the vapour which comprises aromatic hydroxyl compound(s) and is withdrawn fully or partly at the top of at least one reaction column from (b), if appropriate after condensation in at least one condenser, in the process step(s) for removal of compounds whose boiling point is between that of the dialkyl carbonate and that of the alkyl aryl carbonate formed during the preparation of the diaryl carbonate is/are fed to the first reaction column.

12. Process according to at least one of Claims 1 to 11, **characterized in that** the vapour which comprises aromatic hydroxyl compound(s) and is withdrawn at the top of at least one reaction column from (b), if appropriate after condensation in at least one condenser, is fed to at least one further process step comprising at least two distillation columns, the bottom product of the first distillation column being fed to a second distillation column.

13. Process according to Claim 11 or 12, **characterized in that** the aromatic hydroxyl compound(s) obtained in the process step(s) for removal of compounds whose boiling point is between that of the dialkyl carbonate and that of the alkyl aryl carbonate formed during the preparation of the diaryl carbonate is/are withdrawn from a first and only distillation column as a bottom product or from a second or further distillation column as a sidestream.

14. Process according to at least one of Claims 11 to 13, **characterized in that** the product withdrawn at the top of the first distillation column comprises dialkyl carbonate and is fed fully or partly to the further process step (c) comprising at least one distillation column for removal of the alkyl alcohol.

15. Process according to at least one of Claims 1 to 14, **characterized in that** at least one of the reaction columns used in the process and/or at least one of the distillation columns used in the process has one or more top condensers which are integrated into the column, where the d/D ratio of the diameter of the vapour line from the column to the top condenser(s) (d) to column diameter of the column (D) is in the range of 0.2 to 1.

16. Process according to at least one of Claims 1 to 15, **characterized in that** the lines and units which conduct mixtures having a melting point of more than 30°C, preferably more than 40°C, are heated to temperatures above this melting point.

17. Process according to at least one of Claims 1 to 16, **characterized in that**
a. a catalyst-containing stream is obtained from the bottom product of at least one distillation column from process step (e) and is recycled fully or partly, if appropriate after further purification, back into the process, preferably into process step (a),
b. a stream comprising aromatic hydroxyl compound(s) and alkyl aryl carbonate is obtained from at least one distillation column from process step (e) and is recycled fully or partly back into the process, preferably into process step (a) or (b), and
c. compounds having a boiling point above the boiling point of the diaryl carbonate and compounds whose boiling point is between that of the dialkyl carbonate and that of the alkyl aryl carbonate formed during the preparation of the diaryl carbonate are discharged fully or partly from the process together or separately from one another from at least one distillation column from process step (e).

## Revendications

1. Procédé de fabrication d'au moins un carbonate de diaryle à partir d'au moins un carbonate de dialkyle et d'au moins un composé hydroxy aromatique, selon lequel
(a) le ou les carbonates de dialkyle sont mis en réaction en présence d'au moins un catalyseur de transestérification avec le ou les composés hydroxy aromatiques dans une première colonne de réaction contenant au moins une zone d'enrichissement dans la partie supérieure de la colonne et au moins une zone de réaction sous la zone d'enrichissement, qui comprend au moins deux sections,
(b) le produit de fond de la première colonne de réaction est introduit dans au moins une colonne de réaction supplémentaire contenant au moins une zone d'enrichissement dans la partie supérieure de la colonne et au moins une zone de réaction sous la zone d'enrichissement, et davantage mis en réaction dans celle-ci,
(c) le carbonate de dialkyle non transformé dans les colonnes de réaction des étapes (a) et/ou (b) ou formé pendant la réaction est séparé en totalité ou en partie de l'alcool alkylique formé pendant la réaction dans au moins une étape de procédé supplémentaire contenant au moins une colonne de distillation,
(d) la vapeur contenant un ou plusieurs composés hydroxy aromatiques soutirée à la tête d'au moins une colonne de réaction de (b), éventuellement après condensation dans au moins un condensateur, est introduite en totalité ou en partie dans au moins une étape de procédé supplémentaire contenant au moins une colonne de distillation, des composés dont le point d'ébullition est compris entre celui du carbonate de dialkyle et celui du carbonate d'alkylaryle formé pendant la fabrication du carbonate de diaryle étant séparés, et
(e) le produit de fond contenant du carbonate de diaryle obtenu dans la ou les colonnes de réaction supplémentaires de l'étape (b) est introduit dans au moins une étape de procédé supplémentaire pour la purification dans au moins une colonne de distillation contenant au moins une zone d'enrichissement dans la partie supérieure de la colonne et au moins une zone de rectification dans la partie inférieure de la colonne, au moins une des colonnes de réaction choisies parmi la première ou la ou les colonnes de réaction supplémentaires étant équipée d'un ou de plusieurs condensateurs et la chaleur de condensation obtenue par condensation dans ces condensateurs étant recyclée directement ou indirectement dans le procédé,
au moins une zone d'enrichissement de la première colonne de réaction étant éventuellement équipée d'au moins un condensateur intermédiaire et la chaleur de condensation obtenue par condensation dans ce condensateur intermédiaire étant recyclée directement ou indirectement dans le procédé,
et la pression d'exploitation dans la ou les colonnes de distillation de séparation de l'étape de procédé pour la séparation du carbonate de dialkyle et de l'alcool alkylique étant ajustée de sorte que la température de vaporisation au fond de la ou des colonnes de distillation de séparation de l'étape de procédé pour la séparation du carbonate de dialkyle et de l'alcool alkylique soit inférieure à la température de condensation dans le ou les condensateurs de la ou des colonnes de réaction supplémentaires et/ou le ou les condensateurs intermédiaires de la première colonne de réaction éventuellement présents, et la chaleur de condensation obtenue par condensation dans ces condensateurs étant utilisée directement ou indirectement, en totalité ou en partie, pour la séparation du carbonate de dialkyle de l'alcool alkylique formé pendant la réaction.

2. Procédé selon la revendication 1, **caractérisé en ce qu'**au moins une zone d'enrichissement de la première colonne de réaction est équipée d'au moins un condensateur intermédiaire et la chaleur de condensation obtenue par condensation dans ce condensateur intermédiaire est recyclée directement ou indirectement dans le procédé.

3. Procédé selon la revendication 1 ou 2, **caractérisé en ce que** la ou les colonnes de réaction supplémentaires sont équipées d'un ou de plusieurs condensateurs à la tête de la ou des colonnes de réaction supplémentaires et la chaleur de condensation obtenue par condensation dans ces condensateurs est recyclée directement ou indirectement dans le procédé.

4. Procédé selon au moins l'une quelconque des revendications 1 à 3, **caractérisé en ce que** la chaleur de condensation obtenue par condensation dans le ou les condensateurs de la ou des colonnes de réaction supplémentaires et/ou le ou les condensateurs intermédiaires de la première colonne de réaction éventuellement présents est utilisée directement ou indirectement, en partie, pour la séparation du carbonate de dialkyle de l'alcool alkylique formé pendant la réaction et, en partie, pour la vaporisation du carbonate de dialkyle introduit dans la première colonne de réaction.

5. Procédé selon au moins l'une quelconque des revendications 1 à 4, **caractérisé en ce que** la chaleur de condensation obtenue par condensation dans le ou les condensateurs de la ou des colonnes de réaction supplémentaires est utilisée directement ou indirectement, en totalité ou en partie, pour la séparation du carbonate de dialkyle de l'alcool alkylique formé pendant la réaction, et la chaleur de condensation obtenue par condensation dans le ou les condensateurs intermédiaires de la première colonne de réaction est utilisée directement ou indirectement, en totalité ou en partie, pour la vaporisation du carbonate de dialkyle introduit dans la colonne de réaction.

6. Procédé selon au moins l'une quelconque des revendications 1 à 5, **caractérisé en ce que** le produit de fond contenant du carbonate de diaryle obtenu dans la ou les colonnes de réaction supplémentaires de l'étape (b) contient le catalyseur de transestérification issu de la fabrication du carbonate de diaryle.

7. Procédé selon au moins l'une quelconque des revendications 1 à 6, **caractérisé en ce que** le produit de fond contenant du carbonate de diaryle obtenu dans la ou les colonnes de réaction supplémentaires de l'étape (b) est introduit dans au moins une étape de procédé supplémentaire pour la purification, qui comprend au moins une colonne de distillation, un courant latéral contenant du carbonate de diaryle étant soutiré de cette colonne de distillation.

8. Procédé selon la revendication 7, **caractérisé en ce que** le produit de fond contenant du carbonate de diaryle obtenu dans la ou les colonnes de réaction supplémentaires de l'étape (b) contient des composés ayant un point d'ébullition compris entre celui du carbonate de diaryle et celui du carbonate d'alkylaryle formé en tant que produit secondaire pendant la fabrication du carbonate de diaryle en tant qu'impureté, qui sont soutirés dans un courant latéral supplémentaire de la colonne de distillation et éventuellement recyclés dans la ou une des colonnes de réaction supplémentaires de l'étape (b).

9. Procédé selon la revendication 7 ou 8, **caractérisé en ce que** la colonne de distillation est une colonne à paroi de séparation.

10. Procédé selon au moins l'une quelconque des revendications 1 à 9, **caractérisé en ce que** le carbonate de dialkyle séparé en (c) est réintroduit dans la première colonne de réaction, éventuellement après une purification supplémentaire.

11. Procédé selon au moins l'une quelconque des revendications 1 à 10, **caractérisé en ce que** le ou les composés hydroxy aromatiques obtenus lors de la ou des étapes de procédé pour la séparation de composés dont le point d'ébullition est compris entre celui du carbonate de dialkyle et celui du carbonate d'alkylaryle formé pendant la fabrication du carbonate de diaryle, à partir de la vapeur contenant un ou plusieurs composés hydroxy aromatiques soutirée en partie ou en totalité à la tête d'au moins une colonne de réaction de (b), éventuellement après condensation dans au moins un condensateur, sont réintroduits dans la première colonne de réaction.

12. Procédé selon au moins l'une quelconque des revendications 1 à 11, **caractérisé en ce que** la vapeur contenant un ou plusieurs composés hydroxy aromatiques soutirée à la tête d'au moins une colonne de réaction de (b), éventuellement après condensation dans au moins un condensateur, est introduite dans au moins une étape de procédé supplémentaire, contenant au moins deux colonnes de distillation, le produit de fond de la première colonne de distillation étant introduit dans une seconde colonne de distillation.

13. Procédé selon la revendication 11 ou 12, **caractérisé en ce que** le ou les composés hydroxy aromatiques obtenus lors de la ou des étapes de procédé pour la séparation de composés dont le point d'ébullition est compris entre celui du carbonate de dialyle et celui du carbonate d'alkylaryle formé pendant la fabrication du carbonate de diaryle, sont soutirés en tant que produit de fond d'une première colonne de distillation unique ou en tant que courant latéral d'une deuxième colonne de distillation ou d'une colonne de distillation supplémentaire.

14. Procédé selon au moins l'une quelconque des revendications 11 à 13, **caractérisé en ce que** le produit soutiré à la tête de la première colonne de distillation contient du carbonate de dialkyle et est introduit en totalité ou en partie dans l'étape de procédé (c) supplémentaire contenant au moins une colonne de distillation pour la séparation de l'alcool alkylique.

15. Procédé selon au moins l'une quelconque des revendications 1 à 14, **caractérisé en ce qu'**au moins une des colonnes de réaction utilisées dans le procédé et/ou au moins une des colonnes de distillation utilisées dans le procédé comprend un ou plusieurs condensateurs de tête, qui sont intégrés dans la colonne, le rapport d/D entre le diamètre de la conduite de vapeur allant de la colonne vers le ou les condensateurs de tête (d) et le diamètre de la colonne (D) se situant dans la plage allant de 0,2 à 1.

16. Procédé selon au moins l'une quelconque des revendications 1 à 15, **caractérisé en ce que** les conduites et les appareils qui conduisent des mélanges qui présentent un point de solidification supérieur à 30 °C, de préférence supérieur à 40 °C, sont chauffés à des températures supérieures à ce point de solidification.

17. Procédé selon au moins l'une quelconque des revendications 1 à 16, **caractérisé en ce que**
a. un courant contenant du catalyseur est obtenu à partir du produit de fond d'au moins une colonne de distillation de l'étape de procédé (e), et est recyclé en totalité ou en partie dans le procédé, éventuellement après une purification supplémentaire, de préférence dans l'étape de procédé (a),
b. un courant contenant un ou plusieurs composés hydroxy aromatiques et du carbonate d'alkylaryle est obtenu à partir d'au moins une colonne de distillation de l'étape de procédé (e), et est recyclé en totalité ou en partie dans le procédé, de préférence dans l'étape de procédé (a) ou (b), et
c. des composés ayant un point d'ébullition supérieur au point d'ébullition du carbonate de diaryle et des composés dont le point d'ébullition est compris entre celui du carbonate de dialkyle et celui du carbonate d'alkylaryle formé pendant la fabrication du carbonate de diaryle sont évacués en totalité ou en partie du procédé ensemble ou séparément les uns des autres à partir d'au moins une colonne de distillation de l'étape de procédé (e).
